# EUROPEAN PATENT SPECIFICATION

(11) **EP 2 920 157 B1**
(45) Date of publication and mention of the grant of the patent: **11.09.2019**
(21) Application number: 13854903.5
(22) Date of filing: 15.11.2013
(51) Int. Cl.: C07D 259/00, C07D 273/00, C07D 487/18, C07D 487/22, C07F 19/00, A61P 43/00, C07D 498/18

(54) **DI-MACROCYCLES**
DI-MACROCYCLEN
DI-MACROCYCLES

(30) Priority: 16.11.2012 US 201261727568 P; 15.03.2013 US 201361793265 P
(43) Date of publication of application: 23.09.2015
(73) Proprietor: Lumiphore, Inc., Berkeley, CA 94710-2224 (US)
(72) Inventor: MAGDA, Darren, San Leandro, CA 94577 (US); XU, Jide, Richmond, CA 94804 (US); BUTLIN, Nathaniel, G., Pacifica, CA 94044 (US)
(74) Representative: Harrison IP Limited
(86) International application number: PCT/US2013/070356
(87) International publication number: WO 2014/078690

(56) References cited:
- EP-A1- 2 423 201
- WO-A1-2011/079291
- WO-A2-97/02051
- US-A- 5 739 294
- J. XU ET. AL.: "Octadentate Cages of Tb(III) 2-Hydroxyisophthalamides: A New Standard for Luminescent Lanthanide Labels.", JOURNAL OF THE AMERICAL CHEMICAL SOCIETY, vol. 133, no. 49, 19 October 2011 (2011-10-19), pages 19900-19910, XP002755615, DOI: 10.1021/ja2079898
- G-L LAW ET. AL.: "Circularly Polarized Luminescence of Curium. A New Characterization of the 5f-Actinide Complexes.", JOURNAL OF THE AMERICAN CHEMICAL SOCIETY, vol. 134, no. 37, 25 August 2012 (2012-08-25), pages 15545-15549, XP002755616, DOI: 10.1021/ja306354n
- MOYER, BRUCE A. ET AL: 'ENHANCED LIQUID-LIQUID ANION EXCHANGE USING MACROCYCLIC ANION RECEPTORS: EFFECT OF RECEPTOR STRUCTURE ON SULPHATE-NITRATE EXCHANGE SELECTIVITY.' SUPRAMOLECULAR CHEMISTRY vol. 22, no. 11-12, 01 January 2010, pages 653 - 655, XP055255996

## Description

### CROSS-REFERENCE TO RELATED APPLICATIONS

This application claims the benefit under 35 U.S.C. § 119(e) of U.S. Provisional Application No. 61/793,265, filed March 15, 2013, and U.S. Provisional Application No. 61/727,568, filed November 16, 2012.

### STATEMENT REGARDING FEDERALLY SPONSORED RESEARCH OR DEVELOPMENT

This invention was made with Government support under SBIR Phase I grant No. IIP-1215462 awarded by the National Science Foundation. The Government has certain rights in this invention.

### FIELD OF THE INVENTION

The invention relates to chemical compounds and complexes that can be used in therapeutic and diagnostic applications.

### INTRODUCTION

There are several factors to be considered in the design for an alpha chelating agent for anticancer therapy. Some of the key issues apart from the kinetics will be the high affinity for the target metal (such as Th) which at the same time needs to have a low exchange rate for other biologically significant metal ions. So, in our ligand design, the electronic properties of the target metal and ligand are considered and matched. The chelate should also be able to assume the appropriate coordination cavity size and geometry for the desired metal. In this case, Th, an actinide ion, is a "hard" cation and has a large charge-to-radius ratio. Hence, Th prefers "hard" electron donors and negatively charged oxygen donors. A coordination number of 8 or greater is generally preferred by actinide ions as they have a tendency to form stable complexes with ligands of high denticity; however, the selectivity towards the binding of the thorium will be determined by our design of the chelating unit. The effective but nonselective amino-carboxylic acid ligands such as DTPA can deplete essential biological metal ions from patients, thus causing serious health problems. Selecting the correct type of chelating unit, therefore, is an important factor in achieving high selectivity toward the specific metal ion.

A chelator can comprise numerous chelating moieties. Particularly useful chelators contain a number of chelating moieties sufficient to provide, for example, 6, 8 or 10 heteroatoms such as oxygen that coordinate with a metal ion to form a complex. The heteroatoms such as oxygen provide electron density for forming coordinate bonds with a positively charged ion, and such heteroatoms can thus be considered "donors". In some embodiments, the plurality of chelating moieties of a chelator comprises a plurality of oxygen donors and a metal ion (such as a radionuclide) is chelated to the chelator via at least one of the oxygen donors. In some embodiments, a chelator comprises a plurality of oxygen donors and a metal ion (such as a radionuclide) is chelated to the chelator via a plurality or all of the oxygen donors.

Lanthanide metal complexes useful in diagnostic and therapeutic applications are known. Lanthanide complexes of macrocycle cages based on 2-hydroxy-1,3-bisphthalamide units linked by ethylenediamine groups are described in EP 2,423,201. Macrocycles consisting of N-hydroxy-pyridin-2-one units linked by ethylenediamine groups are described in WO2011079291. Polyaza macrocyclic ligands useful for the complexation of lanthanide ions are described in WO1997002051, US 5,739,294 and Supramolecular Chemistry, Vol 22, p653-655 (2010).

### BRIEF DESCRIPTION OF THE DRAWINGS

Figure 1 shows the electrophoretic mobility of DNA oligonucleotide conjugate **15** (upper band) or DNA oligonucleotide **14** (lower band) in the absence or presence of metal cations.
Figure 2 is an illustration showing protein detection using a luminescent di-macrocycle peptide conjugate.
Figure 3A-B. Figure 3A shows absorption and emission spectra of di-macrocyclic chelator **12** with europium(III). Figure 3B shows absorption and emission spectra of di-macrocyclic chelator **12** with terbium(III).

### DESCRIPTION OF EMBODIMENTS

### 1. Definitions

Where substituent groups are specified by their conventional chemical formulae, written from left to right, they optionally equally encompass the chemically identical substituents, which would result from writing the structure from right to left, *e.g.,* -CH₂O- is intended to also recite -OCH₂-.

The term "alkyl", by itself or as part of another substituent, means a straight or branched chain hydrocarbon, which may be fully saturated, mono- or polyunsaturated and includes mono-, di- and multivalent radicals. Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds (i.e., alkenyl and alkynyl moieties). Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl" can refer to "alkylene", which by itself or as part of another substituent means a divalent radical derived from an alkane, as exemplified, but not limited, by -CH₂CH₂CH₂CH₂-. Typically, an alkyl (or alkylene) group will have from 1 to 30 carbon atoms. A "lower alkyl" or "lower alkylene" is a shorter chain alkyl or alkylene group, generally having eight or fewer carbon atoms. In some embodiments, alkyl refers to an alkyl or combination of alkyls selected from C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉ and C₃₀ alkyl. In some embodiments, alkyl refers to C₁-C₂₅ alkyl. In some embodiments, alkyl refers to C₁-C₂₀ alkyl. In some embodiments, alkyl refers to C₁-C₁₅ alkyl. In some embodiments, alkyl refers to C₁-C₁₀ alkyl. In some embodiments, alkyl refers to C₁-C₆ alkyl.

The term "heteroalkyl," by itself or in combination with another term, means an alkyl in which one or more carbons are replaced with one or more heteroatoms selected from the group consisting of O, N, Si and S, (preferably O, N and S), wherein the nitrogen and sulfur atoms may optionally be oxidized and the nitrogen heteroatom may optionally be quaternized. The heteroatoms O, N, Si and S may be placed at any interior position of the heteroalkyl group or at the position at which the alkyl group is attached to the remainder of the molecule. In some embodiments, depending on whether a heteroatom terminates a chain or is in an interior position, the heteroatom may be bonded to one or more H or substituents such as (C₁, C₂, C₃, C₄, C₅ or C₆) alkyl according to the valence of the heteroatom. Examples of heteroalkyl groups include, but are not limited to, -CH₂-CH₂-O-CH₃, -CH₂-CH₂-NH-CH₃, -CH₂-CH₂-N(CH₃)-CH₃, -CH₂-S-CH₂-CH₃, -CH₂-CH₂,-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. No more than two heteroatoms may be consecutive, as in, for example, -CH₂-NH-OCH₃ and -CH₂-O-Si(CH₃)₃, and in some instances, this may place a limit on the number of heteroatom substitutions. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. The designated number of carbons in heteroforms of alkyl, alkenyl and alkynyl includes the heteroatom count. For example, a (C₁, C₂, C₃, C₄, C₅ or C₆) heteroalkyl will contain, respectively, 1, 2, 3, 4, 5 or 6 atoms selected from C, N, O, Si and S such that the heteroalkyl contains at least one C atom and at least one heteroatom, for example 1-5 C and 1 N or 1-4 C and 2 N. Further, a heteroalkyl may also contain one or more carbonyl groups. In some embodiments, a heteroalkyl is any C₂-C₃₀ alkyl, C₂-C₂₅ alkyl, C₂-C₂₀ alkyl, C₂-C₁₅ alkyl, C₂-C₁₀ alkyl or C₂-C₆ alkyl in any of which one or more carbons are replaced by one or more heteroatoms selected from O, N, Si and S (or from O, N and S). In some embodiments, each of 1, 2, 3, 4 or 5 carbons is replaced with a heteroatom.The terms "alkoxy," "alkylamino" and "alkylthio" (or thioalkoxy) are used in their conventional sense, and refer to those alkyl and heteroalkyl groups attached to the remainder of the molecule via an oxygen atom, a nitrogen atom (e.g., an amine group), or a sulfur atom, respectively.

The terms "cycloalkyl" and "heterocycloalkyl", by themselves or in combination with other terms, refer to cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1 -(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The term "aryl" means a polyunsaturated, aromatic substituent that can be a single ring or optionally multiple rings (preferably 1, 2 or 3 rings) that are fused together or linked covalently. In some embodiments, aryl is a 3, 4, 5, 6, 7 or 8 membered ring, which is optionally fused to one or two other 3, 4, 5, 6, 7 or 8 membered rings. The term "heteroaryl" refers to aryl groups (or rings) that contain 1, 2, 3 or 4 heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl.

In some embodiments, any of alkyl, heteroalkyl, cycloalkyl, heterocycloalkyl, aryl and heteroaryl is optionally substituted. That is, in some embodiments, any of these groups is substituted or unsubstituted. In some embodiments, substituents for each type of radical are selected from those provided below.

Substituents for the alkyl, heteroalkyl, cycloalkyl and heterocycloalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) are generically referred to as "alkyl group substituents". In some embodiments, an alkyl group substituent is selected from -halogen, -OR', =O, =NR', =N-OR', -NR'R",-SR', -SiR'R"R"', -OC(O)R', -C(O)R', -CO₂R', -CONR'R", -OC(O)NR'R",-NR"C(O)R', -NR'-C(O)NR"R''', -NR"C(O)₂R', -NR-C(NR'R"R''')=NR"", -NR-C(NR'R")=NR '", -S(O)R', -S(O)₂R', -S(O)₂NR'R", -NRSO₂R', -CN and -NO₂ in a number ranging from zero to (2m'+1), where m' is the total number of carbon atoms in such radical. In one embodiment, R', R", R'" and R"" are each independently selected from hydrogen, alkyl (e.g., C₁, C₂, C₃, C₄, C₅ and C₆ alkyl). In one embodiment, R', R", R'" and R"" each independently refer to hydrogen, substituted or unsubstituted heteroalkyl, substituted or unsubstituted aryl, *e.g.,* aryl substituted with 1-3 halogens, substituted or unsubstituted alkyl, alkoxy or thioalkoxy groups, or arylalkyl groups. In one embodiment, R', R", R'" and R"" are each independently selected from hydrogen, alkyl, heteroalkyl, cycloalkyl, heterocycloalkyl, aryl, heteroaryl, alkoxy, thioalkoxy groups, and arylalkyl. When R' and R" are attached to the same nitrogen atom, they can be combined with the nitrogen atom to form a 5-, 6-, or 7-membered ring. For example, -NR'R" can include 1-pyrrolidinyl and 4-morpholinyl. In some embodiments, an alkyl group substituent is selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl.

Similar to the substituents described for the alkyl radical, substituents for the aryl and heteroaryl groups are generically referred to as "aryl group substituents". In some embodiments, an aryl group substituent is selected from -halogen, -OR', =O, =NR', =N-OR', -NR'R", -SR',-SiR'R"R"', -OC(O)R', -C(O)R', -CO₂R', -CONR'R", -OC(O)NR'R",-NR"C(O)R', -NR'-C(O)NR"R"',-NR"C(O)₂R', -NR-C(NR'R"R'")=NR"", -NR-C(NR'R")=NR"', -S(O)R',-S(O)₂R', -S(O)₂NR'R", -NRSO₂R', -CN and -NO₂, -R', -N₃, -CH(Ph)₂, fluoro(C₁-C₄)alkoxy, and fluoro(C₁-C₄)alkyl, in a number ranging from zero to the total number of open valences on the aromatic ring system. In some embodiments, R', R", R'" and R"" are independently selected from hydrogen and alkyl (e.g., C₁, C₂, C₃, C₄, C₅ and C₆ alkyl). In some embodiments, R', R", R'" and R"" are independently selected from hydrogen, substituted or unsubstituted alkyl, substituted or unsubstituted heteroalkyl, substituted or unsubstituted aryl and substituted or unsubstituted heteroaryl. In some embodiments, R', R", R'" and R"" are independently selected from hydrogen, alkyl, heteroalkyl, aryl and heteroaryl. In some embodiments, an aryl group substituent is selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl.

Two of the substituents on adjacent atoms of the aryl or heteroaryl ring may optionally be replaced with a substituent of the formula -T-C(O)-(CRR')_{q}-U-, wherein T and U are independently -NR-, -O-, -CRR'- or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O)-, -S(O)₂-, -S(O)₂NR'- or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula -(CRR')ₛ-X-(CR"R"')_{d}-, where s and d are independently integers of from 0 to 3, and X is -O-, -NR'-, -S-,-S(O)-, -S(O)₂-, or -S(O)₂NR'-. The substituents R, R', R" and R'" are preferably independently selected from hydrogen or substituted or unsubstituted (C₁-C₆)alkyl.

The term "acyl" refers to a species that includes the moiety -C(O)R, where R has the meaning defined herein. Exemplary species for R include H, halogen, substituted or unsubstituted alkyl, substituted or unsubstituted aryl, substituted or unsubstituted heteroaryl, and substituted or unsubstituted heterocycloalkyl. In some embodiments, R is selected from H and (C₁-C₆)alkyl.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like. In some embodiments, halogen refers to an atom selected from F, Cl and Br.

The term "heteroatom" includes oxygen (O), nitrogen (N), sulfur (S) and silicon (Si). In some embodiments, a heteroatom is selected from N and S. In some embodiments, the heteroatom is O.

Unless otherwise specified, the symbol "R" is a general abbreviation that represents a substituent group that is selected from acyl, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound includes more than one R, R', R", R'" and R"" group, they are each independently selected.

For groups with solvent exchangeable protons, the ionized form is equally contemplated. For example, -COOH also refers to -COO⁻ and -OH also refers to -O⁻.

Any of the compounds disclosed herein can be made into a pharmaceutically acceptable salt. The term "pharmaceutically acceptable salts" includes salts of compounds that are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge et al., Journal of Pharmaceutical Science, 66: 1-19 (1977)). Certain specific compounds of the present invention contain both basic and acidic functionalities that allow the compounds to be converted into either base or acid addition salts. The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In addition to salt forms, the present invention provides any of the compounds disclosed herein in a prodrug form. Prodrugs of the compounds described herein are those compounds that readily undergo chemical changes under physiological conditions to provide the compounds of the present invention.

Certain compounds of the present invention can exist in unsolvated forms as well as solvated forms, including hydrated forms. In general, the solvated forms are equivalent to unsolvated forms and are encompassed within the scope of the present invention. Certain compounds of the present invention may exist in multiple crystalline or amorphous forms. In general, all physical forms are equivalent for the uses contemplated by the present invention and are intended to be within the scope of the present invention.

The compounds of the present invention may also contain unnatural proportions of atomic isotopes at one or more of the atoms that constitute such compounds. For example, the compounds may be labeled with deuterium (²H) or radiolabeled with radioactive isotopes, such as for example tritium (³H), iodine-125 (¹²⁵I) or carbon-14 (¹⁴C). All isotopic variations of the compounds of the present invention, whether radioactive or not, are intended to be encompassed within the scope of the present invention.

The symbol 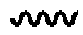 , displayed perpendicular to a bond, indicates the point at which the displayed moiety is attached to the remainder of the molecule.

In some embodiments, the definition of terms used herein is according to IUPAC.

### 2. Compositions

The invention provides numerous chelators and metal ion complexes thereof. Generally, a chelator comprises a plurality of chelating agents that are linked together by way of three or more scaffold moieties.

Accordingly, in one aspect, the invention provides a di-macrocycle having the structure: 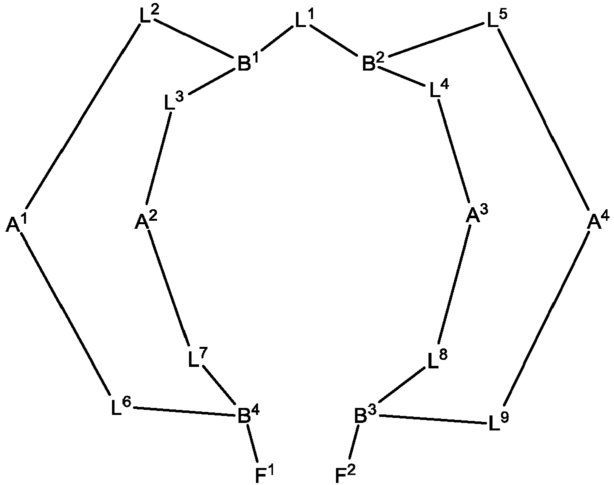 wherein
B¹, B², B³, and B⁴ are independently selected from N, C, B, Si, and P;
F¹ and F² are independently selected from H, C₁-C₈ alkyl, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and S and C₃-C₈ heterocycloalkyl, and wherein, when F1 and F2 are not H, F1 and F2 each comprise a modifying moiety selected from a solubilizing group, an estradiol-derived moiety, , an oligonucleotide, ssDNA, dsDNA, RNA, and a peptide, and optionally wherein F1 and F2 are independently selected from a polyether and a peptide;
L¹, L², L³, L⁴, L⁵, L⁶, L⁷, L⁸, and L⁹ are independently selected from C₁-C₈ alkyl optionally substituted with a linker, heteroalkyl of 2, 3, 4, 5 or 6 atoms selected from C, N, O, Si and S such that the heteroalkyl contains at least on C atom and at least one heteroatom, and C₃-C₈ heterocycloalkyl;
A¹, A², A³ and A⁴ are members independently selected from the general structures: 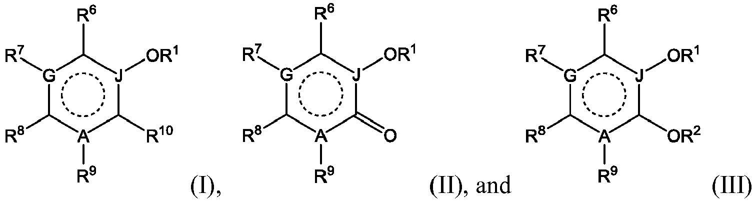 wherein
   A and G are independently selected from carbon, nitrogen and oxygen;
   J is selected from carbon and nitrogen;
   each R¹ and R² are independently selected from H, and a single negative charge;
   each R⁶, R⁷, R⁸, R⁹, and R¹⁰ are independently selected from
      a bond to L², L³, L⁴, L⁵, L⁶, L⁷, L⁸, or L⁹, alkanediyl attached to L², L³, L⁴, L⁵, L⁶, L⁷, L⁸, or L⁹,
      H, alkyl, heteroalkyl, halogen, CN,
      CF₃, -C(O)R¹⁷, -SO₂NR¹⁷R¹⁸, -NR¹⁷R¹⁸, -OR¹⁷, -S(O)₂R¹⁷, -COOR¹⁷, -S(O )₂OR¹⁷, -OC(O)R¹⁷, -C(O)NR¹⁷R¹⁸, -NR¹⁷C(O)R¹⁸, -NR¹⁷SO₂R¹⁸, and -NO₂,
   wherein
      at least two of R⁶, R⁷, R⁸, R⁹, and R¹⁰ are optionally joined to form a ring system selected from cycloalkyl, heterocycloalkyl, aryl and heteroaryl;
      R¹⁷ and R¹⁸ are independently selected from H, alkyl, heteroalkyl, aryl, heteroaryl and heterocycloalkyl; and
      R¹⁷ and R¹⁸, together with the atoms to which they are attached, are optionally joined to form a 5-, 6- or 7-membered ring.
      when A is oxygen, R⁹ is not present; and
      when G is oxygen, R⁷ is not present;
      A¹ is attached to L² and L⁶ through two members selected from R⁶, R⁷, R⁸, R⁹ and R¹⁰;
      A² is attached to L³ and L⁷ through two members selected from R⁶, R⁷, R⁸, R⁹ and R¹⁰;
      A³ is attached to L⁴ and L⁸ through two members selected from R⁶, R⁷, R⁸, R⁹ and R¹⁰; and
      A⁴ is attached to L⁵ and L⁹ through two members selected from R⁶, R⁷, R⁸, R⁹ and R¹⁰.

In one embodiment of the invention, when A¹ has a structure according to formula (I), A¹ is attached to L² and L⁶ through R⁶ and R¹⁰;
when A¹ has a structure according to formula (II) or (III), A¹ is attached to L² and L⁶ through R⁶ and R⁹;
when A² has a structure according to formula (I), A² is attached to L³ and L⁷ through R⁶ and R¹⁰;
when A² has a structure according to formula (II) or (III), A² is attached to L³ and L⁷ through R⁶ and R⁹;
when A³ has a structure according to formula (I), A³ is attached to L⁴ and L⁸ through R⁶ and R¹⁰;
when A³ has a structure according to formula (II) or (III), A³ is attached to L⁴ and L⁸ through R⁶ and R⁹;
when A⁴ has a structure according to formula (I), A⁴ is attached to L⁵ and L⁹ through R⁶ and R¹⁰; and
when A⁴ has a structure according to formula (II) or (III), A⁴ is attached to L⁵ and L⁹ through R⁶ and R⁹.

In a further embodiment of the invention,
when A¹ has a structure according to formula (I), at least one of R⁶ and R¹⁰ of A¹ is a bond attached to L² or L⁶;
when A² has a structure according to formula (I), at least one of R⁶ and R¹⁰ of A² is a bond attached to L³ or L⁷;
when A³ has a structure according to formula (I), at least one of R⁶ and R¹⁰ of A³ is a bond attached to L⁴ or L⁸; and
when A⁴ has a structure according to formula (I), at least one of R⁶ and R¹⁰ of A⁴ is a bond attached to L⁵ or L⁹.

In one embodiment of the invention, each of A¹, A², A³ and A⁴ is independently selected from:

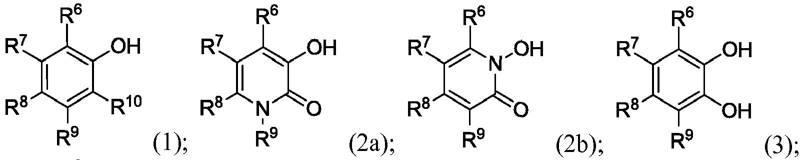

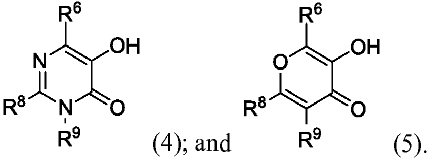

In a further embodiment of the invention,
when A¹ has a structure according to formula (1), A¹ is attached to L² and L⁶ through R⁶ and R¹⁰;
when A¹ has a structure according to formula (2a), (2b), (3), (4) or (5), A¹ is attached to L² and L⁶ through R⁶ and R⁹;
when A² has a structure according to formula (1), A² is attached to L³ and L⁷ through R⁶ and R¹⁰;
when A² has a structure according to formula (2a), (2b), (3), (4) or (5), A² is attached to L³ and L⁷ through R⁶ and R⁹;
when A³ has a structure according to formula (1), A³ is attached to L⁴ and L⁸ through R⁶ and R¹⁰;
when A³ has a structure according to formula (2a), (2b), (3), (4) or (5), A³ is attached to L⁴ and L⁸ through R⁶ and R⁹;
when A⁴ has a structure according to formula (1), A⁴ is attached to L⁵ and L⁹ through R⁶ and R¹⁰; and
when A⁴ has a structure according to formula (2a), (2b), (3), (4) or (5), A⁴ is attached to L⁵ and L⁹ through R⁶ and R⁹.

In a further embodiment of the invention,
when A¹ has a structure according to formula (1), at least one of R⁶ and R¹⁰ of A¹ is a bond attached to L² or L⁶;
when A² has a structure according to formula (1), at least one of R⁶ and R¹⁰ of A² is a bond attached to L³ or L⁷;
when A³ has a structure according to formula (1), at least one of R⁶ and R¹⁰ of A³ is a bond attached to L⁴ or L⁸; and
when A⁴ has a structure according to formula (1), at least one of R⁶ and R¹⁰ of A⁴ is a bond attached to L⁵ or L⁹.

In one embodiment, the di-macrocycle of the invention has the structure: 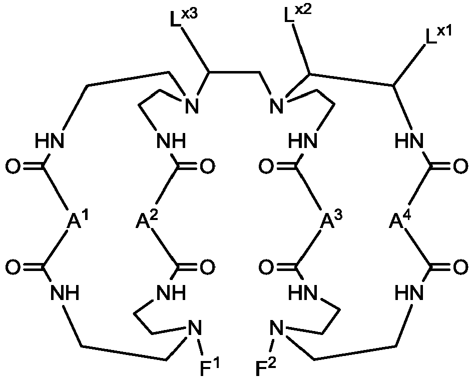 wherein L^{x1}, L^{x2} and L^{x3} are independently selected from H and a linker.

In one embodiment, the di-macrocycle of the invention has the structure:

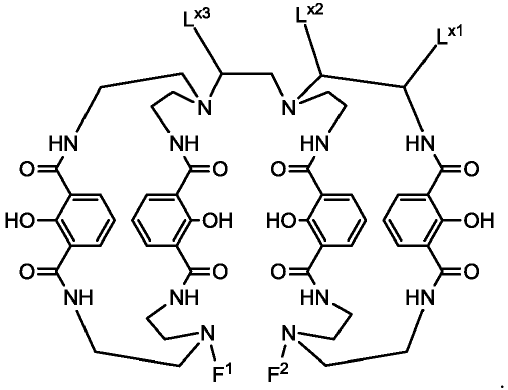

In one embodiment, the di-macrocycle of the invention has the structure: 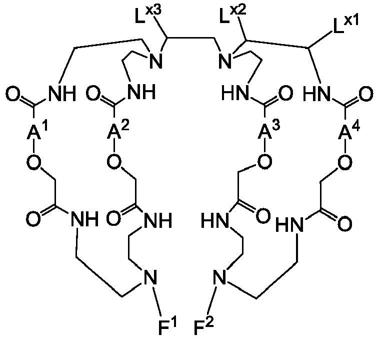 wherein L^{x1}, L^{x2} and L^{x3} are independently selected from H and a linker.

In one embodiment, the di-macrocycle of the invention has the structure:

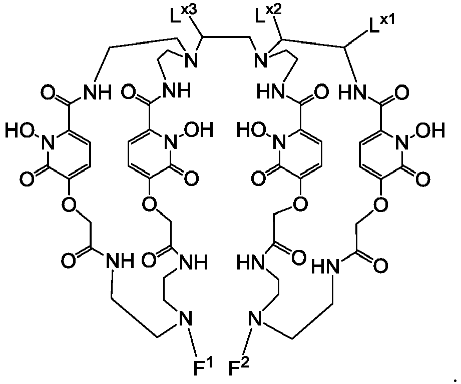

In a further aspect, the invention provides a di-macrocycle having the structure: 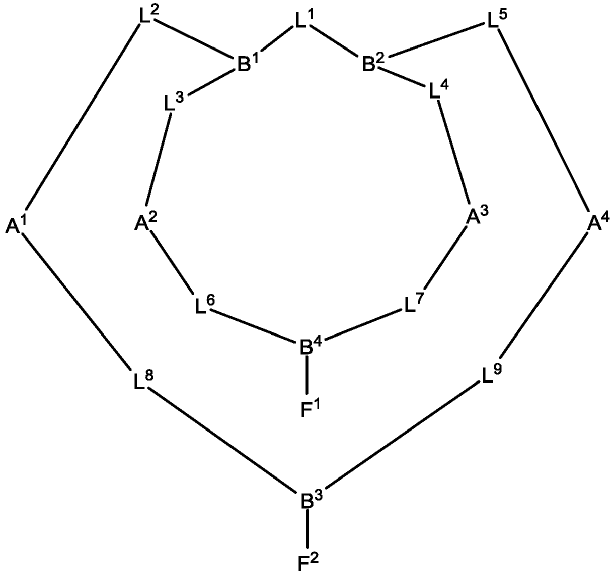 wherein
B¹, B², B³, and B⁴ are independently selected from N, C, B, Si, and P;
F¹ and F² are independently selected from H, C₁-C₈ alkyl, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and S and C₃-C₈ heterocycloalkyl, and wherein, when F1 and F2 are not H, F1 and F2 each comprise a modifying moiety selected from a solubilizing group, an estradiol-derived moiety, an oligonucleotide, ssDNA, dsDNA, RNA, and a peptide, and optionally wherein F1 and F2 are independently selected from a polyether and a peptide;
L¹, L², L³, L⁴, L⁵, L⁶, L⁷, L⁸, and L⁹ are independently selected from C₁-C₈ alkyl optionally substituted with a linker, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and S and C₃-C₈ heterocycloalkyl;
A¹, A², A³ and A⁴ are members independently selected from the general structures: 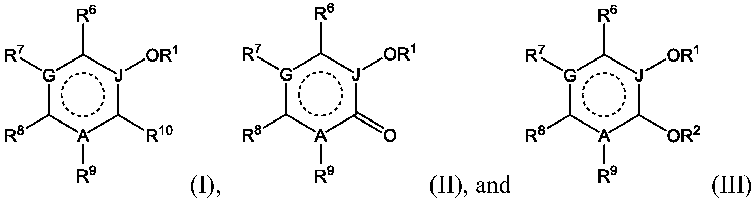 wherein
   A and G are independently selected from carbon, nitrogen and oxygen;
   J is selected from carbon and nitrogen;
   each R¹ and R² are independently selected from H, and a single negative charge;
   each R⁶, R⁷, R⁸, R⁹, and R¹⁰ are independently selected from
      a bond to L², L³, L⁴, L⁵, L⁶, L⁷, L⁸, or L⁹, alkanediyl attached to L², L³, L⁴, L⁵, L⁶, L⁷, L⁸, or L⁹,
      H, alkyl, heteroalkyl, halogen, CN,
      CF₃, -C(O)R¹⁷, -SO₂NR¹⁷R¹⁸, -NR¹⁷R¹⁸, -OR¹⁷, -S(O)₂R¹⁷, -COOR¹⁷, -S(O )₂OR¹⁷, -OC(O)R¹⁷, -C(O)NR¹⁷R¹⁸, -NR¹⁷C(O)R¹⁸, -NR¹⁷SO₂R¹⁸, and -NO₂,
   wherein
      at least two of R⁶, R⁷, R⁸, R⁹, and R¹⁰ are optionally joined to form a ring system selected from cycloalkyl, heterocycloalkyl, aryl and heteroaryl;
      R¹⁷ and R¹⁸ are independently selected from H, alkyl, heteroalkyl, aryl, heteroaryl and heterocycloalkyl; and
      R¹⁷ and R¹⁸, together with the atoms to which they are attached, are optionally joined to form a 5-, 6- or 7-membered ring.
      when A is oxygen, R⁹ is not present; and
      when G is oxygen, R⁷ is not present;
      A¹ is attached to L² and L⁸ through two members selected from R⁶, R⁷, R⁸, R⁹ and R¹⁰;
      A² is attached to L³ and L⁶ through two members selected from R⁶, R⁷, R⁸, R⁹ and R¹⁰;
      A³ is attached to L⁴ and L⁷ through two members selected from R⁶, R⁷, R⁸, R⁹ and R¹⁰; and
      A⁴ is attached to L⁵ and L⁹ through two members selected from R⁶, R⁷, R⁸, R⁹ and R¹⁰.

In one embodiment of the invention, the di-macrocycle is covalently modified with at least one linker.

In one embodiment of the invention, one of L¹, L², L³, L⁴, L⁵, L⁶, L⁷, L⁸, and L⁹ is substituted with a linker.

In one embodiment of the invention,
when A¹ has a structure according to formula (I), A¹ is attached to L² and L⁸ through R⁶ and R¹⁰;
when A¹ has a structure according to formula (II) or (III), A¹ is attached to L² and L⁸ through R⁶ and R⁹;
when A² has a structure according to formula (I), A² is attached to L³ and L⁶ through R⁶ and R¹⁰;
when A² has a structure according to formula (II) or (III), A² is attached to L³ and L⁶ through R⁶ and R⁹;
when A³ has a structure according to formula (I), A³ is attached to L⁴ and L⁷ through R⁶ and R¹⁰;
when A³ has a structure according to formula (II) or (III), A³ is attached to L⁴ and L⁷ through R⁶ and R⁹;
when A⁴ has a structure according to formula (I), A⁴ is attached to L⁵ and L⁹ through R⁶ and R¹⁰; and
when A⁴ has a structure according to formula (II) or (III), A⁴ is attached to L⁵ and L⁹ through R⁶ and R⁹.

In one embodiment of the invention, each of A¹, A², A³ and A⁴ is independently selected from:

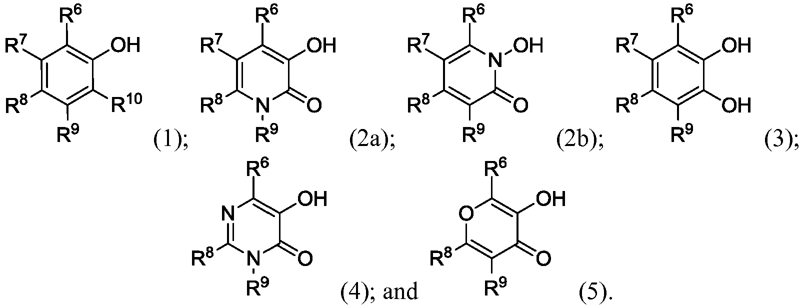

In one embodiment of the invention,
when A¹ has a structure according to formula (1), A¹ is attached to L² and L⁸ through R⁶ and R¹⁰;
when A¹ has a structure according to formula (2a), (2b), (3), (4) or (5), A¹ is attached to L² and L⁸ through R⁶ and R⁹;
when A² has a structure according to formula (1), A² is attached to L³ and L⁶ through R⁶ and R¹⁰;
when A² has a structure according to formula (2a), (2b), (3), (4) or (5), A² is attached to L³ and L⁶ through R⁶ and R⁹;
when A³ has a structure according to formula (1), A³ is attached to L⁴ and L⁷ through R⁶ and R¹⁰;
when A³ has a structure according to formula (2a), (2b), (3), (4) or (5), A³ is attached to L⁴ and L⁷ through R⁶ and R⁹;
when A⁴ has a structure according to formula (1), A⁴ is attached to L⁵ and L⁹ through R⁶ and R¹⁰; and
when A⁴ has a structure according to formula (2a), (2b), (3), (4) or (5), A⁴ is attached to L⁵ and L⁹ through R⁶ and R⁹.

In one embodiment, the di-macrocycle of the invention has the structure: 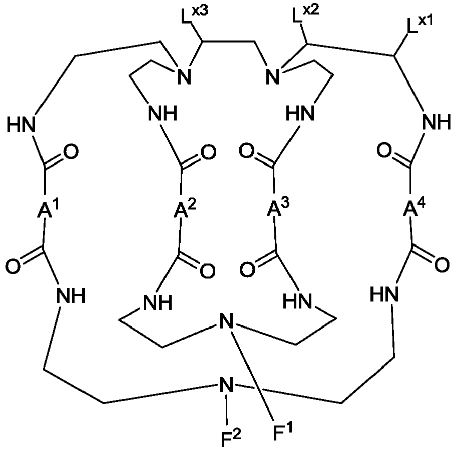 wherein L^{x1}, L^{x2} and L^{x3} are independently selected from H and a linker.

In one embodiment, the di-macrocycle of the invention has the structure:

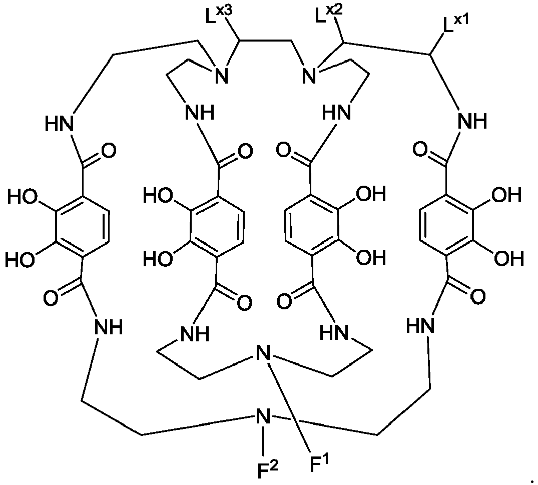

In one embodiment, the di-macrocycle of the invention has the structure:

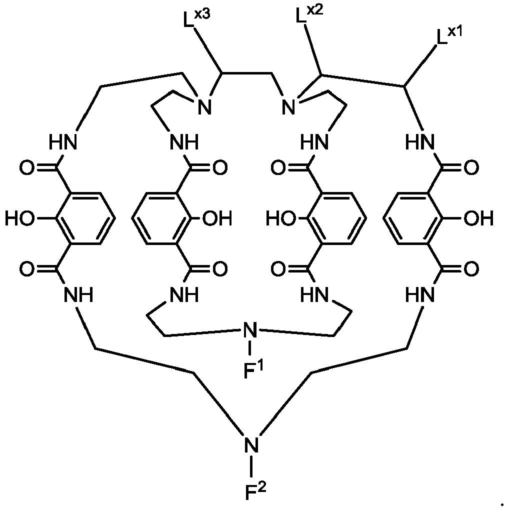

In a yet further aspect, the invention provides a complex comprising a di-macrocycle of the invention and a metal ion,
optionally wherein the metal ion is a lanthanide, optionally the lanthanide is selected from Eu and Tb,
optionally wherein the metal ion is an actinide,
optionally wherein the metal ion is selected from yttrium(III), europium(III), terbium(III), zirconium(IV) and thorium(IV),
optionally wherein the metal ion is a radionuclide selected from isotopes of U, Pu, Fe, Cu, Sm, Gd, Tb, Dy, Ho, Er, Yb, Lu, Y, Th, Zr, In, Ga, Bi, Ra, At and Ac,
optionally wherein said metal ion is ²²⁷Th(IV) or ⁸⁹Zr(IV),
optionally wherein said metal is a member selected from ¹⁷⁷Lu, ¹⁶⁶Ho, ¹⁵³Sm, ⁹⁰Y, ⁸⁶Y, ¹⁶⁶Dy, ¹⁶⁵Dy, ¹⁶⁹Er, ¹⁷⁵Yb, ²²⁵Ac, ¹⁴⁹Tb, ¹⁵³Gd, and ²³⁰U or
optionally wherein said metal is a member selected from ¹¹¹In, ⁶⁷Ga, ⁶⁷Cu, ⁶⁴Cu, ¹⁸⁶Re, ¹⁸⁸Re, ¹¹¹Ag, ¹⁰⁹Pd, ²¹²Pb, ²⁰³Pb, ²¹²Bi, ²¹³Bi, ^{195m}Pt, ²⁰¹Tl, ⁵⁵Co, and ^{99m}Tc.

In one embodiment of the invention, the estradiol-derived group is selected from estradiol and the estradiol-derived group of example 13.

### 2.1.1. Chelating Moieties

A¹, A², A³, A⁴ and A⁵ are chelating moieties having a structure independently selected from: 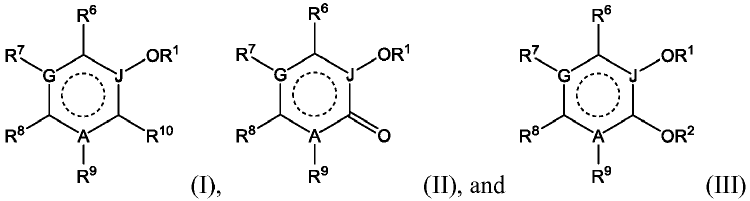 wherein
A and G are independently selected from carbon, nitrogen and oxygen;
wherein when A is oxygen, R⁹ is not present; and when G is oxygen, R⁷ is not present;
J is selected from carbon and nitrogen;
each R¹ and R² are independently selected from H, an enzymatically labile group, a hydrolytically labile group, a metabolically labile group, a photolytically labile group and a single negative charge;
each R⁶, R⁷, R⁸, R⁹, and R¹⁰ are independently selected from a bond to S¹, S² or S³, alkanediyl attached to S¹, S² or S³, H, substituted or unsubstituted alkyl, substituted or unsubstituted heteroalkyl, halogen, CN,
CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C (O)NR¹⁷R¹⁸, -NR¹⁷C(O)R¹⁸, -NR¹⁷SO₂R¹⁸, and -NO₂,
wherein
at least two of R⁶, R⁷, R⁸, R⁹, and R¹⁰ are optionally joined to form a ring system selected from substituted or unsubstituted cycloalkyl, substituted or unsubstituted heterocycloalkyl, substituted or unsubstituted aryl and substituted or unsubstituted heteroaryl;
R¹⁷ and R¹⁸ are independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl; and
R¹⁷ and R¹⁸, together with the atoms to which they are attached, are optionally joined to form a 5-, 6- or 7-membered ring;
wherein A¹, A², A³, A⁴ and A⁵ are each attached to S¹ and S² or S¹ and S³ through two members selected from R⁶, R⁷, R⁸, R⁹ and R¹⁰.

In some embodiments, when any of A¹, A², A³, A⁴ and A⁵ has a structure according to formula (I), the respective chelating moiety is attached to S¹ and S² or S¹ and S³ through R⁶ and R¹⁰.
In some embodiments, when any of A¹, A², A³, A⁴ and A⁵ has a structure according to formula (II) or (III), the respective chelating moiety is attached to S¹ and S² or S¹ and S³ through R⁶ and R⁹.

In some embodiments, at least one of R⁶ and R¹⁰ in (I) is a bond attached to S¹, S², or S³.

In some embodiments, A¹, A², A³, A⁴ and A⁵ are chelating moieties having a structure independently selected from: 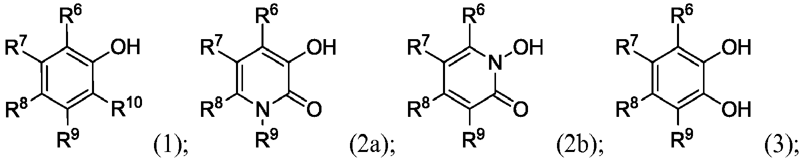 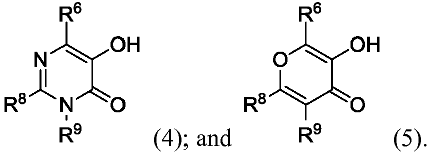 R⁶, R⁷, R⁸, R⁹, and R¹⁰ are as defined herein.

In some embodiments, A¹, A², A³, A⁴ and A⁵ are chelating moieties having a structure independently selected from: 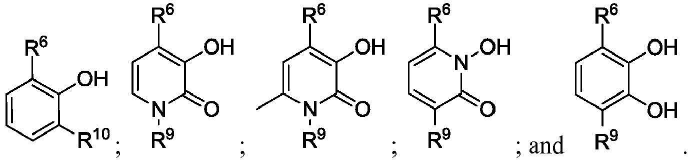 R⁶, R⁹, and R¹⁰ are as defined herein.

### 2.1.2. Scaffold Moieties

A "scaffold moiety" is any moiety useful for covalently linking two or more chelating moieties in any of the chelators (di-macrocycles) disclosed herein. In exemplary embodiments, any two scaffold moieties disclosed herein are joined via a plurality of chelating moieties to form a macrocycle. In exemplary embodiments, one or more scaffold moieties of a chelator is substituted with a linker. In one embodiment, the scaffold moiety is selected from substituted or unsubstituted alkyl and substituted or unsubstituted heteroalkyl. Exemplary scaffold moieties include linear or branched ethers and amines. In some embodiments, the linker is attached to a targeting moiety. In some embodements, the scaffold moiety comprises a targeting moiety.

Other exemplary scaffold moieties may include, but are not limited to:

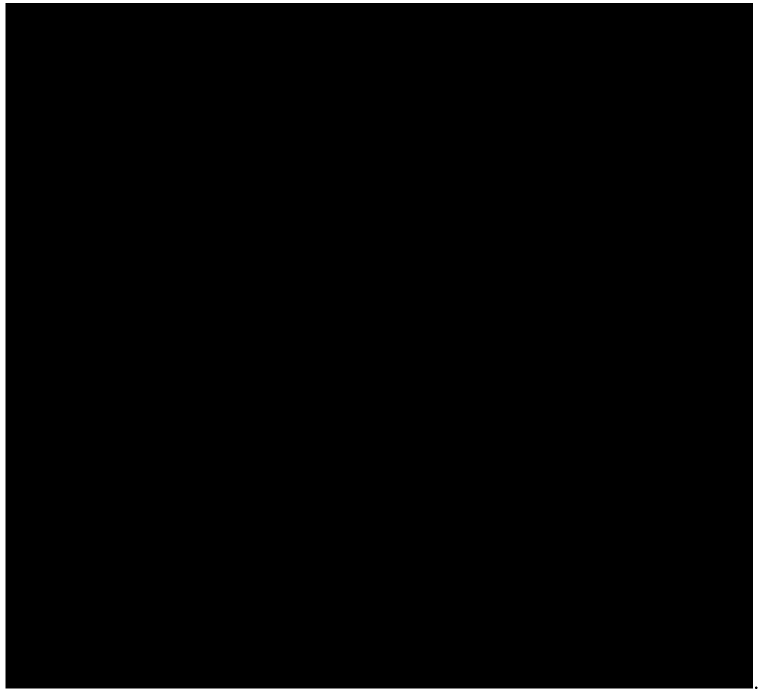

"X" represents a locus of attachment for a chelating moiety, and in exemplary embodiments includes a heteroatom such as nitrogen. Thus, in some embodiments, X is NR'R", wherein R' and R" are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, halogen, CN,
CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C (O)NR¹⁷R¹⁸, -NR¹⁷C(O)R¹⁸, -NR¹⁷SO₂R¹⁸, -NO₂; and R¹⁷ and R¹⁸ are each independently selected from H, substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl; wherein at least one R' or R" comprises a bond to a chelating moiety. The chelating moiety can be attached to a scaffold via any appropriate linker.

In some embodiments, a scaffold moiety is linear. One exemplary scaffold moiety is X-(CH₂)₃-X-(CH₂)₄-X-(CH₂)₃-X, which is preferably substituted (e.g. with a linker) at at least one of the alkyl moieties. That is, one exemplary scaffold moiety is spermine based. Other exemplary scaffold moieties include 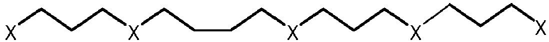 and 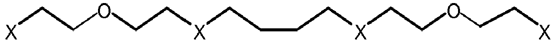 any of which is preferably substituted (e.g. with a linker) at at least one of the alkyl moieties. X is as given in the previous paragraph.

One preferred moiety for at least one of the X moieties is the 1,2-HOPO amide moiety, but those of skill in the art will appreciate that other chelating moieties in any used in any combination. In each of the scaffold structures, an aryl moiety or alkyl moiety can be substituted with one or more "aryl group substituent" or "alkyl group substituent" as defined herein.

A particularly useful scaffold moiety for any chelator described herein has the structure 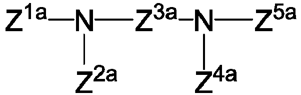 wherein Z^{1a}, Z^{2a}, Z^{3a}, Z^{4a} and Z^{5a} are selected from substituted or unsubstituted alkyl and substituted or unsubstituted heteroalkyl; and Z^{1a}, Z^{2a}, Z^{4a} and Z^{5a} comprise a bond to one of the chelating moieties.

In some embodiments, Z^{3a} is substituted or unsubstituted (C₁, C₂, C₃, C₄, C₅ or C₆) alkyl. In some embodiments, Z^{3a} is substituted or unsubstituted -(CH₂)ₘ(CH₂CH₂O)ₙ(CH₂)ₚ-, wherein m, n and p are integers independently selected from 1, 2, 3, 4, 5 and 6. In some embodiments, Z^{3a} is ethyl. In some embodiments, Z^{3a} is ethyl substituted with =O.

In some embodiments, Z^{1a}, Z^{2a}, Z^{4a} and Z^{5a} have a structure selected from Z'R^{20a}N(H)C(O)Z", Z'R^{20a}N(H)C(O)R^{21a}Z" and Z'R^{21a}Z" wherein Z' is a bond to the second scaffold moiety, Z" is a bond to one of the plurality of chelating moieties, R^{20a} is selected from substituted or unsubstituted alkyl and substituted or unsubstituted heteroalkyl. and R^{21a} is selected from substituted or unsubstituted alkyl and substituted or unsubstituted heteroalkyl. In some embodiments, R^{20a} is selected from substituted or unsubstituted (C₁, C₂, C₃, C₄, C₅ or C₆) alkyl and substituted or unsubstituted (C₁, C₂, C₃, C₄, C₅ or C₆) heteroalkyl. In some embodiments, R^{20a} is selected from substituted or unsubstituted ethyl. In some embodiments, R^{21a} is from substituted or unsubstituted -(CH₂)_{w}O- wherein w is selected from 1, 2, 3, 4, 5 and 6. In exemplary embodiments, w is 1 or 3.

In some embodiments, at least one of Z^{1a}, Z^{2a}, Z^{3a}, Z^{4a} and Z^{5a} is substituted with a linker.

Another particularly useful scaffold moiety for any chelator herein has the structure

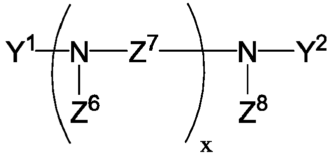

x is selected from 1, 2, 3 and 4. In exemplary embodiments, x is 1. In exemplary embodiments, x is 2. In exemplary embodiments, x is 3. In exemplary embodiments, x is 4.

Y¹ and Y² are each independently selected from H, substituted or unsubstituted alkyl and substituted or unsubstituted heteroalkyl. In exemplary embodiments, Y¹ and Y² are H.

Z⁷ is selected from substituted or unsubstituted alkyl and substituted or unsubstituted heteroalkyl. In exemplary embodiments, at least one Z⁷ is substituted with a linker. In some embodiments, each Z⁷ is independently substituted or unsubstituted (C₁, C₂, C₃, C₄, C₅ or C₆) alkyl. In exemplary embodiments, each Z⁷ is independently substituted or unsubstituted propyl or butyl. In some embodiments, each Z⁷ is independently substituted or unsubstituted heteroalkyl.

In exemplary embodiments, each Z⁷ is independently substituted or unsubstituted -(CH₂)ₘ(CH₂CH₂O)ₙ(CH₂)ₚ-, wherein m, n and p are integers independently selected from 1, 2, 3, 4, 5 and 6. In exemplary embodiments, each Z⁷ is substituted or unsubstituted -(CH₂)₂O(CH₂)₂-.

Z⁶ and Z⁸ are independently selected from -C(O)-, substituted or unsubstituted alkyl, and substituted or unsubstituted heteroalkyl; and each of Z⁶ and Z⁸ comprises a bond to one of the chelating moieties.

In exemplary embodiments, Z⁶ and Z⁸ are -C(O)-.

Another useful scaffold moiety has the structure: 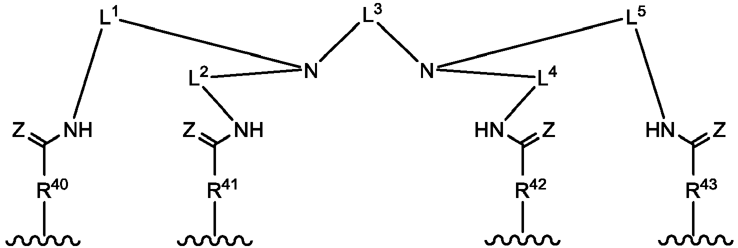 in which each Z is independently selected from O and S. In some embodiments, L³ comprises -(CH₂CH₂O)ₘR³¹- wherein m is an integer selected from 0, 1, 2, 3, 4, 5, 6, 7, 8 and 9. In some embodiments, m is 0. In some embodiments, m is 1. In some embodiments, L³ is -CH₂CH₂OCH₂CH₂-. L¹, L², L⁴, L⁵ and R³¹ are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. In exemplary embodiments, L¹, L², L⁴, L⁵ are independently selected substituted or unsubstituted (C₁, C₂, C₃, C₄, C₅ or C₆) alkyl. In some embodiments, R³¹ is substituted or unsubstituted (C₁, C₂, C₃, C₄, C₅ or C₆) alkyl. In exemplary embodiments, L¹, L², L⁴, L⁵ are independently selected substituted or unsubstituted ethyl. In some embodiments, R³¹ is substituted or unsubstituted ethyl. In exemplary embodiments, L¹, L², L⁴, L⁵ are ethyl, one or more of which is substituted with a linker. In some embodiments, L¹ is substituted with a linker. In some embodiments, L² is substituted with a linker. In some embodiments, L³ is substituted with a linker. In some embodiments, L⁴ is substituted with a linker. In some embodiments, L⁵ is substituted with a linker. In some embodiments, L¹ is ethyl substituted with a linker. In some embodiments, L² is ethyl substituted with a linker. In some embodiments, L³ is ethyl substituted with a linker. In some embodiments, L⁴ is ethyl substituted with a linker. In some embodiments, L⁵ is ethyl substituted with a linker. In some embodiments, R⁴⁰, R⁴¹, R⁴² and R⁴³ are bonds. In some embodiments, R⁴⁰, R⁴¹, R⁴² and R⁴³ are -(CH₂)_{w}O-, wherein w is selected from 0, 1, 2, 3, 4, 5, 6, 7, 8, 9 and 10. In exemplary embodiments, w is 3.

Another useful scaffold has the structure 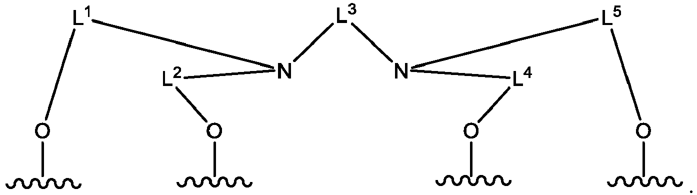 In some embodiments, L³ comprises -(CH₂CH₂O)ₘR³¹- wherein m is an integer selected from 0, 1, 2, 3, 4, 5, 6, 7, 8 and 9. In some embodiments, m is 0. In some embodiments, m is 1. In some embodiments, L³ is -CH₂CH₂OCH₂CH₂-. In some embodiments, L³ is -C(O)C(O)-. L¹, L², L⁴, L⁵ and R³¹ are independently selected from substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. In exemplary embodiments, L¹, L², L⁴, L⁵ are independently selected substituted or unsubstituted (C₁, C₂, C₃, C₄, C₅ or C₆) alkyl. In some embodiments, R³¹ is substituted or unsubstituted (C₁, C₂, C₃, C₄, C₅ or C₆) alkyl. In exemplary embodiments, L¹, L², L⁴, L⁵ are independently selected substituted or unsubstituted ethyl. In exemplary embodiments, L¹, L², L⁴, L⁵ are independently selected substituted or unsubstituted propyl. In some embodiments, R³¹ is substituted or unsubstituted ethyl. In exemplary embodiments, L¹, L², L⁴, L⁵ are ethyl, one or more of which is substituted with a linker. In some embodiments, L¹ is substituted with a linker. In some embodiments, L² is substituted with a linker. In some embodiments, L³ is substituted with a linker. In some embodiments, L⁴ is substituted with a linker. In some embodiments, L⁵ is substituted with a linker. In some embodiments, L¹ is propyl substituted with a linker. In some embodiments, L² is propyl substituted with a linker. In some embodiments, L³ is propyl substituted with a linker. In some embodiments, L⁴ is propyl substituted with a linker. In some embodiments, L⁵ is propyl substituted with a linker.

In some embodiments, a scaffold is selected from: 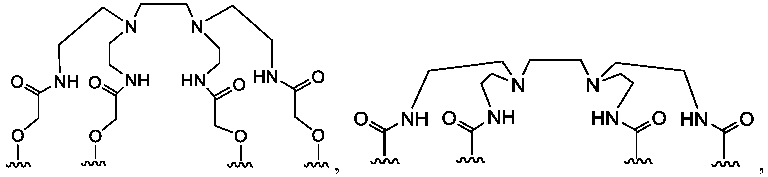 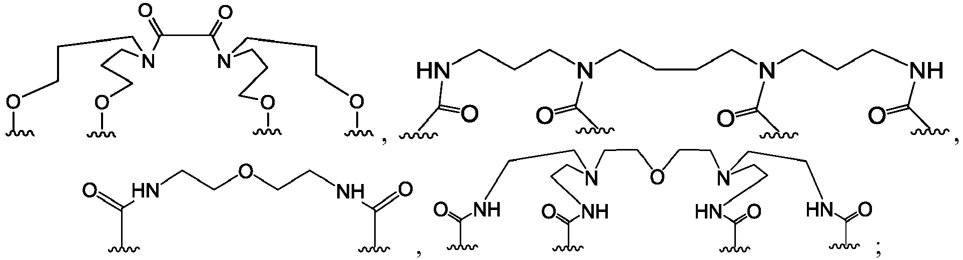 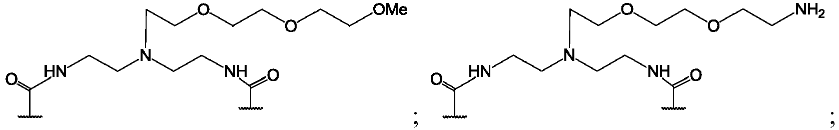 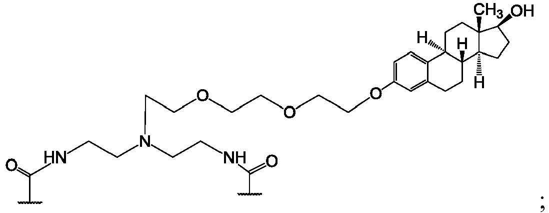 and 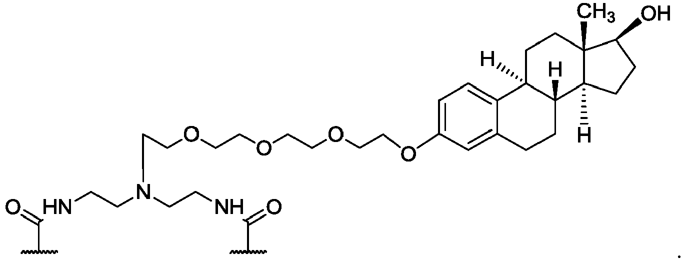 In any of these structures, one or more methyl, ethyl, propyl or butyl moieties can be substituted with one or more linkers. In some embodiments, two of these scaffold moieties, in which one or more methyl, ethyl, propyl or butyl moieties are optionally substituted with one or more linkers, are used to form a macrocycle.

In some embodiments, any one, two or all of the first, second and third scaffold moiety comprise a linker. In some embodiments, the linker is attached to a targeting moiety. In some embodiments, any one, two or all of the first, second and third scaffold moiety comprise a targeting moiety.

In some embodiments the first scaffold moiety is as defined for S¹ herein. In some embodiments, the second and third scaffold moieties are as defined for S² and S³ herein.

In some embodiments, any one, two or all of S¹, S² and S³ comprise a linker. In some embodiments, S¹ comprises a linker. In some embodiments, S² or S³ comprises a linker. In some embodiments, the linker is attached to a targeting moiety. In some embodiments, any one, two or all of S¹, S² and S³ comprise a targeting moiety. In some embodiments, S¹ comprises a targeting moiety. In some embodiments, S² or S³ comprises a targeting moiety.

### S¹

In some embodiments, S¹ has the structure: 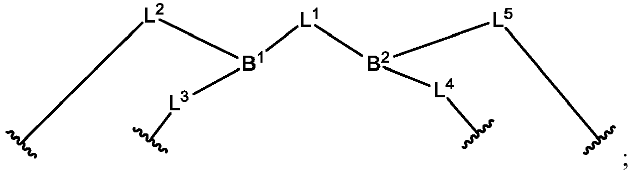 wherein
L¹, L², L³, L⁴, and L⁵ are independently selected from substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, L¹, L², L³, L⁴, and L⁵ are independently selected from substituted or unsubstituted alkyl and substituted or unsubstituted heteroalkyl.
In some embodiments, L¹ is 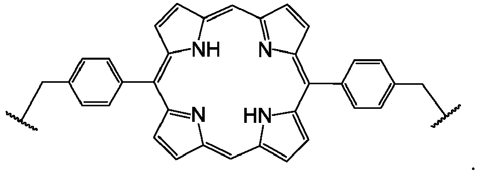 In some embodiments, one of L⁵ and L¹ is substituted with a linker. In some embodiments, L⁵ is substituted with a linker. Linkers are as defined herein.
B¹ and B² are independently selected from the elements capable of 3, 4, or 5 covalent bonds. In some embodiments, B¹ and B² are independently selected from N, C, B, Si, and P. In some embodiments, B¹ and B² are independently selected from N and C. In some embodiments, B¹ and B² are N.

In some embodiments, S¹ has the structure: 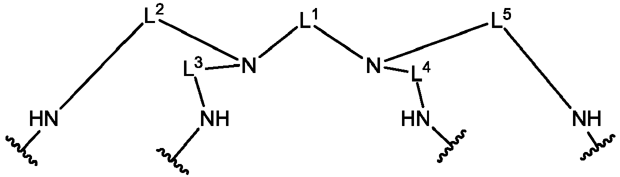 wherein L¹, L², L³, L⁴, and L⁵ are as defined herein.

In some embodiments S¹ has the structure: 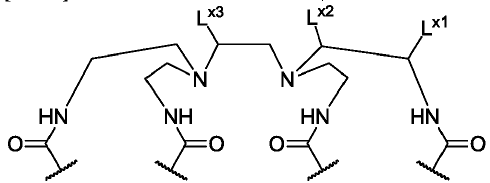 wherein L^{x1}, L^{x2} and L^{x3} are independently selected from H and a linker. In some embodiments, only one of L^{x1}, L^{x2} and L^{x3} is a linker. In some embodiments, L^{x1} is a linker. Linkers are as defined herein. In some embodiments, L^{x1}, L^{x2} and L^{x3} are H.

In some embodiments, S¹ has the structure: 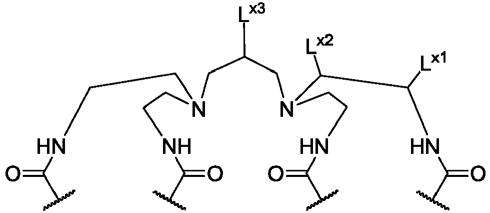 wherein L^{x1}, L^{x2} and L^{x3} are independently selected from H and a linker. In some embodiments, only one of L^{x1}, L^{x2} and L^{x3} is a linker. In some embodiments, L^{x3} is a linker. Linkers are as defined herein. In some embodiments, L^{x1}, L^{x2} and L^{x3} are H.

In some embodiments, S¹ has the structure:

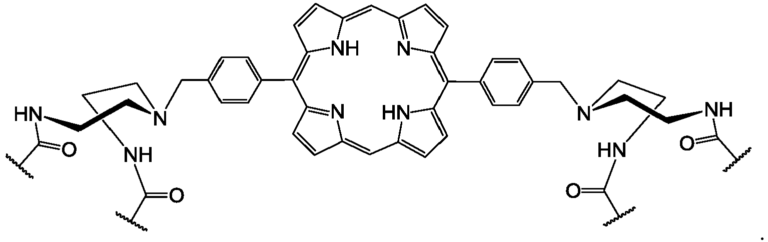

### S² and S³

In some embodiments, S² has the structure: 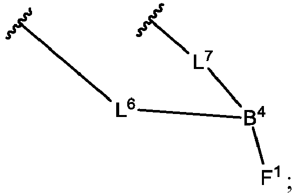 wherein
L⁶ and L⁷ are independently selected from substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, L⁶ and L⁷ are independently selected from substituted or unsubstituted alkyl and substituted or unsubstituted heteroalkyl.
In some embodiments, one of L⁶ and L⁷ is substituted with a linker. Linkers are as defined herein.
B⁴ is selected from the elements capable of 3, 4, or 5 covalent bonds. In some embodiments, B⁴ is selected from N, C, B, Si, and P. In some embodiments, B⁴ is selected from N and C. In some embodiments, B⁴ is N.
F¹ is selected from H, substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, F¹ is as defined herein.

In some embodiment S² has the structure: 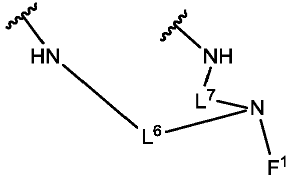 wherein L⁶, L⁷ and F¹ are as defined herein.

In some embodiment S² has the structure: 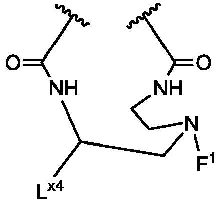 wherein L^{x4} is H or a linker. F¹ is as defined herein. Linkers are as defined herein.

In some embodiment S² has the structure: 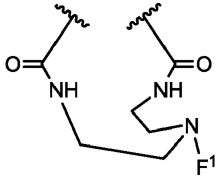 wherein F¹ is as defined herein.

In some embodiment S² has the structure: 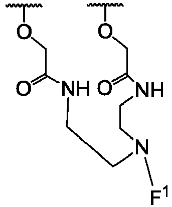 wherein F¹ is as defined herein.

In some embodiment S² has the structure: 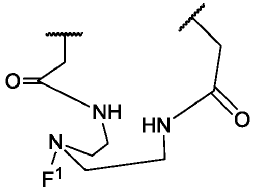 wherein F¹ is as defined herein.

In some embodiments, S³ has the structure: 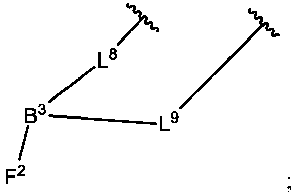 wherein
L⁸ and L⁹ are independently selected from substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, L⁸ and L⁹ are independently selected from substituted or unsubstituted alkyl and substituted or unsubstituted heteroalkyl.
In some embodiments, one of L⁸ and L⁹ is substituted with a linker. Linkers are as defined herein.
B³ is selected from the elements capable of 3, 4, or 5 covalent bonds. In some embodiments, B³ is selected from N, C, B, Si, and P. In some embodiments, B³ is selected from N and C. In some embodiments, B³ is N.
F² is selected from H, substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, F² is as defined herein.

In some embodiment S³ has the structure: 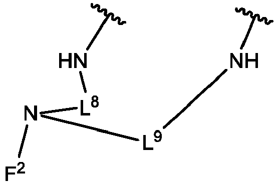 wherein L⁸, L⁹ and F² are as defined herein.

In some embodiment S³ has the structure: 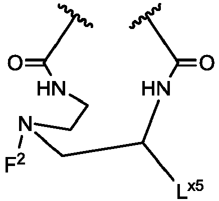 wherein L^{x5} is H or a linker. F² is as defined herein. Linkers are as defined herein.

In some embodiment S³ has the structure: 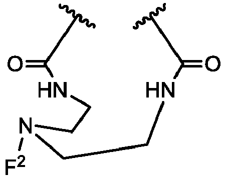 wherein F² is as defined herein.

In some embodiment S³ has the structure: 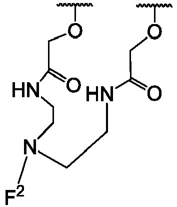 wherein F² is as defined herein.

In some embodiment S³ has the structure: 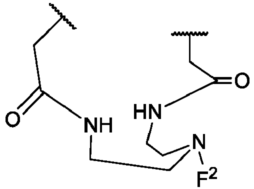 wherein F² is as defined herein.

In some embodiments, S² and S³ have the same structure.

### 2.1.3. Linker to Functional / Targeting Moiety

A "linker", "linking member", or "linking moiety" as used herein is a moiety that joins or potentially joins, covalently or noncovalently, a first moiety to a second moiety. In particular, a linker attaches or could potentially attach a chelator described herein to another molecule, such as a targeting moiety. In some embodiments, a linker attaches or could potentially attach a chelator described herein to a solid support. A linker comprising a reactive functional group that can be further reacted with a reactive functional group on a structure of interest in order to attach the structure of interest to the linker is referred to as a "functionalized linker". In exemplary embodiments, a linker is a functionalized linker. In exemplary embodiments, a chelator comprises one or more functionalized linkers. In some embodiments, a linker comprises a targeting moiety. In some embodiments, a linker to a targeting moiety comprises a bond to the targeting moiety.

A linker can be any useful structure for that joins a chelator to a reactive functional group or a targeting moiety, such as an antibody. Examples of a linker include 0-order linkers (i.e., a bond), substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. Further exemplary linkers include substituted or unsubstituted (C₁, C₂, C₃, C₄, C₅, C₆, C₇, C₈, C₉ or C₁₀) alkyl, substituted or unsubstituted heteroalkyl, -C(O)NR'-, -C(O)O-, -C(O)S-, and -C(O)CR'R", wherein R' and R" are members independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl. In some embodiments, a linker includes at least one heteroatom. Exemplary linkers also
include -C(O)NH-, -C(O), -NH-, -S-, -O-, and the like. In an exemplary embodiment, a linker is a heteroalkyl substituted with a reactive functional group.

### Reactive Functional Groups

In one embodiment, a linker comprises a reactive functional group (or a "reactive functional moiety", used synonymously), which can be further reacted to covalently attach the linker to a targeting moiety. Reactive functional groups and classes of reactions useful in practicing the present invention are generally those that are well known in the art of bioconjugate chemistry. Currently favored classes of reactions available with reactive functional groups of the invention are those which proceed under relatively mild conditions. These include, but are not limited to nucleophilic substitutions (e.g., reactions of amines and alcohols with acyl halides and activated esters), electrophilic substitutions (e.g., enamine reactions) and additions to carbon-carbon and carbon-heteroatom multiple bonds (e.g., Michael reactions and Diels-Alder reactions). These and other useful reactions are discussed, for example, in March, Advanced Organic Chemistry (3rd Ed., John Wiley & Sons, New York, 1985); Hermanson, Bioconjugate Techniques (Academic Press, San Diego, 1996); and Feeney et al., Modification of Proteins, Advances in Chemistry Series, Vol. 198 (American Chemical Society, Washington, D.C., 1982).

In some embodiments, a reactive functional group refers to a group selected from olefins, acetylenes, alcohols, phenols, ethers, oxides, halides, aldehydes, ketones, carboxylic acids, esters, amides, cyanates, isocyanates, thiocyanates, isothiocyanates, amines, hydrazines, hydrazones, hydrazides, diazo, diazonium, nitro, nitriles, mercaptans, sulfides, disulfides, sulfoxides, sulfones, sulfonic acids, sulfinic acids, acetals, ketals, anhydrides, sulfates, sulfenic acids isonitriles, amidines, imides, imidates, nitrones, hydroxylamines, oximes, hydroxamic acids thiohydroxamic acids, allenes, ortho esters, sulfites, enamines, ynamines, ureas, pseudoureas, semicarbazides, carbodiimides, carbamates, imines, azides, azo compounds, azoxy compounds, and nitroso compounds. Reactive functional groups also include those used to prepare bioconjugates, e.g., N-hydroxysuccinimide esters, maleimides and the like. Methods to prepare each of these functional groups are well known in the art and their application or modification for a particular purpose is within the ability of one of skill in the art (*see,* for example, Sandler and Karo, eds., Organic Functional Group Preparations, (Academic Press, San Diego, 1989)).

A reactive functional group can be chosen according to a selected reaction partner. As an example, an activated ester, such as an NHS ester will be useful to label a protein *via* lysine residues. Sulfhydryl reactive groups, such as maleimides can be used to label proteins *via* amino acid residues carrying an SH-group (e.g., cystein). Antibodies may be labeled by first oxidizing their carbohydrate moieties (e.g., with periodate) and reacting resulting aldehyde groups with a hydrazine containing ligand.

The reactive functional groups can be chosen such that they do not participate in, or interfere with, the reactions necessary to assemble the reactive ligand. Alternatively, a reactive functional group can be protected from participating in the reaction by means of a protecting group. Those of skill in the art understand how to protect a particular functional group so that it does not interfere with a chosen set of reaction conditions. For examples of useful protecting groups, see, for example, Greene et al., PROTECTIVE GROUPS IN ORGANIC SYNTHESIS, John Wiley & Sons, New York, 1991.

### Amines and Amino-Reactive Groups

In one embodiment, a reactive functional group is selected from an amine, (such as a primary or secondary amine), hydrazine, hydrazide and sulfonylhydrazide. Amines can, for example, be acylated, alkylated or oxidized. Useful non-limiting examples of amino-reactive groups include N-hydroxysuccinimide (NHS) esters, sulfur-NHS esters, imidoesters, isocyanates, isothiocyanates, acylhalides, arylazides, p-nitrophenyl esters, aldehydes, sulfonyl chlorides, thiazolides and carboxyl groups.

NHS esters and sulfur-NHS esters react preferentially with a primary (including aromatic) amino groups of a reaction partner. The imidazole groups of histidines are known to compete with primary amines for reaction, but the reaction products are unstable and readily hydrolyzed. The reaction involves the nucleophilic attack of an amine on the acid carboxyl of an NHS ester to form an amide, releasing the N-hydroxysuccinimide.

Imidoesters are the most specific acylating reagents for reaction with amine groups of a molecule such as a protein. At a pH between 7 and 10, imidoesters react only with primary amines. Primary amines attack imidates nucleophilically to produce an intermediate that breaks down to amidine at high pH or to a new imidate at low pH. The new imidate can react with another primary amine, thus crosslinking two amino groups, a case of a putatively monofunctional imidate reacting bifunctionally. The principal product of reaction with primary amines is an amidine that is a stronger base than the original amine. The positive charge of the original amino group is therefore retained. As a result, imidoesters do not affect the overall charge of the conjugate.

Isocyanates (and isothiocyanates) react with the primary amines of the conjugate components to form stable bonds. Their reactions with sulfhydryl, imidazole, and tyrosyl groups give relatively unstable products.

Acylazides are also used as amino-specific reagents in which nucleophilic amines of the reaction partner attack acidic carboxyl groups under slightly alkaline conditions, e.g. pH 8.5.

Arylhalides such as 1,5-difluoro-2,4-dinitrobenzene react preferentially with the amino groups and tyrosine phenolic groups of the conjugate components, but also with its sulfhydryl and imidazole groups.

p-Nitrophenyl esters of carboxylic acids are also useful amino-reactive groups. Although the reagent specificity is not very high, α- and ε-amino groups appear to react most rapidly.

Aldehydes react with primary amines of the conjugate components (*e.g*., ε-amino group of lysine residues). Although unstable, Schiff bases are formed upon reaction of the protein amino groups with the aldehyde. Schiff bases, however, are stable, when conjugated to another double bond. The resonant interaction of both double bonds prevents hydrolysis of the Schiff linkage. Furthermore, amines at high local concentrations can attack the ethylenic double bond to form a stable Michael addition product. Alternatively, a stable bond may be formed by reductive amination.

Aromatic sulfonyl chlorides react with a variety of sites of the conjugate components, but reaction with the amino groups is the most important, resulting in a stable sulfonamide linkage.

Free carboxyl groups react with carbodiimides, soluble in both water and organic solvents, forming pseudoureas that can then couple to available amines yielding an amide linkage. Yamada et al., Biochemistry, 1981, 20: 4836-4842, e.g., teach how to modify a protein with carbodiimides.

### Sulfhydryl and Sulfhydryl-Reactive Groups

In another embodiment, a reactive functional group is selected from a sulfhydryl group (which can be converted to disulfides) and sulfhydryl-reactive group. Useful non-limiting examples of sulfhydryl-reactive groups include maleimides, alkyl halides, acyl halides (including bromoacetamide or chloroacetamide), pyridyl disulfides, and thiophthalimides.

Maleimides react preferentially with the sulfhydryl group of the conjugate components to form stable thioether bonds. They also react at a much slower rate with primary amino groups and the imidazole groups of histidines. However, at pH 7 the maleimide group can be considered a sulfhydryl-specific group, since at this pH the reaction rate of simple thiols is 1000-fold greater than that of the corresponding amine.

Alkyl halides react with sulfhydryl groups, sulfides, imidazoles, and amino groups. At neutral to slightly alkaline pH, however, alkyl halides react primarily with sulfhydryl groups to form stable thioether bonds. At higher pH, reaction with amino groups is favored.

Pyridyl disulfides react with free sulfhydryl groups via disulfide exchange to give mixed disulfides. As a result, pyridyl disulfides are relatively specific sulfhydryl-reactive groups.

Thiophthalimides react with free sulfhydryl groups to also form disulfides.

### Other Reactive Functional Groups

Other exemplary reactive functional groups include:
(i) carboxyl groups and various derivatives thereof including, but not limited to, N-hydroxybenztriazole esters, acid halides, acyl imidazoles, thioesters, p-nitrophenyl esters, alkyl, alkenyl, alkynyl and aromatic esters;
(ii) hydroxyl groups, which can be converted to esters, ethers, aldehydes, etc.;
(iii) haloalkyl groups, wherein the halide can be displaced with a nucleophilic group such as, for example, an amine, a carboxylate anion, thiol anion, carbanion, or an alkoxide ion, thereby resulting in the covalent attachment of a new group at the site of the halogen atom;
(iv) dienophile groups, which are capable of participating in Diels-Alder reactions such as, for example, maleimido groups;
(v) aldehyde or ketone groups, such that subsequent derivatization is possible via formation of carbonyl derivatives such as, for example, imines, hydrazones, semicarbazones or oximes, or via such mechanisms as Grignard addition or alkyllithium addition;
(vi) alkenes, which can undergo, for example, cycloadditions, acylation, Michael addition, etc;
(vii) epoxides, which can react with, for example, amines and hydroxyl groups;
(ix) phosphoramidites and other standard functional groups useful in nucleic acid synthesis and
(x) any other functional group useful to form a covalent bond between the functionalized ligand and a molecular entity or a surface.

### Functional Groups with Non-specific Reactivities

In addition to the use of site-specific reactive moieties, the present invention contemplates the use of non-specific reactive groups to link a chelator to a targeting moiety. Non-specific groups include photoactivatable groups, for example.

Photoactivatable groups are ideally inert in the dark and are converted to reactive species in the presence of light. In one embodiment, photoactivatable groups are selected from precursors of nitrenes generated upon heating or photolysis of azides. Electron-deficient nitrenes are extremely reactive and can react with a variety of chemical bonds including N-H, O-H, C-H, and C=C. Although three types of azides (aryl, alkyl, and acyl derivatives) may be employed, arylazides are presently preferrred. The reactivity of arylazides upon photolysis is better with N-H and O-H than C-H bonds. Electron-deficient arylnitrenes rapidly ring-expand to form dehydroazepines, which tend to react with nucleophiles, rather than form C-H insertion products. The reactivity of arylazides can be increased by the presence of electron-withdrawing substituents such as nitro or hydroxyl groups in the ring. Such substituents push the absorption maximum of arylazides to longer wavelength. Unsubstituted arylazides have an absorption maximum in the range of 260-280 nm, while hydroxy and nitroarylazides absorb significant light beyond 305 nm. Therefore, hydroxy and nitroarylazides are most preferable since they allow to employ less harmful photolysis conditions for the affinity component than unsubstituted arylazides.

In another preferred embodiment, photoactivatable groups are selected from fluorinated arylazides. The photolysis products of fluorinated arylazides are arylnitrenes, all of which undergo the characteristic reactions of this group, including C-H bond insertion, with high efficiency (Keana et al., J. Org. Chem. 55: 3640-3647, 1990).

In another embodiment, photoactivatable groups are selected from benzophenone residues. Benzophenone reagents generally give higher crosslinking yields than arylazide reagents.

In another embodiment, photoactivatable groups are selected from diazo compounds, which form an electron-deficient carbene upon photolysis. These carbenes undergo a variety of reactions including insertion into C-H bonds, addition to double bonds (including aromatic systems), hydrogen attraction and coordination to nucleophilic centers to give carbon ions.

In still another embodiment, photoactivatable groups are selected from diazopyruvates. For example, the p-nitrophenyl ester of p-nitrophenyl diazopyruvate reacts with aliphatic amines to give diazopyruvic acid amides that undergo ultraviolet photolysis to form aldehydes. The photolyzed diazopyruvate-modified affinity component will react like formaldehyde or glutaraldehyde forming intraprotein crosslinks.

In exemplary embodiments, a linker joins a chelator to a targeting moiety. That is, in exemplary embodiments, a linker comprises a targeting moiety. In some embodiments, a chelator comprises a linker to a targeting moiety. Any linker described herein may be a linker comprising a reactive functional group that could react with a reactive functional group on a targeting moiety to join the linker to the targeting moiety. Any linker described herein may be a linker comprising a bond to a targeting moiety. The term "targeting moiety" refers to a moiety serves to target or direct the molecule to which it is attached (e.g., a chelator or a chelator complexed to a metal ion (such as a radionuclide)) to a particular location or molecule. Thus, for example, a targeting moiety may be used to target a molecule to a specific target protein or enzyme, or to a particular cellular location, to a particular cell type or to a diseased tissue. As will be appreciated by those in the art, the localization of proteins within a cell is a simple method for increasing effective concentration. For example, shuttling an imaging agent and/or therapeutic into the nucleus confines them to a smaller space thereby increasing concentration. Finally, the physiological target may simply be localized to a specific compartment, and the agents must be localized appropriately.

The targeting moiety can be a small molecule (e.g., MW < 500D), which includes both non-peptides and peptides. Examples of a targeting moiety also include peptides, polypeptides (including proteins, and in particular antibodies, which includes antibody fragments), nucleic acids, oligonucleotides, carbohydrates, lipids, hormones (including proteinaceous and steroid hormones), growth factors, lectins, receptors, receptor ligands, cofactors and the like. Targets of a targeting moiety can include a complementary nucleic acid, a receptor, an antibody, an antigen or a lectin, for example.

In exemplary embodiments, a targeting moiety can bind to a target with high binding affinity. In other words, a targeting moiety with high binding affinity to a target has a high specificity for or specifically binds to the target. In some embodiments, a high binding affinity is given by a dissociation constant K_{d} of about 10⁻⁷ M or less. In exemplary embodiments, a high binding affinity is given by a dissociation constant K_{d} of about 10⁻⁸ M or less, about 10⁻⁹ M or less, about 10⁻¹⁰ M or less, about 10⁻¹¹ M or less, about 10⁻¹² M or less, about 10⁻¹³ M or less, about 10⁻¹⁴ M or less or about 10⁻¹⁵ M or less. A compound may have a high binding affinity for a target if the compound comprises a portion, such as a targeting moiety, that has a high binding affinity for the target.

In exemplary embodiments, a targeting moiety is an antibody. An "antibody" refers to a protein comprising one or more polypeptides substantially encoded by all or part of the recognized immunoglobulin genes. The recognized immunoglobulin genes, for example in humans, include the kappa (κ), lambda (λ) and heavy chain genetic loci, which together compose the myriad variable region genes, and the constant region genes mu (µ), delta (δ), gamma (γ), epsilon (ε) and alpha (α), which encode the IgM, IgD, IgG, IgE, and IgA isotypes respectively. Antibody herein is meant to include full length antibodies and antibody fragments, and may refer to a natural antibody from any organism, an engineered antibody or an antibody generated recombinantly for experimental, therapeutic or other purposes as further defined below. Antibody fragments include Fab, Fab', F(ab')₂, Fv, scFv or other antigen-binding subsequences of antibodies and can include those produced by the modification of whole antibodies or those synthesized de novo using recombinant DNA technologies. The term "antibody" refers to both monoclonal and polyclonal antibodies. Antibodies can be antagonists, agonists, neutralizing, inhibitory or stimulatory.

While a targeting moiety may be appended to a chelator in order to localize the compound to a specific region in an animal, certain chelators have a natural affinity for cells, tissue, organs or some other part of the animal. For example, a chelator disclosed herein might have a natural or intrinsic affinity for bone. Thus, in some embodiments, a chelator (di-macrocycle), does not comprise a targeting moiety or a linker to a targeting moiety. A chelator lacking a targeting moiety can be used in any method that does not require specific targeting.

In some embodiments, a chelator comprises a linker to a solid support. That is, any linker described herein may be a linker comprising a reactive functional group that could react with a reactive functional group on a solid support to join the linker to the solid support. Any linker described herein may be a linker comprising a bond to a solid support. A "solid support" is any material that can be modified to contain discrete individual sites suitable for the attachment or association of a chelator. Suitable substrates include biodegradable beads, non-biodegradable beads, silica beads, magnetic beads, latex beads, glass beads, quartz beads, metal beads, gold beads, mica beads, plastic beads, ceramic beads, or combinations thereof. Of particular use are biocompatible polymers, including biodegradable polymers that are slowly removed from the system by enzymatic degradation. Example biodegradable materials include starch, cross-linked starch, poly(ethylene glycol), polyvinylpyrrolidine, polylactides (PLA), polyglycolides (PGA), poly(lactide-co-glycolides) (PLGA), polyanhydrides, polyorthoesters, poly(DTH iminocarbonate), poly(bisphenol A iminocarbonate), polycyanoacrylate, polyphosphazene, mixtures thereof and combinations thereof. Other suitable substances for forming the particles exist and can be used. In some embodiments, a solid support is a bead comprising a cross-linked starch, for example, cross-linked potato starch. Beads made from starch are completely biodegradable in the body, typically by serum amylase, a naturally occurring enzyme found in the body. In these embodiments, the chelator optionally further comprises a targeting moiety or a linker to a targeting moeity. In cases where a chelator that is attached to a solid support does not comprise a targeting moiety, the chealtor can be localized directly by the practitioner, for example, by direct surgical implantation.

In some embodiments, a linker has the structure -L¹¹-X, wherein L¹¹ is selected from a bond, acyl, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl; and X is a reactive functional group or a targeting moiety.

In some embodiments, L¹¹ is selected from substituted or unsubstituted alkyl and substituted or unsubstituted heteroalkyl. In some embodiments, L¹¹ is heteroalkyl. In some embodiments, L¹¹ is (C₁, C₂, C₃, C₄, C₅, C₆, C₇, C₈, C₉, C₁₀, C₁₁, C₁₂, C₁₃, C₁₄, C₁₅, C₁₆, C₁₇, C₁₈, C₁₉ or C₂₀) alkyl in which 1, 2 or 3 atoms are replaced with a heteroatom, such as nitrogen or oxygen.

In some embodiments, X is selected from -NH₂ and -CO(O)H.

In some embodiments, -L¹¹-X is selected from

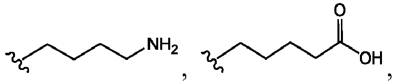

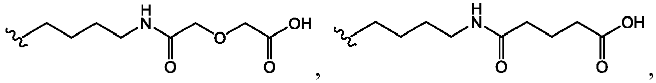

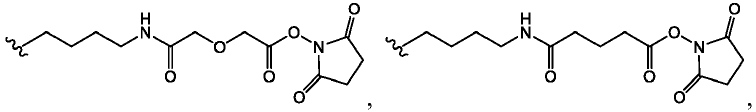

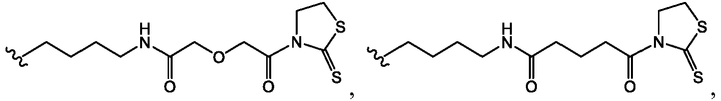

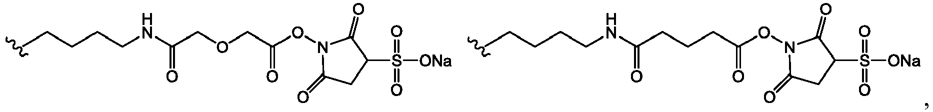

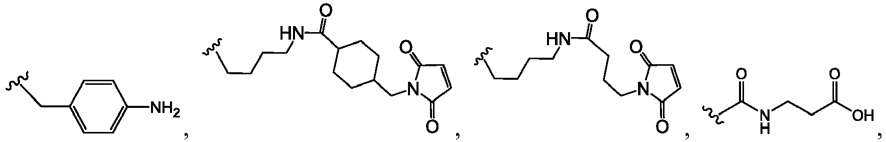

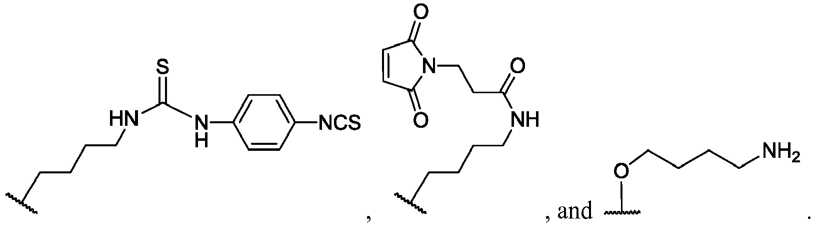

In exemplary embodiments, X is a targeting moiety.

In exemplary embodiments, a linker is a linker to a targeting moiety. In some embodiments, the targeting moiety is selected from a polypeptide, a nucleic acid, a lipid, a polysaccharide, a small molecule, a cofactor and a hormone. In exemplary embodiments, the targeting moiety is an antibody or antibody fragment.

In some embodiments, a linker includes an aliphatic carbon chain or a polyethyleneglycol (PEG) chain. Thus, a linker can comprise a structure selected from: 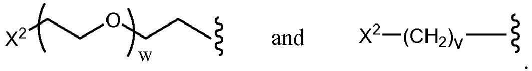 The integer v is selected from 1 to 20, and w is an integer from 1 to 1,000 or 1 to 500 or 1 to 100 or 1 to 50 or 1 to 10.

Exemplary X² groups include OH, alkoxy, and one of the following structures: 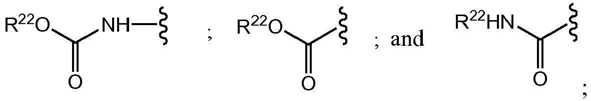 wherein R²² is a member selected from H, substituted or unsubstituted alkyl, substituted or unsubstituted heteroalkyl, substituted or unsubstituted aryl, substituted or unsubstituted heteroaryl and substituted or unsubstituted heterocycloalkyl. The integer v is selected from 1 to 20, and w is an integer from 1 to 1,000 or 1 to 500 or 1 to 100 or 1 to 50 or 1 to 10.

In some embodiments, a linker has the structure: 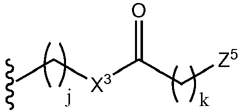 wherein Z⁵ is selected from H, OR²³, SR²³, NHR²³, OCOR²⁴, OC(O)NHR²⁴, NHC(O)OR²³, OS(O)₂OR²³, and C(O)R²⁴. R²³ is selected from H, substituted or unsubstituted alkyl, and substituted or unsubstituted heteroalkyl. R²⁴ is selected from H, OR²⁵, NR²⁵NH₂, SH, C(O)R²⁵, NR²⁵H, substituted or unsubstituted alkyl and substituted or unsubstituted heteroalkyl. R²⁵ is selected from H, substituted or unsubstituted alkyl and substituted or unsubstituted alkyl. X³ is selected from O, S and NR²⁶, wherein R²⁶ is a member selected from H, substituted or unsubstituted alkyl and substituted or unsubstituted heteroalkyl. The integers j and k are members independently selected from 1, 2, 3, 4, 5, 6, 7, 8, 9, 10, 11, 12, 13, 14, 15, 16, 17, 18, 19 and 20. In some embodiments, the integers j and k are members independently selected from 1, 2,3,4,5,6.

In a linker with multiple reactive functional groups, a particular functional group can be chosen such that it does not participate in, or interfere with, the reaction controlling the attachment of the functionalized spacer component to another ligand component. Alternatively, the reactive functional group can be protected from participating in the reaction by the presence of a protecting group. Those of skill in the art understand how to protect a particular functional group from interfering with a chosen set of reaction conditions. For examples of useful protecting groups, *See* Greene et al., PROTECTIVE GROUPS IN ORGANIC SYNTHESIS, John Wiley & Sons, New York, 1991.

### 2.1.4. Modifying Moiety

In some embodiments, one, two or all of S¹, S² and S³ comprise a modifying moiety. Each of the modifying moieties can be the same or different.
The modifying moiety modifies various properties of the di-macrocycle and/or a complex formed between the di-macrocycle and a metal ion, such as solubility, charge, or affinity. In some embodiments, the modifying moiety does not interact with the metal when the di-macrocycle is complexed to a metal. In some embodiments, the modifying moiety is a solubilizing group, an estradiol-derived moiety, a prodrug moiety (for example, with a cleavable moiety), an oligonucleotide, ssDNA, dsDNA, RNA, or a peptide. The solubilizing group improves solubility of the di-macrocycle and/or a complex formed between the di-macrocycle and a metal ion in aqueous media.

In some embodiments, S² and S³ comprise a modifying moiety. In some embodiments, S¹ comprises a linker; and S² and S³ comprise a modifying moiety. In some embodiments, S² and S³ comprise a modifying moiety; and S² or S³ further comprises a linker.

In some embodiments, F¹, F² or both comprise a modifying moiety. In some embodiments, F¹ and F² comprise a modifying moiety.
In some embodiments, F¹, F² or both are modifying moieties. In some embodiments, F¹ and F² are modifying moieties.

In some embodiments, F¹, F² or both are substituted or unsubstituted heteroalkyl. In some embodiments, F¹, F² or both are a substituted or unsubstituted polyether. In some embodiments, F¹, F² or both comprise an estradiol-derived moiety. In some embodiments, F¹, F² or both are a polyether substituted with an estradiol-derived moiety.
In some embodiments, F¹, F² or both are members independently selected from: 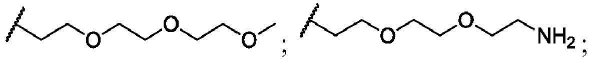 and 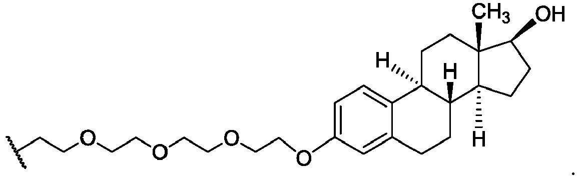 In some embodiments, F¹, F² or both are a peptide. In some embodiments, F¹, F² or both are 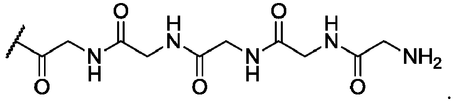 In some embodiments, F¹, F² or both comprise an oligunucleotide. In some embodiments, F¹ and F² each comprise an oligonucleotide. In some embodiments, the oligonucleotide of F¹ is complementary to the oligonucleotide of F².
In some embodiments, F¹ and F² are the same.

In some embodiments, F¹, F² or both are a linker.

### 2.1.5. Exemplary Di-Macrocycles

The invention provides a di-macrocycle having the structure: 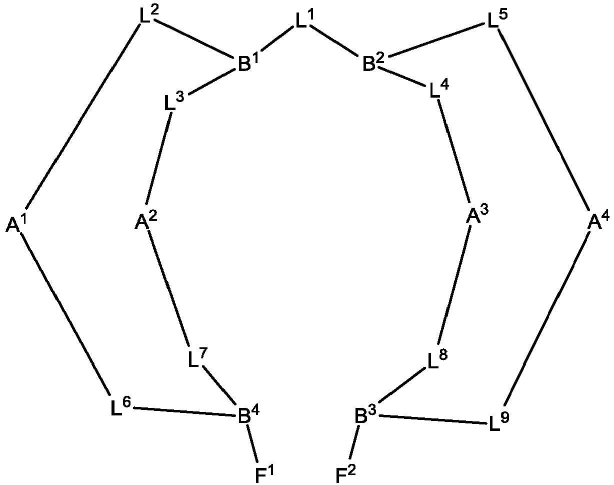 as defined herein. In some embodiments, F¹ and F² are modifying moieties. Modifying moieties are as defined herein.
In some embodiments, the di-macrocycle is covalently modified with at least one linker. In some embodiments, one of L¹, L², L³, L⁴, L⁵, L⁶, L⁷, L⁸, and L⁹ is substituted with a linker. In some embodiments, L⁵ is substituted with a linker. In some embodiments, L¹ is substituted with a linker. In some embodiments, one of L⁶, L⁷, L⁸ and L⁹ is substituted with a linker.

The invention provides a di-macrocycle having the structure: 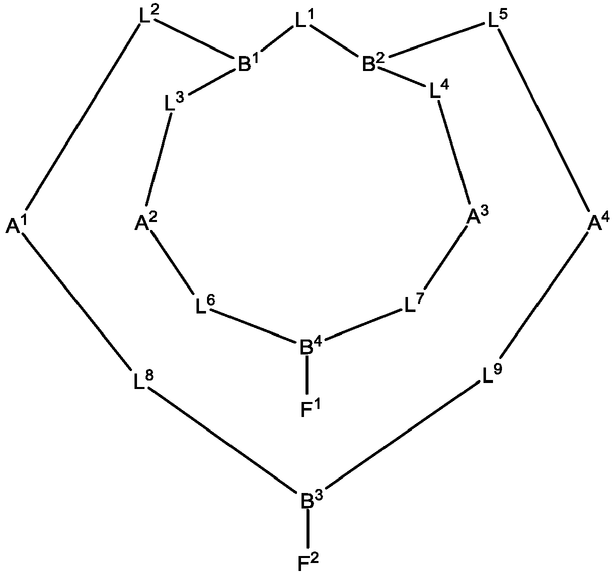 as defined herein.
In some embodiments, F¹ and F² are modifying moieties. Modifying moieties are as defined herein.
In some embodiments, the di-macrocycle is covalently modified with at least one linker. In some embodiments, one of L¹, L², L³, L⁴, L⁵, L⁶, L⁷, L⁸, and L⁹ is substituted with a linker. In some embodiments, L⁵ is substituted with a linker. In some embodiments, L¹ is substituted with a linker. In some embodiments, one of L⁶, L⁷, L⁸ and L⁹ is substituted with a linker.

The disclosure provide a di-macrocycle having the structure: 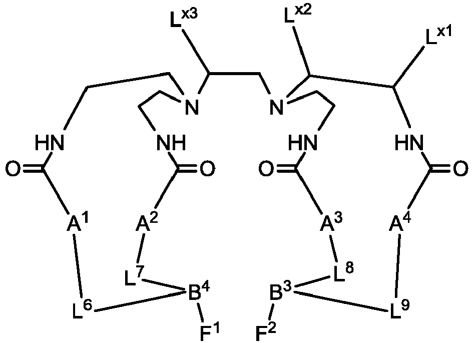 wherein
B³ and B⁴ are independently selected from N and C;
F¹ and F² are independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl;
L⁶, L⁷, L⁸, and L⁹ are independently selected from substituted or unsubstituted alkyl, and substituted or unsubstituted heteroalkyl;
A¹, A², A³ and A⁴ are members independently selected from: 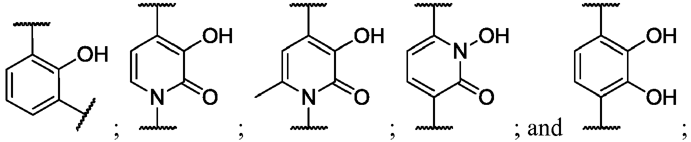 and
L^{x1}, L^{x2} and L^{x3} are independently selected from H and a linker.
In some embodiments, F¹ and F² are modifying moieties. Modifying moieties are as defined herein.
In some embodiments, the di-macrocycle is covalently modified with at least one linker. In some embodiments, one of L^{x1}, L^{x2}, and L^{x3} is a linker. In some embodiments, L^{x1} is a linker. In some embodiments, one of L⁶, L⁷, L⁸ and L⁹ is substituted with a linker.

The disclosure provide a di-macrocycle having the structure: 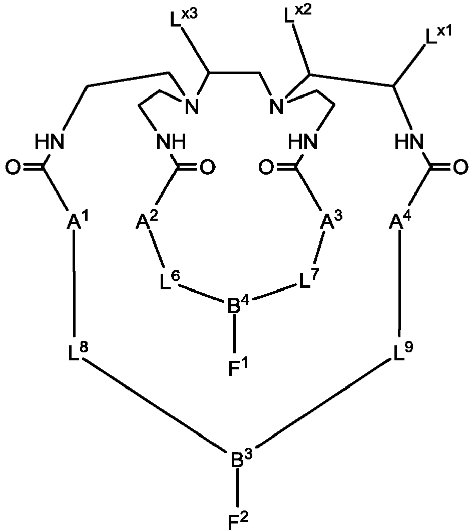 wherein
B³ and B⁴ are independently selected from N and C;
F¹ and F² are independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl;
L⁶, L⁷, L⁸, and L⁹ are independently selected from substituted or unsubstituted alkyl, and substituted or unsubstituted heteroalkyl;
A¹, A², A³ and A⁴ are members independently selected from: 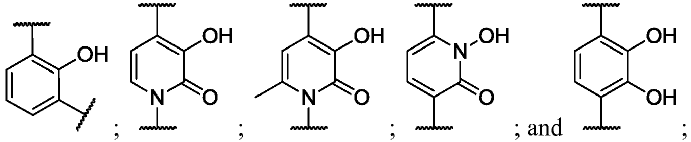 and
L^{x1}, L^{x2} and L^{x3} are independently selected from H and a linker.
In some embodiments, F¹ and F² are modifying moieties. Modifying moieties are as defined herein.
In some embodiments, the di-macrocycle is covalently modified with at least one linker. In some embodiments, one of L^{x1}, L^{x2}, and L^{x3} is a linker. In some embodiments, L^{x1} is a linker. In some embodiments, one of L⁶, L⁷, L⁸ and L⁹ is substituted with a linker.

The disclosure provides a di-macrocycle having the structure: 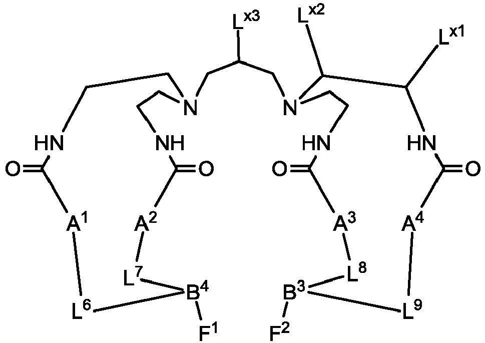 wherein
B³ and B⁴ are independently selected from N and C;
F¹ and F² are independently selected from H, substituted or unsubstituted alkyl, substituted or unsubstituted heteroalkyl, substituted or unsubstituted aryl, substituted or unsubstituted heteroaryl and substituted or unsubstituted heterocycloalkyl;
L⁶, L⁷, L⁸, and L⁹ are independently selected from substituted or unsubstituted alkyl, and substituted or unsubstituted heteroalkyl;
A¹, A², A³ and A⁴ are members independently selected from: 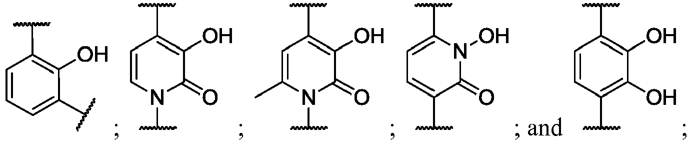 and
L^{x1}, L^{x2} and L^{x3} are independently selected from H and a linker.
In some embodiments, F¹ and F² are modifying moieties. Modifying moieties are as defined herein.
In some embodiments, the di-macrocycle is covalently modified with at least one linker. In some embodiments, one of L^{x1}, L^{x2}, and L^{x3} is a linker. In some embodiments, L^{x3} is a linker. In some embodiments, one of L⁶, L⁷, L⁸ and L⁹ is substituted with a linker.

The disclosure provides a di-macrocycle having the structure: 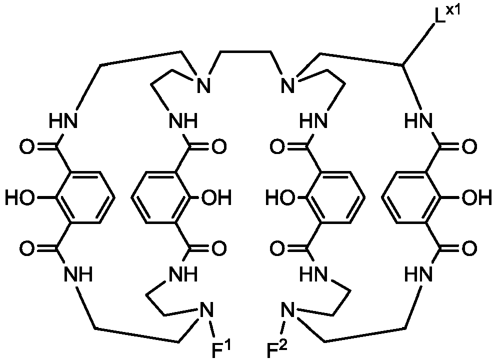 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 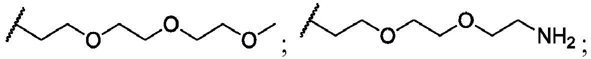 and 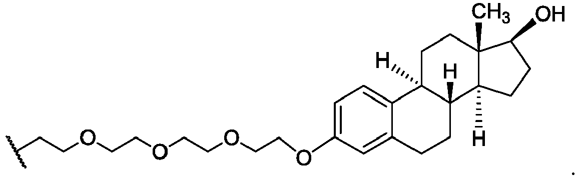 In some embodiments, F¹ and F² are the same. In some embodiments, F¹ and F² are different.

The disclosure provides a di-macrocycle having the structure: 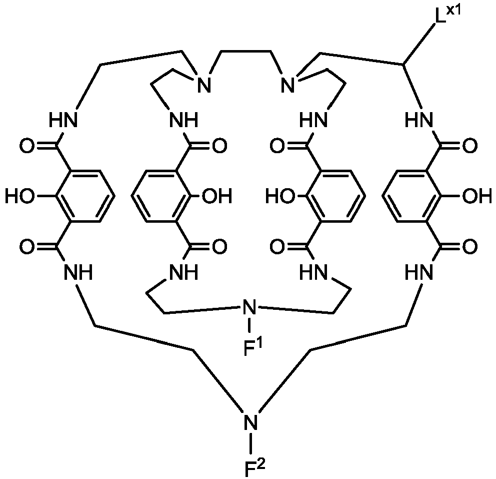 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 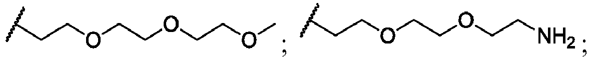 and 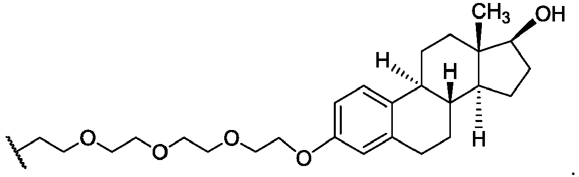 In some embodiments, F¹ and F² are the same. In some embodiments, F¹ and F² are different.

The disclosure provides a di-macrocycle having the structure: 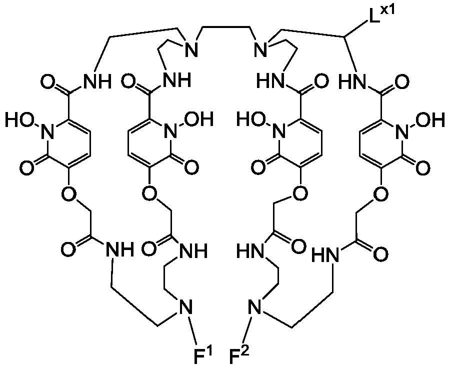 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 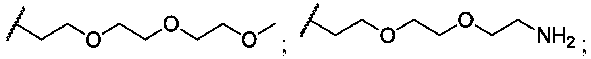 and 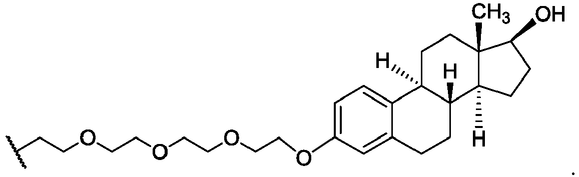 In some embodiments, F¹ and F² are the same. In some embodiments, F¹ and F² are different.

The disclsoure provides a di-macrocycle having the structure: 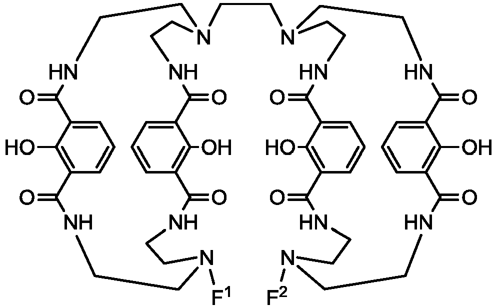 wherein F¹ and F² are modifying moieties.
In some embodiments, F¹ and F² are peptides. In some embodiments, F¹ and F² are the same. In some embodiments, F¹ and F² are each 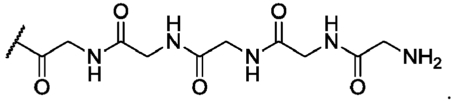 In some embodiments, F¹ and F² are different.
In some embodiments, F¹ and F² each comprise an oligonucleotide. In some embodiments, the oligonucleotide of F¹ is complementary to the oligonucleotide of F².

The disclosure provides a di-macrocycle having the structure: 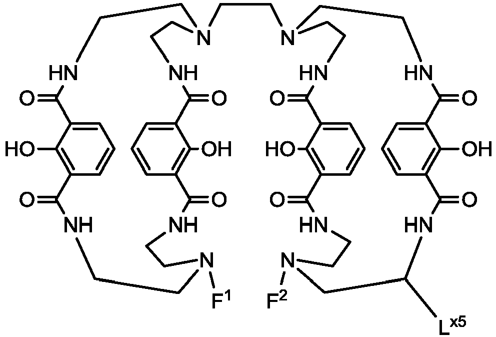 wherein L^{x5} is a linker; and F¹ and F² are modifying moieties.
Linkers are as defined herein.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 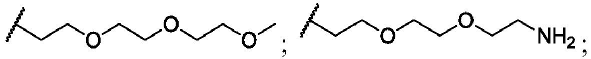 and 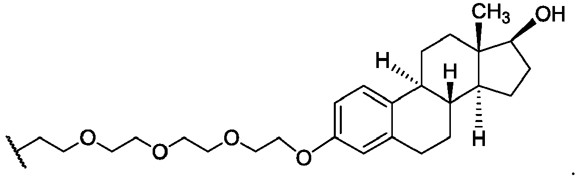 In some embodiments, F¹ and F² are the same. In some embodiments, F¹ and F² are different.

The disclosure provides a di-macrocycle having the structure: 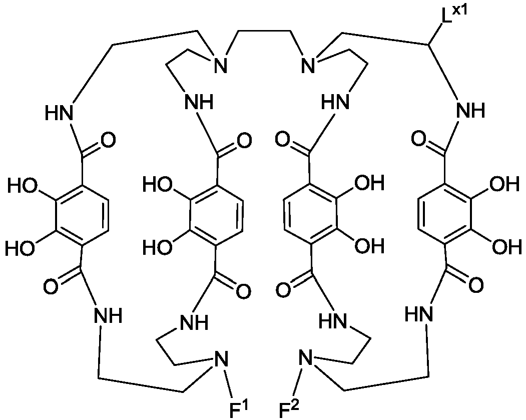 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 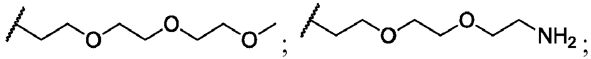 and 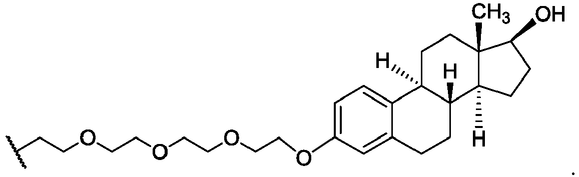 In some embodiments, F¹ and F² are the same. In some embodiments, F¹ and F² are different.

The disclosure provides a di-macrocycle having the structure: 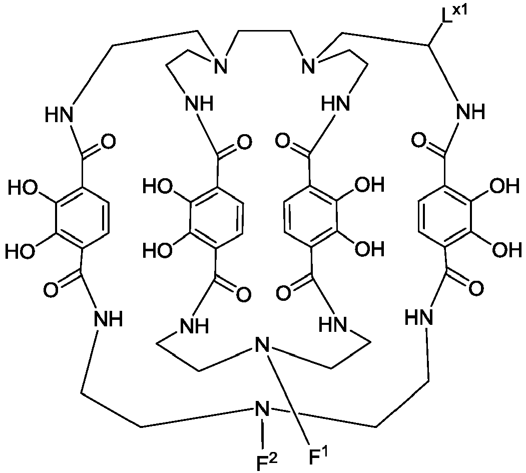 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 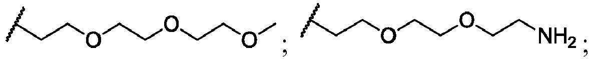 and 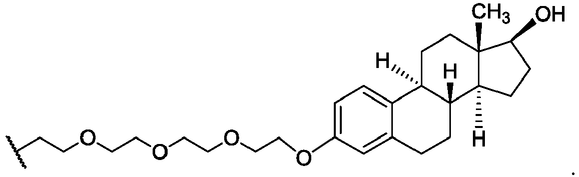 In some embodiments, F¹ and F² are the same. In some embodiments, F¹ and F² are different.

The disclosure provides a di-macrocycle having the structure: 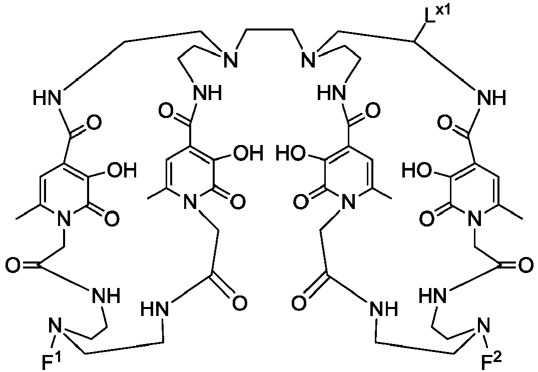 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 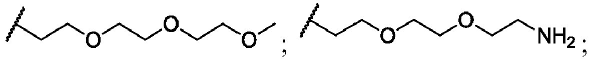 and 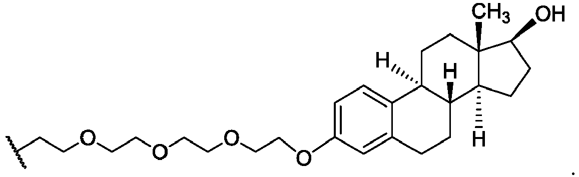 In some embodiments, F¹ and F² are the same. In some embodiments, F¹ and F² are different.

The disclosure provides a di-macrocycle having the structure: 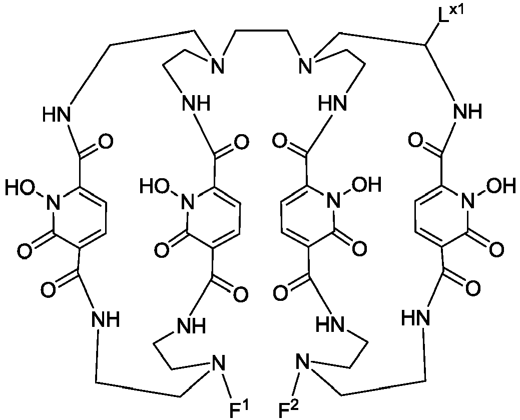 wherein L^{x1} is H or a linker; and F¹ and F² are modifying moieties.
Linkers are as defined herein.
In some embodiments, L^{x1} is H. In some embodiments, L^{x1} is a linker.
In some embodiments, F¹ and F² are substituted or unsubstituted heteroalkyl. In some embodiments, F¹ and F² are a substituted or unsubstituted polyether. In some embodiments, F¹ and F² are a polyether substituted with an estradiol-derived moiety.
In some embodiments, F¹ and F² are members independently selected from: 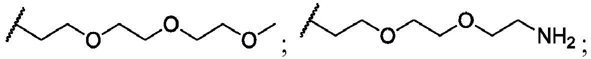 and 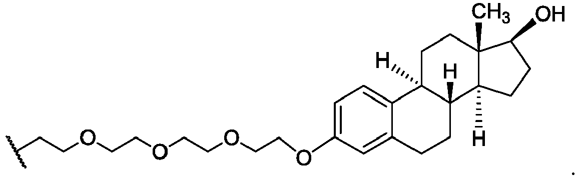 In some embodiments, F¹ and F² are the same. In some embodiments, F¹ and F² are different.

Additional exemplary di-macrocycles are shown in the Examples.

### 2.2. Complexes

In one aspect, the invention provides a complex of a di-macrocycle disclosed herein with a metal ion.

Any of the combinations of di-macrocycles disclosed herein and a metal ion disclosed herein are encompassed by this disclosure and specifically provided by the invention.

### 2.2.1. Metals

In some embodiments, the metal is an actinide. In some embodiments, the metal is a lanthanide. In some embodiments, the lanthanide is Tb. In some embodiments, the lanthanide is Eu.
In some embodiments, the metal ion is yttrium(III). In some embodiments, the metal ion is europium(III). In some embodiments, the metal ion is terbium(III). In some embodiments, the metal ion is zirconium(IV). In some embodiments, the metal ion is thorium(IV).
In some embodiments, the metal is a radionuclide. In some embodiments, the metal ion is ²²⁷Th(IV). In some embodiments, the metal ion is ⁸⁹Zr(IV).

In some embodiments, the metal is ¹⁷⁷Lu. In some embodiments, the metal is ¹⁶⁶Ho. In some embodiments, the metal is ¹⁵³Sm. In some embodiments, the metal is ⁹⁰Y. In some embodiments, the metal is ⁸⁶Y. In some embodiments, the metal is ¹⁶⁶Dy. In some embodiments, the metal is ¹⁶⁵Dy. In some embodiments, the metal is ¹⁶⁹Er. In some embodiments, the metal is ¹⁷⁵Yb. In some embodiments, the metal is ²²⁵Ac. In some embodiments, the metal is ¹⁴⁹Tb. In some embodiments, the metal is ¹⁵³Gd. In some embodiments, the metal is ²³⁰U.

In some embodiments, the metal is ¹¹¹In. In some embodiments, the metal is ⁶⁷Ga. In some embodiments, the metal is ⁶⁷Cu. In some embodiments, the metal is ⁶⁴Cu. In some embodiments, the metal is ¹⁸⁶Re. In some embodiments, the metal is ¹⁸⁸Re. In some embodiments, the metal is ¹¹¹Ag. In some embodiments, the metal is ¹⁰⁹Pd. In some embodiments, the metal is ²¹²Pb. In some embodiments, the metal is ²⁰³Pb. In some embodiments, the metal is ²¹²Bi. In some embodiments, the metal is ²¹³Bi. In some embodiments, the metal is ^{195m}Pt. In some embodiments, the metal is ²⁰¹Tl. In some embodiments, the metal is ⁵⁵Co. In some embodiments, the metal is ^{99m}Tc.

### 2.2.1.1. Radionuclides

The chelating moieties disclosed herein can be used to bind metal ions, in particular, a radionuclide. The term "radionuclide" or "radioisotope" refers to a radioactive isotope or element with an unstable nucleus that tends to undergo radioactive decay. Numerous decay modes are known in the art and include alpha decay, proton emission, neutron emission, double proton emission, spontaneous fission, cluster decay, β⁻ decay, positron emission (β⁺ decay), electron capture, bound state beta decay, double beta decay, double electron capture, electron capture with positron emission, double positron emission, isomeric transition and internal conversion.

Exemplary radionuclides include alpha-emitters, which emit alpha particles during decay. In some embodiments, a radionuclide is an emitter of a gamma ray or a particle selected from an alpha particle, an electron and a positron.

In some embodiments, the radionuclide is an actinide. In some embodiments, the radionuclide is a lanthanide. In some embodiments, the radionuclide is a 3⁺ ion. In some embodiments, the radionuclide is a 4⁺ ion. In some embodiements the radionuclide is a 2⁺ ion.

Of particular use in the complexes provided herein are radionuclides selected from isotopes of U, Pu, Fe, Cu, Sm, Gd, Tb, Dy, Ho, Er, Yb, Lu, Y, Th, Zr, In, Ga, Bi, Ra, At and Ac. In some embodiments, a radionuclide is selected form radium-223, thorium-227, astatine-211, bismuth-213, Lutetium-177, and actinium-225. Other useful radioisotopes include bismuth-212, iodine-123, copper-64, iridium-192, osmium-194, rhodium-105, samarium-153, and yttrium-88, yttrium-90, and yttrium-91. In exemplary embodiments, the radionuclide is thorium, particularly selected from thorium-227 and thorium-232. In some embodiments, thorium-226 is excluded. In some embodiments, U is excluded. In some embodiments, uranium-230 is excluded. That is, in some embodiments, a radionuclide is not U, or a radionuclide is not uranium-230 or a radionuclide is not thorium-226.

²³²Th exists in nature as an α-emitter with a half life of 1.4 x 10¹⁰ yr. In aqueous solution, Th(IV) is the only oxidation state. Thorium(IV) ion is bigger than Pu(IV) and usually forms complexes with 9 or higher coordination number. For example, the crystal structure of both Th(IV) complexes of simple bidentate 1,2-HOPO and Me-3,2-HOPO have been determined as nine coordinated species.

Similar to other actinide ions, thorium(IV) prefers forming complexes with oxygen, especially negative oxygen donor ligands. Thorium(IV) also prefers octadentate or higher multidentate ligands:

| Ligand | **Acac** | **NTA** | **HEDTA*** | **EDTA**** | **DTPA** | **TTHA** |
|---|---|---|---|---|---|---|
| Ligand Type | Bi-dentate | Tetra- | Hexa- | Hexa- | Octa- | Deca- |
| **Log K₁** | **7.85** | **16.9** | **18.5** | **25.3** | **30.34** | **31.9** |

| | | | | | | |
|---|---|---|---|---|---|---|
| *with one alcoholic oxygen and three carboxyl groups; **with four carboxyl groups. | | | | | | |

Other radionuclides with diagnostic and therapeutic value that can be used with the compounds disclosed herein can be found, for example, in U.S. Patent Nos. 5,482,698 and 5,601,800; and Boswell and Brechbiel, Nuclear Medicine and Biology, 2007 October, 34(7): 757-778 and the manuscript thereof made available in PMC 2008 October 1.

### 3. Uses

The chelators and complexes disclosed herein can be used in a wide variety of therapeutic and diagnostic settings.

The disclosure provides a method of treating a disease in an animal comprising administering a complex disclosed herein to the animal, whereby the disease is ameliorated or eliminated.

The disclosure provides a method of diagnosing a disease in an animal comprising (a) administering a complex disclosed herein to the animal and (b) detecting the presence or absence of a signal emitted by the complex. In some embodiments, the detecting step comprises obtaining an image based on the signal.

In some embodiments, the disease is cancer.

In some embodiments, the complex comprises a linker to a targeting moiety and the method further comprises localizing the complex to a targeting site in the animal by binding the targeting moiety to the targeting site.

The compounds disclosed herein are particularly well suited for the preparation of stable, pre-labeled antibodies for use in the diagnosis and treatment of cancer and other diseases. For example, antibodies expressing affinity for specific tumors or tumor-associated antigens are labeled with a diagnostic radionuclide-complexed chelate, and the labeled antibodies can be further stabilized through lyophilization. Where a chelate is used, it generally is covalently attached to the antibody. The antibodies used can be polyclonal or monoclonal, and the radionuclide-labeled antibodies can be prepared according to methods known in the art. The method of preparation will depend upon the type of radionuclide and antibody used. A stable, lyophilized, radiolabeled antibody can be reconstituted with suitable diluent at the time of intended use, thus greatly simplifying the on site preparation process. The methods herein can be applied to stabilize many types of pre-labeled antibodies, including, but not limited to, polyclonal and monoclonal antibodies to tumors associated with melanoma, colon cancer, breast cancer, prostate cancer, etc. Such antibodies are known in the art and are readily available.

### 4. Synthesis

Any scaffold moiety can be derivatized with at least one linker, such as a functionalized linker. Thus, in one exemplary embodiment, a linker, such as a functionalized linker, can be attached to the scaffold moiety. In another exemplary embodiment, a linker, such as a functionalized linker, is attached to a chelating moiety. A functionalized linker can reacted to form a bond with a targeting moiety. The linker can also be attached to any other linker within a compound.

Scaffold moieties that include a linker can be prepared by the following exemplary methods.

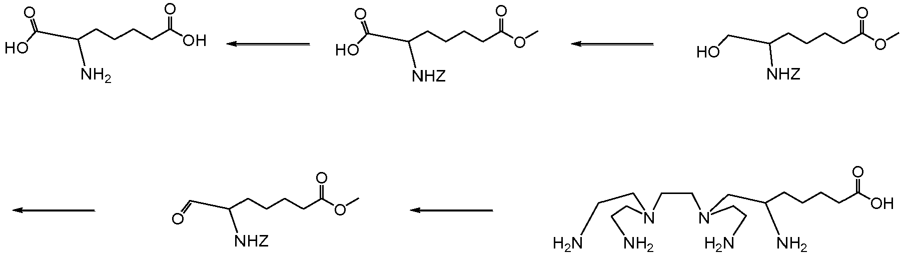

Other functionalize scaffolds include those in which the chiral carbon is placed on the central ethylene bridge of H22-amine. An exemplary route to such a scaffold initiates with 2,3-Diaminopropionic acid, as its carboxyl group is connected directly to the amine backbone to give a very rigid geometry, extended carboxyl chain is needed to provide flexibility for eventual protein conjugating. A synthetic scheme to the scaffold is shown in scheme 1.2.

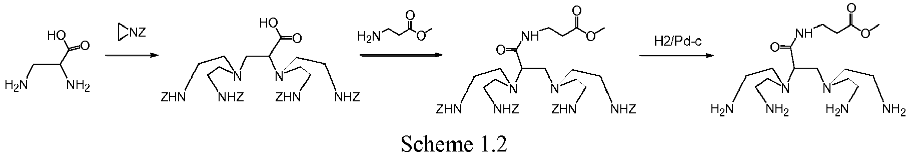

Variations on this synthesis include the use of a nitrophenylalanine or a BOC-amino group, which are optionally converted to carboxyl groups. Synthetic routes to these scaffolds are shown in Schemes 1.3 and 1.4.

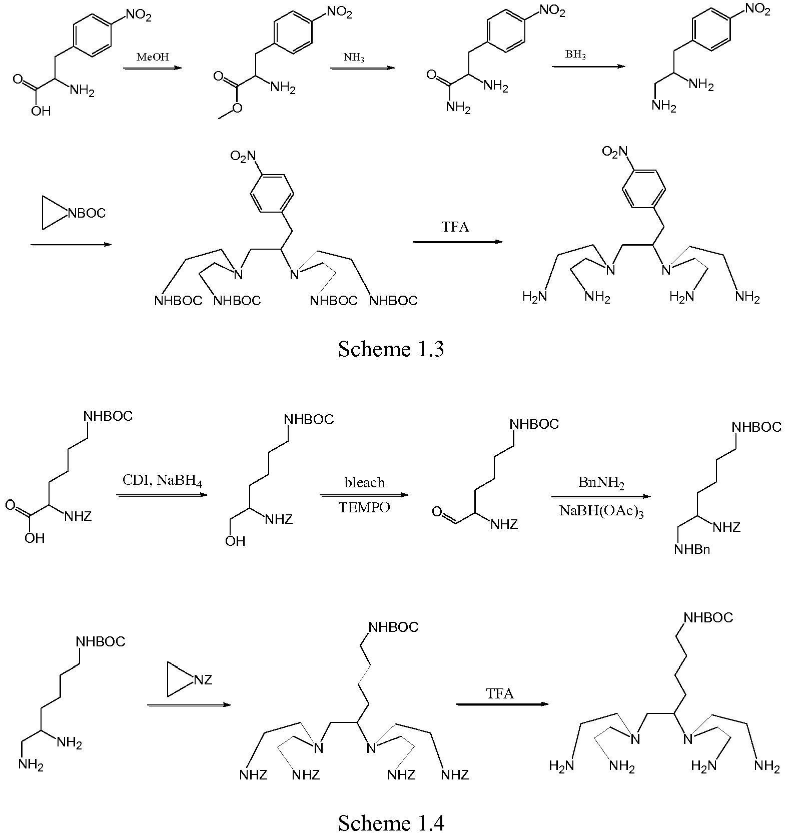

One concern with HOPO chelating moieties is that it might be difficult to couple these to a targeting moiety, such as an antibody, without protection in some form or another. One approach for HOPO chelating moiety protection/deprotection is to use a metal complex in the coupling reaction, then remove the metal from the metal complex-antibody conjugate after coupling to make room for the radionuclide (transmetalation). Another approach is to use ortho-nitrobenzyl in place of the benzyl protective group in the HOPO chelating moiety synthesis, and photodeprotect this after coupling the potential chelating moiety to the antibody.

Additional guidance for deprotecting, activating and attaching one or more chelating moieties to one or more scaffolds can be found, for example in US Patents 5,624,901; 6,406,297; 6,515,113 and 6,846,915; US Patent Application Publications 2008/0213780; 2008/0213917 and 2010/0015725; and PCT/US2010/046517.

Exemplary di-macrocycles, any of which can be derivatized with a linker (e.g., a functionalized linker or a linker comprising a targeting moiety) are disclosed throughout the application.

### EXAMPLES

The compounds and complexes of the invention are synthesized by an appropriate combination of generally well-known synthetic methods. Techniques useful in synthesizing the compounds of the invention are both readily apparent and accessible to those of skill in the relevant art. The discussion below is offered to illustrate certain of the diverse methods available for use in assembling the compounds of the invention, it is not intended to limit the scope of reactions or reaction sequences that are useful in preparing the compounds of the present invention.

### EXAMPLE 1

### Synthesis of an octa-coordinating di-macrocyclic bifunctional chelator (Scheme 1).

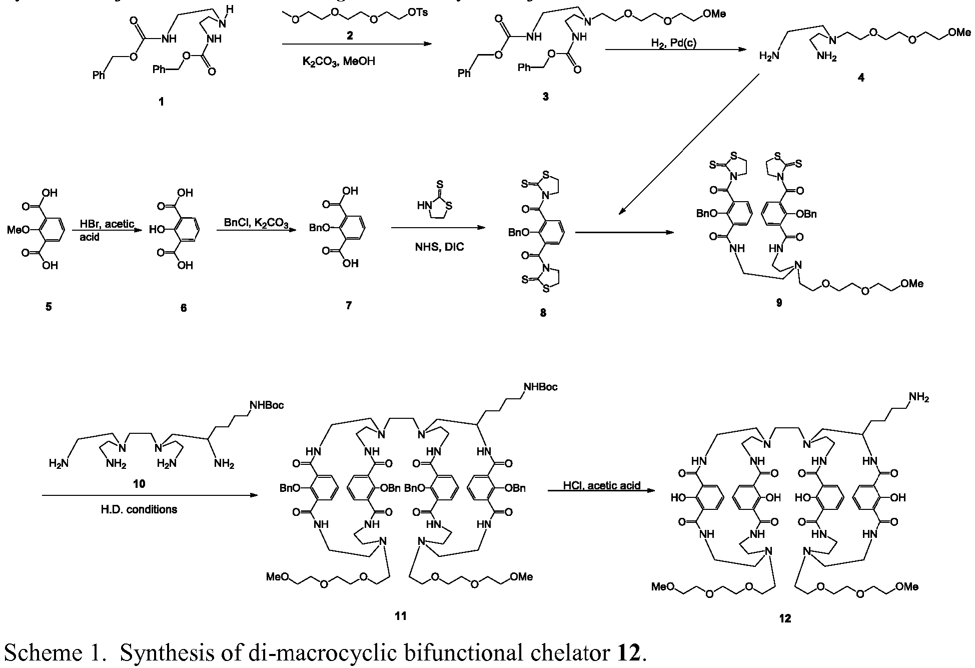

Preparation of macrocyclic ligands began with di-Z-diethylenetriamine **1** as a starting material as shown in Scheme 1. Amine **1** was alkylated with [2-[2-(2-methoxyethoxy)ethoxy]ethoxy] p-toluene sulfonate **2** to provide the tertiary amine **3,** which was deprotected under reducing conditions to provide triamine **4.** In a separate sequence, 2-hydroxyisophthalic acid **6** was first protected as the benzyl ether **7,** then condensed with 2-mercaptothiazole to form the amide **8.** The activated amide **8** was reacted with triamine **4** under pseudo-first order conditions to provide the amide **9,** which was reacted with amine **10** under high dilution conditions to form the di-macrocycle **11.** Protective groups were removed from compound **11** using a solution of concentrated hydrochloric acid in acetic acid to provide di-macrocycle **12.**

N,N"-Bis(carbobenzyloxy)-N'-[2-[2-(2-methoxyethoxy)ethoxy]ethoxy]-bis(2-aminoethyl)amine **3.** *N,N*"-Di-Z-diethylenetriamine **1** (1.00 g, 2.69 mmol), [2-[2-(2-methoxyethoxy)ethoxy]ethoxy] p-toluene sulfonate **2** (1.529 g, 4.80 mmol), potassium carbonate (557 mg, 4.04 mmol), and sodium iodide (404 mg, 2.69 mmol) were dried together in vacuo. Anhydrous acetonitrile (15 mL) was added, and the resulting solution was heated at reflux for 28 hr. The residue was dissolved in dichloromethane (25 mL) and washed with 1 M sodium hydroxide (15 mL). The aqueous phase was extracted with dichloromethane (10 mL) and solvent was removed from the combined organic extracts under reduced pressure. The crude product was purified by silica gel chromatography using 1 - 2% methanol in dichloromethane as eluents. Fractions containing product were combined, solvent was removed under reduced pressure, and the residue dried in vacuo to provide N,N"-bis(carbobenzyloxy)-N'-[2-[2-(2-methoxyethoxy)ethoxy]ethoxy]-bis(2-aminoethyl)amine **3** (1.028 g, 73.8%). ¹H NMR (300 MHz, CDCl₃): δ = 7.30 (s, 10H, ArH), 5.05 (s, 4H, PhCH₂O), 3.50 (m, 4H, CH₂CH₂O), 3.42 (m, 6H, CH₂CH₂O), 3.29 (s, 3H, OMe), 3.21 (m, 4H, CH₂CH₂N), 2.62 (m, 6H, CH₂CH₂N). ¹³C NMR (400 MHz, CDCl₃): δ = 156.8, 136.9, 128.4, 128.1, 128.0, 71.8, 70.5, 70.3, 70.2, 70.0, 66.5, 58.9, 54.3, 53.3, 39.2. FTMS pESI: calculated for C₂₇H₄₀N₃O₇ [MH]⁺, 518.2861, found, 518.2857.

N'-[2-[2-(2-methoxyethoxy)ethoxy]ethoxy]-bis(2-aminoethyl)amine **4.** N,N"-bis(carbobenzyloxy)-N'-[2-[2-(2-methoxyethoxy)ethoxy]ethoxy]-bis(2-aminoethyl)amine **3** (1.028 g, 1.99 mmol) was dissolved in ethyl alcohol (100 mL). Palladium on carbon (10% wet, 100 mg) was added, and the atmosphere was exchanged for hydrogen. After 19.5 hr, the solution was filtered through Celite® to remove catalyst, the Celite was washed with ethyl alcohol (100 mL), solvent was removed under reduced pressure, and the residue dried in vacuo to provide N'-[2-[2-(2-methoxyethoxy)ethoxy]ethoxy]-bis(2-aminoethyl)amine **4** (481 mg, 97.1%). ¹H NMR (300 MHz, CDCl₃): δ = 3.58 (m, 6H, CH₂CH₂O), 3.49 (m, 4H, CH₂CH₂O), 3.33 (s, 3H, OMe), 2.70 (t, 4H, CH₂CH₂N), 2.62 (t, 2H, CH₂CH₂N), 2.51 (t, 4H, CH₂CH₂N), 1.83 (br s, 4H, NH₂). ¹³C NMR (400 MHz, CDCl₃): δ = 71.9, 70.6, 70.4, 70.3, 69.9, 59.0, 57.8, 53.7, 39.7. FTMS pESI: calculated for C₁₁H₂₈N₃O₃ [MH]⁺, 250.2125, found, 250.2123.

2-Hydroxy-isophthalic Acid **6.** 2-Methoxyisophthalic acid **5** (25 g, 0.127 mol) was dissolved in a 1:1 mixture of 48% HBr and glacial acetic acid (700 mL) in a 1 L round bottom flask. The mixture was heated at reflux for 48 hr, whereupon the 2-hydroxyisophthalic acid deposited after cooling as slight pink crystals. These were collected by filtration and dried in vacuo to provide 2-hydroxyisophthalic acid (20.5 g, 89%). ¹H NMR (500 MHz, DMSO-d₆, 25 °C) δ: 6.942 (t, J = 7.5, 1H, ArH), 7.950 (d, J = 7.5, 1H, ArH), 10.51 (s, br, 1H, phenol H). ¹³C NMR (500 MHz, DMSO-d₆, 25 °C) δ: 117.19, 118.21, 135.66, 161.25, 169.24.

Dibenzyl 2-benzyloxyisophthalate **6A.** 2-Hydroxyisophthalic acid **6** (75 g, 0.38 mol), benzyl chloride (158 g, 1.25 mol), and anhydrous K₂CO₃ (172 g, 1.25 mol) were added to 500 mL of dry dimethylformamide. The mixture was heated at 75 °C under nitrogen for 18 hr. The reaction mixture was cooled to ambient temperature and filtered, and the filtrate was evaporated to dryness under reduced pressure. The resulting residue was dissolved in dichloromethane (2 L) and filtered through a silica gel plug. Solvent was removed under reduced pressure and the residue was dried in vacuo to provide dibenzyl 2-(benzyloxy)benzene-1,3-dicarboxylate **6A** as a thick pale yellow oil (158 g, 90%). ¹H NMR (500 MHz, CDCl₃): *δ* 5.07 (s, 2H, CH2), 5.31 (s, 4H, CH2), 7.23 (t, 1H, ArH), 7.3-7.4 (m, 15H, ArH), 7.97 (d, 2H, ArH). ¹³C NMR (125 MHz, CDCl₃): *δ* 66.9, 77.7, 123.5, 127.1, 127.7, 128.0, 128.0, 128.1, 128.2, 128.3, 134.8, 135.3, 136.6, 157.8, 165.2 ppm; MS (FAB+): *m*/*z* 453 [MH]⁺.

2-Benzyloxyisophthalic acid 7. To a solution of **6A** (155 g, 0.34 mmol) in 2 L of a 1:4 mixture of MeOH:H₂O was added NaOH (40 g, 1.0 mol), and the reaction mixture was stirred at room temperature for 18 h. The solvents were removed under vacuum, and the resulting residue was dissolved in brine and washed with dichloromethane. The aqueous layer was acidified to pH 1 with conc. HCl, causing the product to form a precipitate. The product, a white solid, was collected by filtration and dried under vacuum to provide compound **7** (85.6 g, 92%). mp 235-237 °C; ¹H NMR (500 MHz, CDCl₃): *δ* 5.23 (s, 2H, CH2), 7.39-7.41 (m, 3H, ArH), 7.425 (t, 1H, ArH), 7.48-7.51 (m, 2H, ArH), 8.35 (d, 2H, ArH). ¹³C NMR (125 MHz, D₂O-NaOD): *δ* 76.1, 123.4, 127.7, 128.0, 128.2, 128.5, 133.9, 136.2, 149.9, 176.6 ppm; MS (ESI-): *m*/*z* 271.1 [M - H]⁻

2-Benzyloxy-bis(2-mercaptothiazole)isophthalamide **8.** To a solution of 2-benzyloxyisophthalic acid **7** (68 g, 0.25 mol) in anhydrous dichloromethane, oxalyl chloride (76 g, 0.6 mol) and a few drops of dimethylformamide were added with stirring. After 8 hours, volatiles were removed under reduced pressure and the residue dried overnight in vacuo. The intermediate diacid chloride was dissolved in anhydrous dichloromethane (500 mL) and added dropwise to a cooled solution of 2-mercaptothiazole (62 g, 0.52 mol) in triethylamine (90 mL) and dichloromethane (500 mL). After the addition was complete, the solution was allowed to warm to ambient temperature and stirred overnight. The solution was washed with IN HCl (500 mL), brine (500 mL) and IN NaOH (500 mL). Solvent was removed under reduced pressure, and the crude product was purified by silica gel chromatography using methanol in dichloromethane as eluents. Fractions containing product were combined, solvent was removed under reduced pressure, and the residue dried in vacuo to provide 2-benzyloxy-bis(2-mercaptothiazole)isophthalamide **8** (75 g, 63%). mp 149-151 °C. ¹H NMR (300 MHz, CDCl₃): δ = 7.47 (d, 2H, ArH), 7.35 (m, 5H, ArH), 7.18 (t, 1H, ArH), 5.01 (s, 2H, OCH₂), 4.39 (t, 4H, CH₂), 3.02 (t, 4H, CH₂). ¹³C NMR (300 MHz, CDCl₃): δ = 200.9, 167.2, 153.2, 136.6, 132.1, 128.7, 128.5, 128.3, 127.7, 123.7, 55.7, 28.8. FTMS pESI: calculated for C₂₁H₁₉N₂O₃S₄ [MH]⁺, 475.0279, found, 475.0282.

N,N"-bis[1-benzyloxy-2-(2-mercaptothiazoleamido)-6-benzoyl]-N'-[2-[2-(2-methoxyethoxy)ethoxy]ethoxy]-bis(2-aminoethyl)amine **9.** N'-[2-[2-(2-methoxyethoxy)ethoxy]ethoxy]-bis(2-aminoethyl)amine **4** (481 mg, 1.93 mmol) was dissolved in dichloromethane (50 mL) and added dropwise to a solution of 2-benzyloxy-bis(2-mercaptothiazole)isophthalamide **8** (10.515 g, 22.2 mmol) in dichloromethane (250 mL) over a period of 20 hrs. Solvent was removed under reduced pressure, and the crude product was purified by silica gel chromatography using 1 - 3.5% methanol in dichloromethane as eluents. Fractions containing product were combined, solvent was removed under reduced pressure, and the residue dried in vacuo to provide N,N"-bis[1-benzyloxy-2-(2-mercaptothiazoleamido)-6-benzoyl]-N'-[2-[2-(2-methoxyethoxy)ethoxy]ethoxy]-bis(2-aminoethyl)amine **9** (1.209 g, 65.3%). ¹H NMR (300 MHz, CDCl₃): δ = 8.05 (d, 2H, ArH), 7.47 (m, 2H, ArH), 7.30 (s, 10H, ArH), 7.25 (s, 2H, ArH), 4.98 (s, 4H, PhCH₂O), 4.44 (t, 4H, NCH₂CH₂S), 3.55 (m, 2H, CH₂CH₂O), 3.50 (m, 6H, CH₂CH₂O), 3.39 (m, 9H, CH₂CH₂O, CH₂CH₂N, OMe), 3.04 (t, 4H, NCH₂CH₂S), 2.60 (t, 2H, CH₂CH₂N), 2.51 (t, 4H, CH₂CH₂N). ¹³C NMR (400 MHz, CDCl₃): δ = 167.4, 154.2, 136.2, 134.0, 132.3, 129.8, 128.8, 127.8, 124.8, 77.9, 71.9, 70.4, 55.7, 53.4, 37.8, 28.7. FTMS pESI: calculated for C₄₇H₅₄N₅O₉S₄ [MH]⁺, 960.2799, found, 960.2791.

Benzyl and tert-butyloxycarbonyl-protected di-macrocycle **11.** A solution of N,N"-bis[1-benzyloxy-2-(2-mercaptothiazoleamido)-6-benzoyl]-N'-[2-[2-(2-methoxyethoxy)ethoxy]ethoxy]-bis(2-aminoethyl)amine **9** (1.2 g, 1.25 mmol) in dichloromethane (1.0 L) and a solution of 5-amino-6-[(2-aminoethyl)-[2-[bis(2-aminoethyl)amino]ethyl]amino]hexylcarbamic acid tert-butyl ester **10** (0.5 g, 1.25 mmol) in dichloromethane (1.0 L) were added dropwise to dichloromethane (1.0 L) over a period of 5 days. Solvent was removed under reduced pressure, and the crude product was purified by silica gel chromatography using 0.1% triethylamine, 5 - 7.5% methanol in dichloromethane as eluents. The silica gel column was prepared so as to have a short section of aluminum oxide (basic, Brockmann I) on its top. Fractions containing product were combined, solvent was removed under reduced pressure, and the residue dried in vacuo to provide the protected di-macrocycle **11** (340 mg, 14.7%). ¹H NMR (300 MHz, CDCl₃): δ = 7.8 - 7.4 (m, 8H, ArH), 7.29 (s, 20H, ArH), 7.1 - 6.7 (m, 4H, ArH), 5.0 - 4.8 (m, 8H, PhCH₂O), 3.6 - 3.3 (m, 26H, CH₂CH₂O, OMe), 3.2 - 2.1 (m, 35H, CH₂CH₂N), 1.8 (br s, 4H, CH₂CH₂N), 1.4 - 1.2 (m, 15H, CH₂CH₂CH₂, C(CH₃)₃. ¹³C NMR (400 MHz, CDCl₃): δ = 166.1, 166.0, 156.1, 154.1, 136.1, 135.8, 132.8, 132.5, 128.9, 128.8, 128.7, 128.3, 124.6, 124.4, 124.0, 71.9, 71.8, 70.5, 70.3, 70.1, 69.9, 69.7, 68.8, 58.9, 53.8, 53.1, 52.7, 52.3, 40.3, 38.5, 37.7, 37.5, 33.0, 29.7, 28.5, 23.3. FTMS pESI: calculated for C₁₀₁H₁₃₂N₁₃O₂₀ [MH]⁺, 1846.9706, found, 1846.9722.

Di-macrocycle **12.** Benzyl and tert-butyloxycarbonyl-protected di-macrocycle **11** (10 mg, 5.4 µmοl) was dissolved in 12N hydrochloric acid (0.5 mL) and glacial acetic acid (0.5 mL). The solution was stirred under inert atmosphere for 24 hr, whereupon HCl was removed with a stream of inert gas. Solvents were removed under reduced pressure and the residue was dried in vacuo. The residue was dissolved in methanol (600 µL) and transferred to two O-ring microcentrifuge tubes. Ether (ca. 1.5 mL/tube) was added, and the tubes were placed at 4 °C for 30 minutes. The tubes were centrifuged at 12,000 rpm for 3 minutes, decanted, the pellets were washed with ether (ca. 1.5 mL/tube) and allowed to air dry. The pellets were dissolved in methanol (300 µL), transferred to a single microcentrifuge tube, and precipitated with ether as described above. The pellet was dried in vacuo to provide di-macrocycle **12,** pentahydrochloride salt (8.0 mg, 94%). FTMS pESI: calculated for C₆₈H₁₀₀N₁₃O₁₈ [MH]⁺, 1386.7304, found, 1386.7306.

During the synthesis of di-macrocycle **11,** the regioisomer **11R** is also formed. Regioisomer **11R** can be deprotected to provide compound **12R:**

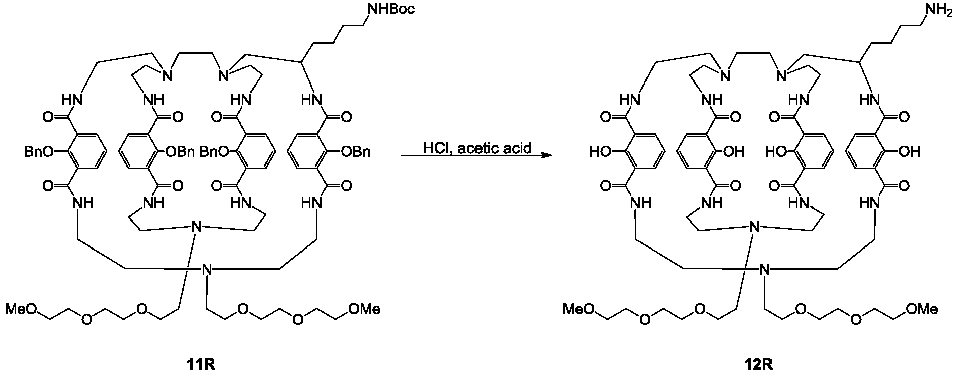

### EXAMPLE 2

### Synthesis of an octa-coordinating di-macrocyclic - oligodeoxynucleotide conjugate (Scheme 2).

Di-macrocycle, 4-isothiocyanatophenylthiourea derivative **13.** Di-macrocycle **12** (5.1 mg, 3.3 µmοl) was dissolved in dimethylformamide (250 µL) and triethylamine (10 µL). The solution was transferred to a microcentrifuge tube containing 1,4-phenyldiisothiocyanate (6.3 mg, 33 µmοl) and mixed at 800 rpm under inert atmosphere for 1.5 hours. Ether (ca. 1.5 mL) was added, and the resulting suspension placed at 4 °C for 60 minutes. The tube was centrifuged at 12,000 rpm for 3 minutes, decanted, the pellet was washed with ether (ca. 1.5 mL) and allowed to air dry. The pellet was dissolved in methanol (250 µL) and precipitated and washed with ether as described above. The pellet was dissolved again in methanol (300 µL) and precipitated and washed with ether as described above. The pellet was dried in vacuo to provide di-macrocycle, 4-isothiocyanatophenylthiourea derivative **13** (1.4 mg, 27%). FTMS pESI: calculated for C₇₆H₁₀₄N₁₅O₁₈S₂ [MH]⁺, 1578.7120, found, 1578.7125.

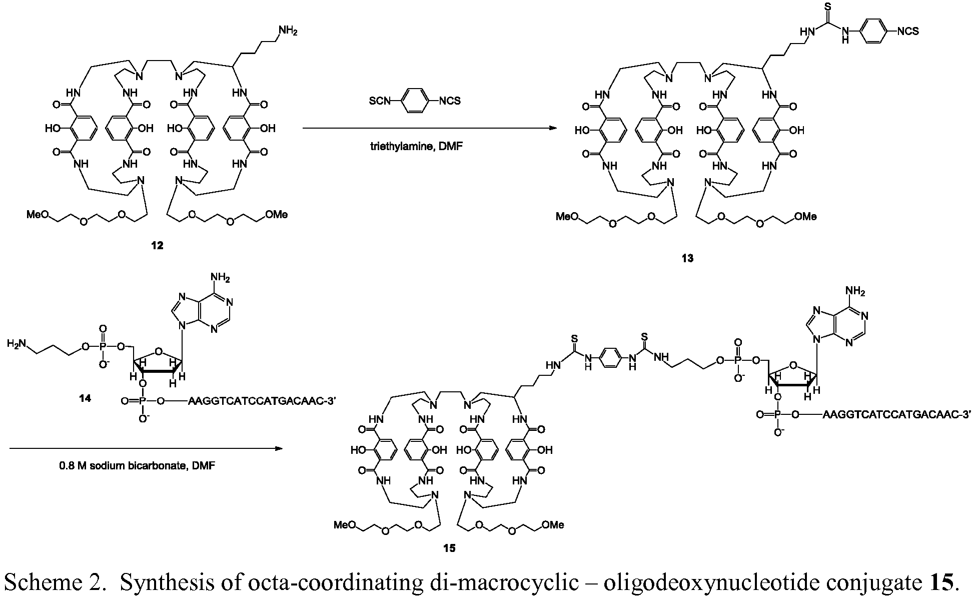

Dimacrocycle - oligodeoxynucleotide conjugate **15.** A DNA 18-base oligonucleotide **(14)** with the sequence 5'-AAGGTCATCCATGACAAC-3' was purchased commercially (Eurogentec, Inc., Seraing, Belgium) and purified using reverse-phase HPLC. The oligonucleotide was modified during synthesis to possess an aminopropyl group attached at the 5'-terminus via a phosphodiester linkage. A solution of DNA oligomer in water (75 µL, 95 nmol) was diluted with sodium bicarbonate buffer (0.8 M, 100 µL) in an eppendorf tube. A solution of di-macrocycle, 4-isothiocyanatophenylthiourea derivative **13** (1 mg, 633 nmol) in anhydrous DMF (50 µL) was freshly prepared, added to the DNA oligomer and mixed at 1200 rpm using a commercial device (Eppendorf Mixmate®) at ambient temperature for 19.5 hours. A solution (45 µL) of glycogen (350 µg/mL) in 3M sodium acetate, pH 5.2 was added to the solution. The solution was mixed, absolute ethanol (1.1 mL) was added, the solution was mixed again, and the tube was stored at -20 °C for 1.5 hours. The eppendorf tube was centrifuged at 12,000 rpm for 20 minutes, the supernatant decanted, and the resulting pellet was washed with cold, 70% aqueous ethanol (1.1 mL). The supernatant was decanted, and the pellet was allowed to dry open to the air. The pellet was dissolved in sterile water (100 µL), and an aliquot (2 µL) was removed to quantify by UV-visible absorbance using the extinction coefficient at 260 nm of 181,600 M⁻¹ cm⁻¹. The resulting stock was found to have a concentration of 882 µM (88.2 nmol, 93% crude yield). There was ca. 40% conversion to conjugate, as estimated from analysis using 20% polyacrylamide gel electrophoresis. The conjugate was used without further purification.

### EXAMPLE 3

### Complexation of a dimacrocycle - oligodeoxynucleotide conjugate with various metal cations as demonstrated by gel electrophoresis.

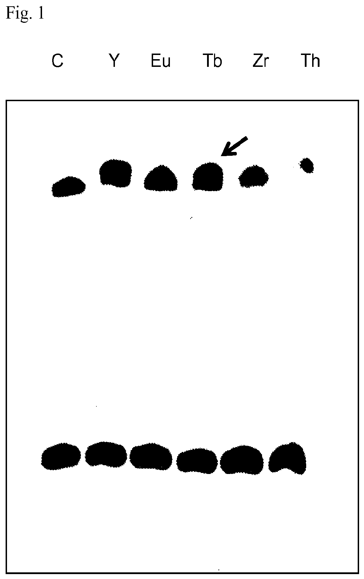

For use in certain applications, such as acting as a bifunctional chelating agent to attach a radioisotope to a site-directing molecule, it is necessary that the chelator be able to coordinate to the metal ion of interest in a kinetically facile and thermodynamically stable manner. To demonstrate the utility of the di-macrocyclic chelator **12** for this type of application, its ability to coordinate to metal cations following conjugation with a site-directing molecule was assessed using a gel electrophoresis assay (Figure 1). In this experiment, a conjugate of **12** with a small (18 base length) DNA oligomer, **15** was treated with a solution containing a metal cation. The electrophoretic mobility of the conjugate on a polyacrylamide gel was then compared with that of the conjugate which was not exposed to the solution of metal cation. Metal complexation in this format is indicated by a gel electrophoresis mobility shift, such that the heavier and more positively charged species formed upon metal complexation migrates more slowly. The gel electrophoretic mobility of the DNA oligomer **14** that was not conjugated to **12,** present in the solution of **15,** was also compared with and without exposure to the metal cation solution.

Figure 1 shows the electrophoretic mobility of DNA oligonucleotide conjugate **15** (upper band) or DNA oligonucleotide **14** (lower band) in the absence or presence of metal cations. C = control, no metal cations. Y, Eu, Tb, Zr, Th = yttrium(III), europium(III), terbium(III), zirconium(IV), and thorium(IV), respectively. Arrow indicates luminescent band observed under hand held ultraviolet lamp.

In particular, a solution of DNA oligomer (3 µL, 33 µM, all concentrations final) was mixed with a solution of metal cation (2 µL, 625 µM) or an equal volume of water, additional water (2.5 µL) and a buffer solution (2.5 µL, 50 mM HEPES, pH 7.5). The solution was incubated at 55 °C for 15 minutes, whereupon the solution was allowed to cool to ambient temperature and a solution of 50% formamide (5 µL) was added. The solution was then applied to a 20% polyacrylamide gel containing 8M urea. Gel electrophoresis was conducted for about 7 hours using a commercial running buffer (Ambion AM9863) containing 89 mM tris(hydroxymethyl)aminomethane (TRIS), 89 mM borate, and 2 mM ethylenediaminetetraacetic acid (EDTA). Upon the completion of electrophoresis, the gel was removed from the glass plates and soaked in a 50% formamide solution containing 12.5 mg/mL Stains-All® (Sigma Chemicals). After staining, the gel was destained in de-ionized water for 2 hours and imaged using a commercial scanner (HP Officejet® J5750).

Inspection of the gel indicates that the oligonucleotide-di-macrocycle conjugate **15** migrates more slowly following treatment with the metal cation solution. In contrast, the gel mobility of the unmodified oligonucleotide is unaffected by metal cation treatment. These data indicate that the di-macrocyclic chelator **12,** when conjugated to a DNA oligomer, coordinates with facility to the metal cations tested and forms a stable complex even upon electophoresis in the presence of the competing chelator EDTA. In summary, our findings suggest that di-macrocyclic chelator **12** when conjugated to a site-directing group coordinates readily with a variety of metal cations including those of the lanthanide series.

Preparation of metal ion stocks. In general, the chloride salts of metal cations were dissolved in 50 mM sodium citrate, pH 5, to provide primary stocks of 25 mM cation. These stocks were diluted to 2.5 mM in sterile water. Yttrium triflate was used instead of the chloride salt. In the case of Th(IV) nitrate, a 25 mM stock was prepared in methanol, and this was diluted to 2.5 mM using additional methanol. A saturated solution of zirconium oxalate in 5 mM sodium citrate, pH 5, was also used in these experiments.

### EXAMPLE 4

### Synthesis of an octa-coordinating di-macrocyclic chelator peptide conjugate (Scheme 3).

A related synthetic approach is used to prepare a symmetrical intermediate that is conjugated to a peptide, an oligonucleotide, or other biomolecules. In this approach, an orthogonal protective group, e.g., butyloxycarbonyl, is used to protect the secondary amine of diethylene triamine. Reaction with benzyl protected dithiazolide under pseudo-first order conditions yields the butyloxycarbonyl protected dithiazolide intermediate that is condensed with tetrakis(2-aminoethyl)-ethylenediamine under high dilution conditions to provide the fully protected dimacrocycle. The butyloxycarbonyl protective groups are removed using trifluoroacetic acid to provide the diamine. This diamine is reacted with two peptides sequentially, for example by using an excess of diamine to condense with the first peptide, then conjugating the resulting mono-peptide conjugate with a second peptide. The peptides may be identical or different. Following removal of benzyl protective groups under reducing conditions, the peptide protective groups are removed, for example, with trifluoroacetic acid. The resulting octa-coordinating di-macrocyclic chelator peptide conjugate is used to form a luminescent coordination complex with terbium(III). This compound can be used to assay for the presence of a protein, for example, that binds one or more of the peptide sequences, effecting terbium(III) release and loss of the luminescent signal.

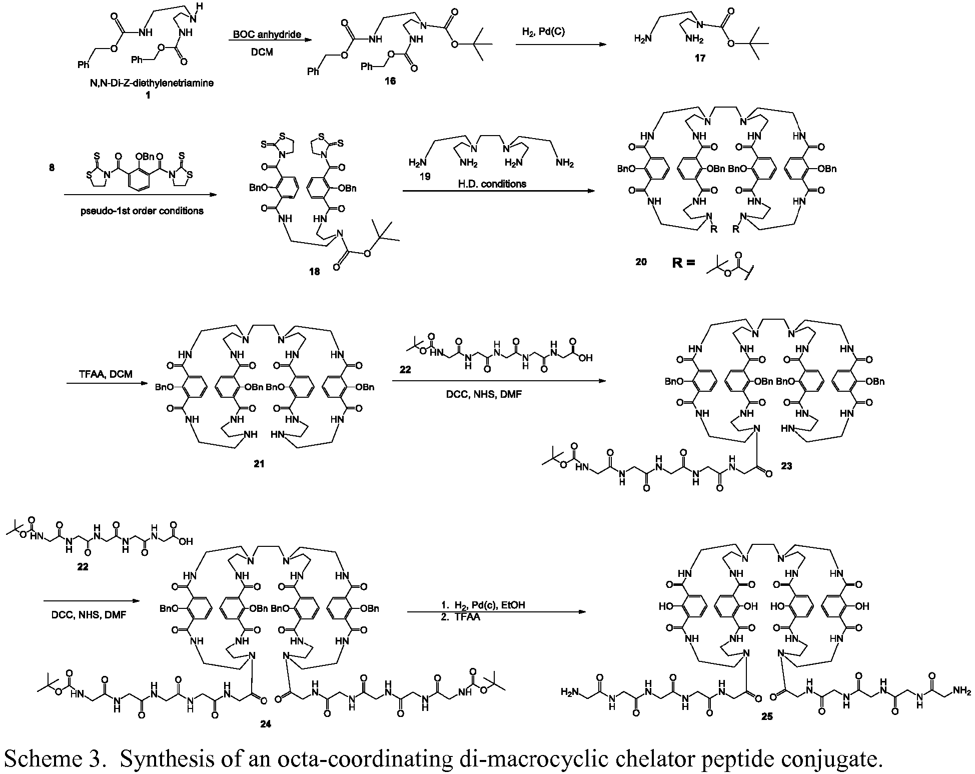

### EXAMPLE 5

### Protein detection using a luminescent di-macrocyclepeptide conjugate.

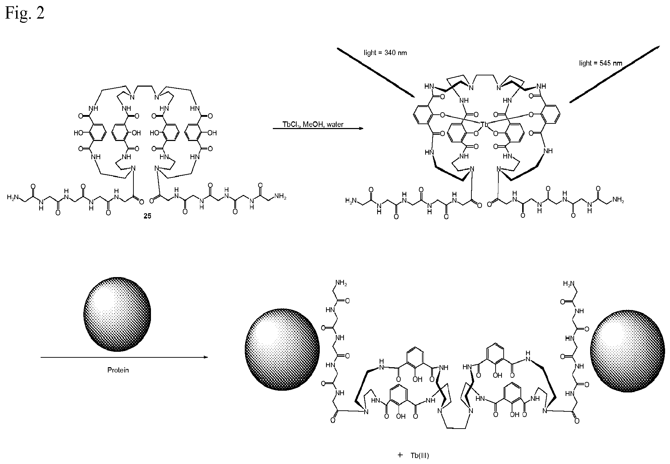

The octa-coordinating di-macrocyclic chelator peptide conjugate prepared above may be used to assay the presence of a protein that binds specifically to the peptide sequence(s) it contains. For example, addition of buffered terbium chloride solution to an aqueous or methanol solution of peptide conjugate 25 will result in formation of a metal complex (or chelate). The chelator in this instance shields the terbium cation from water and also acts as a photosensitizer, such that activation with light of approximately 340 nm results in long lived terbium luminescence at 545 nm (and other wavelengths). Specific association with a protein in an analytical specimen such as serum would be expected to induce a conformational change in the peptide conjugate such that terbium cation would be released into solution and thus lead to a loss of luminescence. A cartoon of this process is shown in Figure 2.

### EXAMPLE 6

### Synthesis of a benzyl-protected 1,2-hydroxypyridinone monoacid monoester (Scheme 4).

Preparation of macrocyclic targets containing 3-pyridinol coordination groups began with 6-methyl-3-pyridinol **26** as a starting material as shown in Scheme 4. Following a literature procedure, **26** was elaborated to the key pyridinol **31,** which was alkylated with bromoethylacetate to provide a suitable pyridine dicarboxylate system. Further standard synthetic transformation led to the protected pyridine diester **36.** Selective saponification was necessary in order to assure regioisomeric purity of subsequent products. In this instance the selective hydrolysis of an aliphatic vs. aromatic ester provided the desired ester **37.**

Methyl 2-bromo-3-[(ethylacetyl)oxy]-6-pyridinecarboxylate **32.** Methyl 2-bromo-3-hydroxy-6-pyridinecarboxylate **31** was prepared as previously described (Kelly, T. R. and Lang, F. J. Org. Chem., 1996, 61, 4623-4633). Compound **31** (4.732 g, 20.4 mmol) was treated with ethyl bromoacetate (3.39 mL, 30.6 mmol) and potassium carbonate (4.22 g, 30.6 mmol) in 125 mL anhydrous acetonitrile and heated at reflux for 6 hr. Solvent was removed under reduced pressure, and the residue was dissolved in dichloromethane (100 mL) and washed with water (50 mL). The aqueous wash was extracted with dichloromethane (25 mL) and the combined dichloromethane extracts were washed with water (25 mL). Solvent was removed under reduced pressure, and the residue was dried in vacuo. The crude product was purified by silica gel chromatography using neat dichloromethane and 1% methanol in dichloromethane as eluents. Fractions containing product were combined, solvent was removed under reduced pressure, and the residue dried in vacuo to provide methyl 2-bromo-3-[(ethylacetyl)oxy]-6-pyridinecarboxylate **32** (6.519 g, 100%). ¹H NMR (300 MHz, CDCl₃): δ = 8.06 (d, 1H, ArH), 7.08 (s, H, ArH), 4.79 (s, 2H, CH₂O), 4.27 (q, 2H, CH₂CH₃), 3.96 (s, 3H, CO₂CH₃), 1.29 (t, 3H, CH₂CH₃. ¹³C NMR (300 MHz, CDCl₃): δ = 167.3, 164.5, 154.5, 141.0, 133.2, 126.0, 119.5, 66.2, 62.3, 53.2, 14.4. FTMS pESI: calculated for C₁₁H₁₃BrNO₅ [MH]⁺, 317.9972, found, 317.9973.

2-Bromo-3-[(carboxymethyl)oxy]-6-pyridinecarboxylic acid **33.** Compound **32** (6.519 g, 20.5 mmol) was dissolved in tetrahydrofuran (85 mL). Sodium hydroxide, 1 M solution (62 mL, 62 mmol) was added and the mixture was stirred for 5 hr. Tetrahydrofuran was removed under reduced pressure, and the solution was acidified to pH 2 using 6N hydrochloric acid (ca. 11 mL). The resulting solids were filtered, washed with water (2 x 10 mL), and dried in vacuo to provide 2-bromo-3-[(carboxymethyl)oxy]-6-pyridinecarboxylic acid **33** (5.657 g, 100%). ¹H NMR (300 MHz, MeOD): δ = 8.07 (d, 1H, ArH), 7.42 (d, 1H, ArH), 4.93 (s, 2H, CH₂O). ¹³C NMR (300 MHz, MeOD): δ = 170.7, 166.5, 156.0, 141.7, 133.3, 127.0, 121.3, 66.5. FTMS - pESI: calculated for C₈H₅BrNO₅ [M]⁻, 273.9357, found, 273.9365.

2-Bromo-3-[(carboxymethyl)oxy]-6-carboxypyridine-N-oxide **34.** Compound **33** (5.657 g, 20.5 mmol) was dissolved in trifluoroacetic acid (113 mL) under inert atmosphere. Hydrogen peroxide, 30% aqueous solution (8.0 mL, 71 mmol) was added and the solution was heated to 80 °C for 3 hr. After cooling, water (25 mL) was added and solvents were removed under reduced pressure. Additional water (25 mL) was added, the resulting suspension was triturated for 0.5 hr, whereupon the solids were filtered using a fritted funnel, washed with water (3 x 5 mL) and dried in vacuo to provide 2-bromo-3-[(carboxymethyl)oxy]-6-carboxypyridine-N-oxide **34** (5.205 g, 87.0%). ¹H NMR (300 MHz, MeOD): δ = 8.29 (d, 1H, ArH), 7.43 (d, 1H, ArH), 5.05 (s, 2H, CH₂O). ¹³C NMR (400 MHz, DMSO, MeOD): δ = 168.9, 160.7, 157.6, 131.7, 127.6, 125.8, 114.9, 66.7. FTMS pESI: calculated for C₈H₇BrNO₆ [MH]⁺, 291.9451, found, 291.9449.

1-Hydroxy-2-oxo-3-[(carboxymethyl)oxy]-6-pyridinecarboxylic acid **35.** Compound **34** (5.205 g, 17.8 mmol) was dissolved in 15% aqueous potassium hydroxide (83 mL) under inert atmosphere. The solution was heated to 80 °C for 36 hr. After cooling, concentrated hydrochloric acid (17 mL) was added, the resulting suspension was triturated for 0.5 hr, whereupon the solids were filtered using a fritted funnel, washed with water (3 x 5 mL) and dried in vacuo to provide crude 1-hydroxy-2-oxo-3-[(carboxymethyl)oxy]-6-pyridinecarboxylic acid **35** (4.667 g, 114%) that was used in the next step without further purification. ¹H NMR (300 MHz, D2O, NaOD): δ = 6.60 (d, 1H, ArH), 6.15 (d, 1H, ArH), 4.30 (s, 2H, CH₂O). ¹³C NMR (400 MHz, D2O, NaOD): δ = 176.3, 170.5, 156.2, 145.9, 140.1, 111.9, 102.8, 67.4. FTMS - pESI: calculated for C₈H₆NO₇ [M]⁻, 228.0150, found, 228.0149.

1-Benzyloxy-2-oxo-3-[(carboxymethyl)oxy]-6-pyridinecarboxylic acid dimethyl ester **36.** Compound **35** (2.340 g, 10.2 mmol) was suspended in methanol (80 mL) under inert atmosphere. Chlorotrimethylsilane (13 mL, 102 mmol) was added, and the suspension was stirred for 44 hr. Solvents were removed under reduced pressure, potassium carbonate (2.819 g, 20.4 mmol) was added, and the residue was dried in vacuo overnight. The mixture was suspended in anhydrous acetonitrile (100 mL), benzyl bromide (2.43 mL, 20.4 mmol) was added, and the suspension was heated at reflux for 6 hr. After cooling, solvents were removed under reduced pressure, the residue was dissolved in dichloromethane (75 mL), and washed with water (50 mL). The aqueous layer was washed with dichloromethane (20 mL), and the combined dichloromethane extracts were washed with water (25 mL). Solvent was removed under reduced pressure and the product was purified by silica gel chromatography using 2% methanol in dichloromethane as eluent. Purified product was dried in vacuo to provide 1-benzyloxy-2-oxo-3-[(carboxymethyl)oxy]-6-pyridinecarboxylic acid dimethyl ester **36** (2.883 g, 81.3%). ¹H NMR (300 MHz, CDC13): δ = 7.57 (m, 2H, ArH), 7.37 (m, 3H, ArH), 6.64 (s, 2H, ArH), 5.40 (s, 2H, CH2Ph), 4.82 (s, 2H, CH₂O), 3.85 (s, 3H, CH3), 3.79 (s, 3H, CH3). ¹³C NMR (400 MHz, CDC13): δ = 168.4, 160.1, 154.8, 152.1, 133.8, 131.1, 130.2, 129.2, 128.6, 114.4, 108.4, 78.6, 66.2, 53.0, 52.4. FTMS +pESI: calculated for C₁₇H₁₈NO₇ [MH]⁺, 348.1078, found, 348.1078.

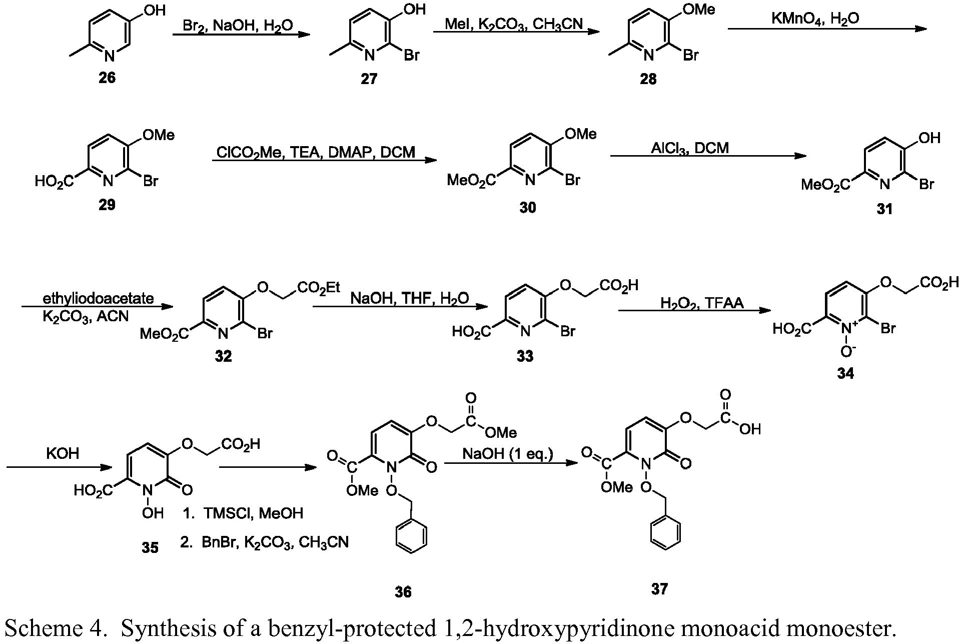

1-Benzyloxy-2-oxo-3-[(carboxymethyl)oxy]-6-pyridinecarboxylic acid 6-methyl ester **37.** Compound **36** (2.348 g, 6.76 mmol) was dissolved in tetrahydrofuran (57 mL) and deionlzed water (14 mL). Sodium hydroxide (1M, 6.42 mL, 6.42 mmol) was added over the course of half an hour by addition funnel. The funnel was rinsed with water (0.5 mL), and the solution was allowed to stir overnight. Tetrahydrofuran was removed under reduced pressure, and the solution was transferred to a separatory funnel using dichloromethane (75 mL) and water (50 mL). The organic layer was removed and saved. The aqueous layer was washed with dichloromethane (3 x 75 mL), and the combined dichloromethane extracts were also saved (to recover unreacted starting material). Hydrochloric acid (1 M, 6.0 mL) was added to the aqueous phase to form a precipitate that was extracted with 20% ethyl acetate in dichloromethane (200 mL and 2 x 100 mL). The combined organic extracts were washed with water (2 x 100 mL) and solvents were removed under reduced pressure. Product was dried in vacuo to provide 1-benzyloxy-2-oxo-3-[(carboxymethyl)oxy]-6-pyridinecarboxylic acid 6-methyl ester **37** (1.764 g, 78.3%). Unreacted starting material (285 mg) was also recovered. ¹H NMR (400 MHz, DMSO-d6): δ = 7.50 (m, 2H, ArH), 7.42 (m, 3H, ArH), 6.78 (d, 1H, ArH), 6.64 (d, 1H, ArH), 5.27 (s, 2H, CH2Ph), 4.39 (s, 2H, CH₂O), 3.81 (s, 3H, CH3). ¹³C NMR (400 MHz, DMSO-d6): δ = 160.3, 154.4, 153.0, 134.3, 130.2, 130.0, 129.0, 128.7, 112.1, 110.0, 78.2, 67.5, 53.4. FTMS +pESI: calculated for C₁₆H₁₆NO₇ [MH]⁺, 334.0921, found, 334.0917.

### EXAMPLE 7

### Synthesis of a 1,2-hydroxypyridinone octa-coordinating di-macrocycle (Scheme 5).

Benzyl-protected 1,2-hydroxypyridinone monoacid monoester **37** is activated using diisopropylcarbodiimide and N-hydroxysuccinimide in dimethylformamide and reacted with diamine **4** to form the diamide **38.** Ester groups are removed from the diamide by hydrolysis, whereupon the resulting diacid **39** is activated using diisopropylcarbodiimide and N-hydroxysuccinimide in dimethylformamide and condensed with tetra-amine **10** under high-dilution conditions to provide the benzyl and tert-butyloxycarbonyl protected di-macrocycle **40.** Removal of protective groups using acidic conditions results in the 1,2-hydroxypyridinone octa-coordinating di-macrocycle **41.**

N,N"-bis[1-Benzyloxy-2-oxo-3-[(carboxyamidomethyl)oxy]-6-(methoxycarbonyl)pyridine]-N'-[2-[2-(2-methoxyethoxy)ethoxy]ethoxy]-bis(2-aminoethyl)amine **38.** 1-Benzyloxy-2-oxo-3-[(carboxymethyl)oxy]-6-pyridinecarboxylic acid 6-methyl ester **37** (821 mg, 2.46 mmol) and N-hydroxysuccinimide (340 mg, 2.95 mmol) were dried in vacuo overnight. Anhydrous dimethylformamide (5 mL) was added to form a solution, diisopropylcarbodiimide (458 µL, 2.95 mmol) was added, and the solution was stirred for 6 hr under nitrogen atmosphere. N'-[2-[2-(2-methoxyethoxy)ethoxy]ethoxy]-bis(2-aminoethyl)amine **4** (256 mg, 1.03 mmol) was dissolved in dimethylformamide (1 mL) and diisopropylethylamine (536 µL, 3.08 mmol) and added to the reaction mixture. After 22 hr, water (ca. 1 mL) was added, and solvents were removed under reduced pressure. The residue was dissolved in dichloromethane (20 mL), washed with water (15 mL), and the aqueous fraction was extracted with dichloromethane (2 x 10 mL). The combined dichloromethane extracts were concentrated and the crude product was purified by silica gel chromatography using 0.1% triethylamine, 5% methanol in dichloromethane as eluents. Fractions containing product were combined, solvent was removed under reduced pressure, and the residue dried in vacuo to provide compound **38** (389 mg, 43.1%). ¹H NMR (300 MHz, CDCl₃): δ = 7.99 (br t, 2H, NH), 7.52 (m, 4H, PhH), 7.36 (m, 6H, PhH), 6.58 (s, 4H, ArH), 5.33 (s, 4H, PhCH₂O), 4.50 (s, 4H, OCH₂C=O), 3.81 (6H, s, CO₂CH₃), 3.55 - 3.39 (m, 14H, CH₂O, CH₂NC=O), 3.32 (s, 3H, OCH₃), 2.74 (m, 6H, CH₂N). ¹³C NMR (600 MHz, CDCl₃): δ = 166.6, 159.6, 154.7, 152.7, 133.7, 130.1, 129.6, 129.1, 129.0, 128.5, 112.4, 108.9, 78.4, 71.9, 71.8, 70.5, 70.3, 69.5, 68.4, 58.9, 53.9, 53.4, 52.8, 37.5. FTMS pESI: calculated for C₄₃H₅₄N₅O₁₅ [M+H]⁺, 880.3611, found, 880.3608.

N,N"-bis[1-Benzyloxy-2-oxo-3-[(carboxyamidomethyl)oxy]-6-carbonyl(2-mercaptothiazolide)pyridine]-N'-[2-[2-(2-methoxyethoxy)ethoxy]ethoxy]-bis(2-aminoethyl)amine **39.** Diester **38** (341 mg, 388 µmol) was dissolved in tetrahydrofuran (7 mL) and water (1 mL). One molar aqueous sodium hydroxide solution (1.356 mL, 1.356 mmol) was added and the solution was stirred for 23 hr. Hydrochloric acid (6N, 226 µL) was added, solvents were removed under reduced pressure, and the residue was dried in vacuo. A portion of the residue (281 mg, 316 µmοl) was treated with TBTU (254 mg, 791 µmol) and 2-mercaptothiazoline (94 mg, 789 µmol), and the flask was dried further in vacuo. Anhydrous dichloromethane (10 mL) was added to form a suspension, whereupon diisopropylethylamine (500 uL, 2.87 mmol) was added to form a solution. After 18 hours, solvent was removed under reduced pressure and the residue was purified by silica gel chromatography to provide compound **39** (136 mg, 40.8%). FTMS pESI: calculated for C₄₇H₅₆N₇O₁₃S₄ [M+H]⁺, 1054.2813, found, 1054.2797.

Benzyl and tert-butyloxycarbonyl-protected di-macrocycle **40.** A solution of compound **39** (ca. 1 g) in dichloromethane (49.5 mL) and triethylamine (0.5 mL) and a solution of 5-amino-6-[(2-aminoethyl)-[2-[bis(2-aminoethyl)amino]ethyl]amino]hexylcarbamic acid tert-butyl ester **10** (one molar equivalent) in dichloromethane, isopropyl alcohol (ca. 5%), and diisopropylethylamine (ca. 3%) (50 mL) are added dropwise to dichloromethane (2 L) over a period of four days using two syringe pumps at a rate of 0.5 mL/hr. After an additional two days of reaction, solvent is removed under reduced pressure, and the crude product is purified by silica gel chromatography. The silica gel column is prepared so as to have a short section (ca. 1.25") of aluminum oxide (basic, Brockmann I) on its bottom. Fractions containing product are combined, solvent is removed under reduced pressure, and the residue dried in vacuo to provide the protected di-macrocycle **40.**

Di-macrocycle **41.** Benzyl and tert-butyloxycarbonyl-protected di-macrocycle **40** (ca. 50 mg) is dissolved in 12N hydrochloric acid (1.0 mL) and glacial acetic acid (1.0 mL). The solution is stirred under inert atmosphere for ca. one day, whereupon HCl is removed with a stream of inert gas. Solvents are removed under reduced pressure and the residue is dried in vacuo. The residue is dissolved in methanol (600 + 300 µL) and transferred to two O-ring microcentrifuge tubes. Ether (ca. 1.5 mL) is added, and the tubes are placed at 4 °C for 1 hr. The tubes are centrifuged at 12,000 rpm for 3 minutes, decanted, the pellets are washed with ether (ca. 1.5 mL) and allowed to air dry. The pellets are dried in vacuo to provide di-macrocycle **41,** pentahydrochloride salt.

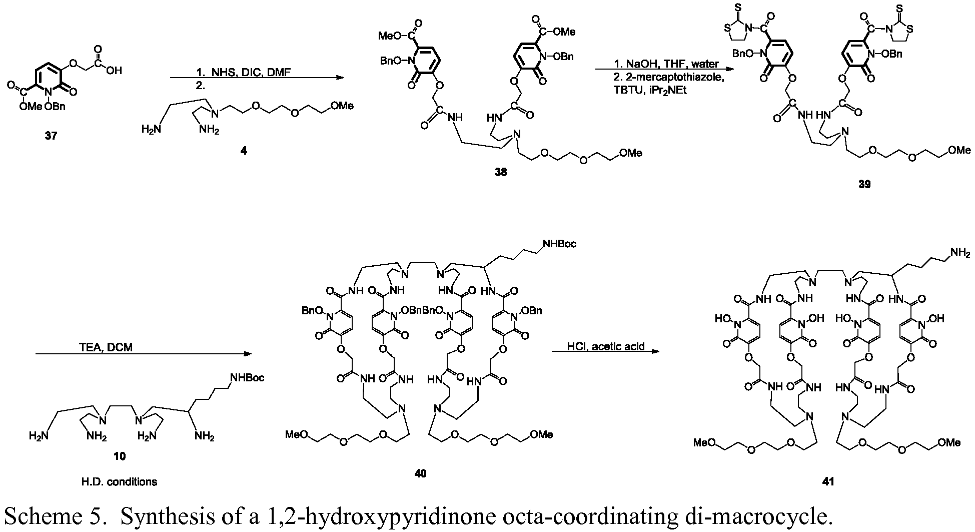

### EXAMPLE 8

### Compound stabilization using complementary DNA oligonucleotides.

Utilizing complimentary oligonucleotides on endocyclic amines allows for complimentary pairing in aqueous solution. The synthetic method is versatile in that it allows for the coupling of different entities on the two free amines during synthesis, prior to formation of the macromolecule.

In yet another approach, the two covalently attached oligonucleotides need not be complementary, such that a third, free oligonucleotide in solution, of appropriate sequence, could be used to stabilize the reported structure. Additionally, if the solution based oligo is from a specimen under investigation, a properly prepared reporter could serve to prime various polymerase chain reactions that would replicate the template DNA.

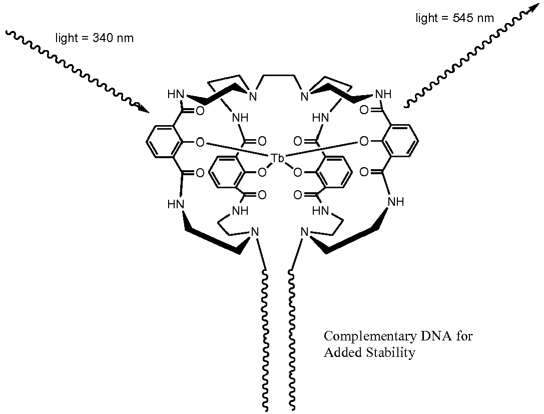

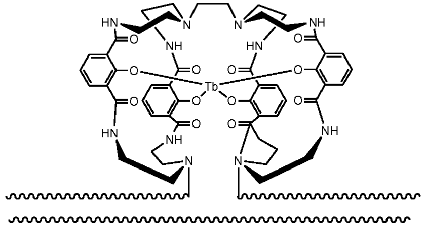

### EXAMPLE 9

### Formation of dsDNA Results in Reporter Attenuation via Structural Disruption of Chelate and Tb loss.

In yet another example, a properly designed chelate is provided with covalently attached oligos that are bound to their compliment sections of a hybridized oligo spanning both segments. An intermediate sequence that lacks complementarity to the reporter, is designed with complementarity with a sequence that is of interest in biological samples. Upon mixing, the loop section hybridizes with the target sequence and the rigid double-stranded oligo forces the reporter open, releasing the Tb and resulting in a subsequent loss of fluorescent signal.

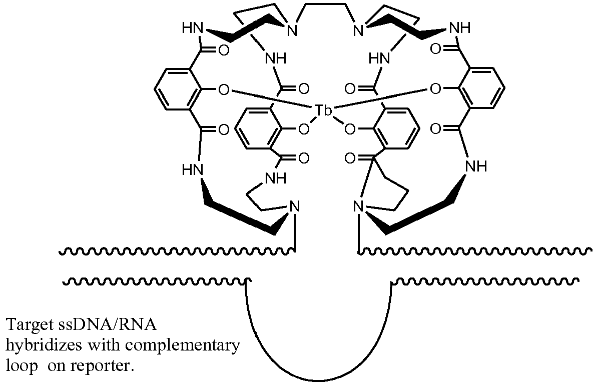

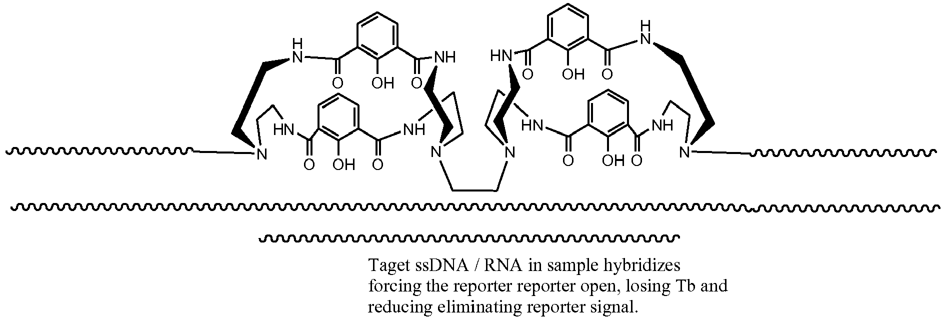

### EXAMPLE 10

### Stepwise synthesis of di-macrocycle (Scheme 6).

Alternative approaches may be used for the large scale manufacture of di-macrocyclic compounds. tert-Butyloxycarbonyl protected diethylenetriamine **17** is condensed with 2-benzyloxy-bis(2-mercaptothiazole)isophthalamide **8,** using pseudo-first order conditions as shown in Scheme 6. Condensation of **18** with diamine **4** under high dilution conditions leads to formation of the mono-macrocycle **42.** Removal of the tert-butyloxycarbonyl protective group with trifluoroacetic acid produces the macrocycle **43.** Alkylation of **43** with 1,2-dibromoethane using pseudo-first order conditions produces alkyl halide **44.** Reaction between benzyloxycarbonyl protected ethylene diamine **45** and the the aldehyde prepared from tert-butyloxycarbonyl and benzyloxycarbonyl protected lysine **46** forms a secondary amine **47,** that is protected as the formamide **48** using triethylorthoformate. Removal of the benzyloxycarbonyl protective groups from **48** under reducing conditions provides the diamine **49,** which is condensed with dithiazolide intermediate **9** under high dilution conditions. This leads to formation of the mono-macrocycle **50.** Removal of the formamide protective group with hydrazine produces the macrocycle **51.** Macrocyclic amine **55** is condensed with macrocyclic bromide **44** to form the protected di-macrocycle **11.** Di-macrocycle **11** is deprotected as shown above in Scheme 1 to form di-macrocycle **12.**

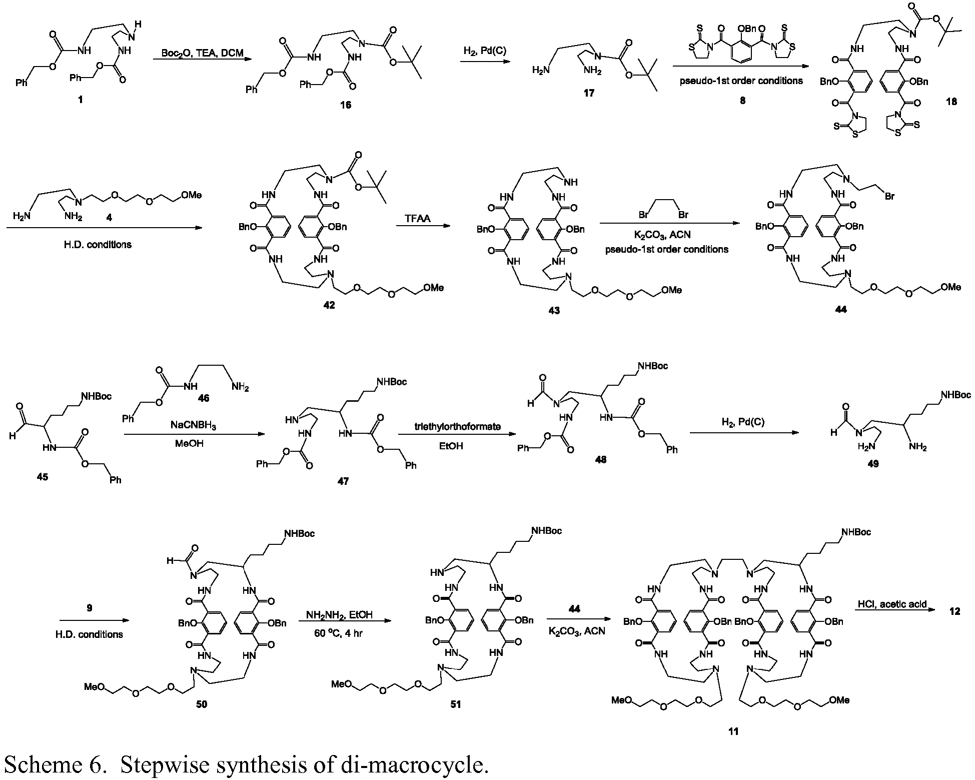

### EXAMPLE 11

### Stepwise synthesis of di-macrocycle (Scheme 7).

Alternative approaches may be used for the large scale manufacture of di-macrocyclic compounds. Reaction between benzyloxycarbonyl protected ethylene diamine **45** and the the aldehyde prepared from tert-butyloxycarbonyl and benzyloxycarbonyl protected lysine **46** forms a secondary amine **47.** As an alternative approach, this amine is reacted with 2-bromoethanol to form the alcohol **52.** Removal of the benzyloxycarbonyl protective groups from **52** under reducing conditions provides the diamine **53,** which is condensed with dithiazolide intermediate **9** under high dilution conditions. This leads to formation of the mono-macrocycle **54.** Activation of the alcohol using p-toluenesulfonyl chloride produces the macrocycle **55.** Macrocyclic amine **55** is used to alkylate macrocyclic amine **43** to form the protected di-macrocycle **11.** Di-macrocycle **11** is deprotected as shown above in Scheme 1 to form di-macrocycle **12.**

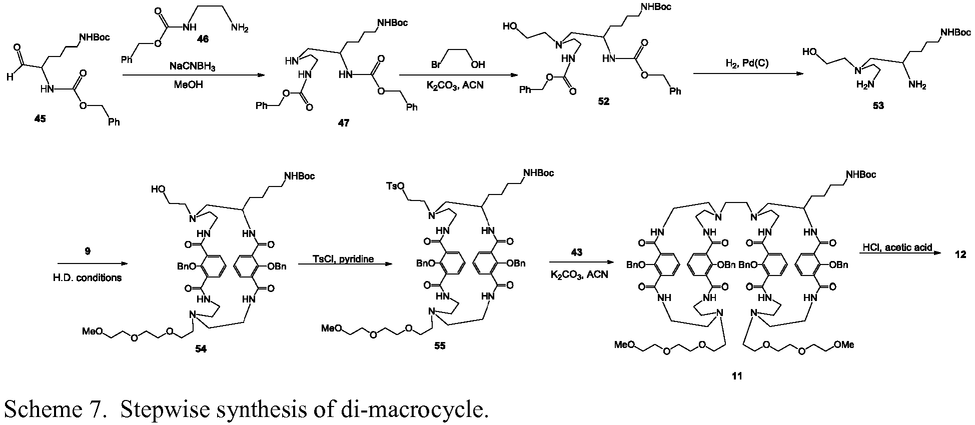

### EXAMPLE 12

### Stepwise synthesis of di-macrocycle (Scheme 8).

Alternative approaches may be used to alter the position of the linker arm in di-macrocyclic compounds. Formamide protected diethylenetriamine **57** is condensed with **8** under pseudo-first order conditions to form dithiazolide **58.** The secondary amine **47** is alkylated to form tertiary amine **59,** which is deprotected under reducing conditions to provide diamine **60.** The dithiazolide **58** is condensed with diamine **60** under high dilution conditions to form macrocycle **61.** The formamide group is removed using hydrazine to form the macrocyclic amine **62,** which is alkylated with the macrocyclic bromide **44** to form the protected di-macrocycle **63.** Di-macrocycle **63** is deprotected under acidic conditions to form di-macrocycle **64.**

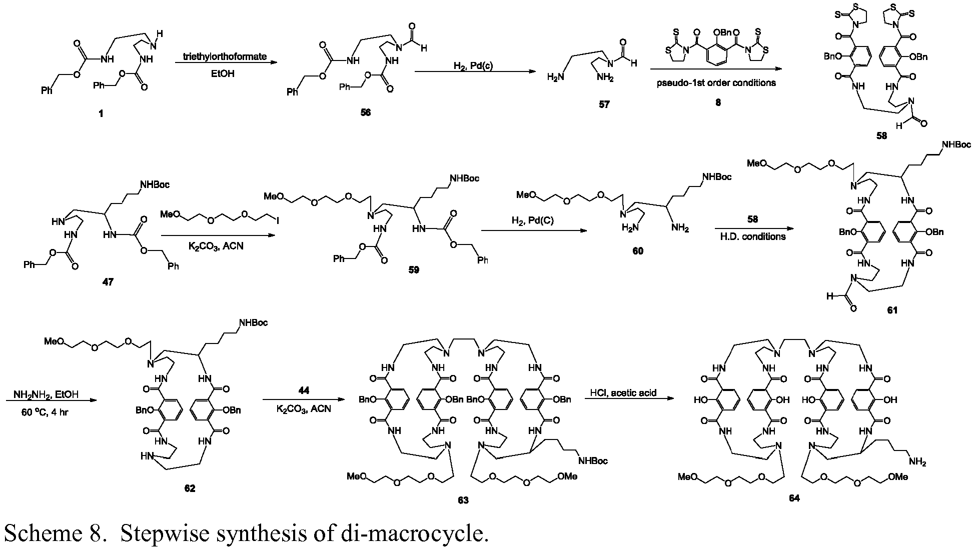

### EXAMPLE 13

### Stepwise synthesis of di-macrocycle (Scheme 9).

Alternative approaches may be used to change the substituents present on the di-macrocycles. Formamide protected dithiazolide **58** is condensed with tetra-amine **10** to form the di-macrocycle **65.** Deprotection of the formamide groups using hydrazine affords the macrocyclic diamine **66.** Alkylation of diamine **66** with a tosylate derived from estradiol, **67,** produces the protected di-macrocycle **68.** Di-macrocycle **68** is deprotected under acidic conditions to form di-macrocycle **69.** The presence of the estradiol-derived moieties on the bifunctional chelator may serve to alter the pharmacokinetics of the derived site-directing molecule, for example by binding to human serum albumin.

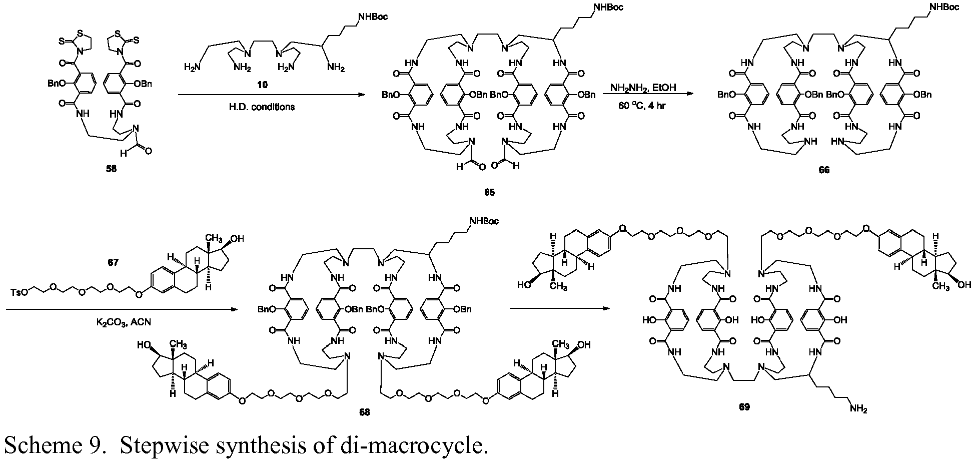

### EXAMPLE 14

### Templated synthesis of di-macrocycle (Scheme 10).

Alternative approaches may be used to manufacture di-macrocycles. Macrocyclic amine **43** is alkylated with 2-bromoethanol to form alcohol **70.** Protective groups are removed under acidic conditions to form macrocycle **71,** which is activated using p-toluenesufonyl chloride in pyridine to afford tosylate **72.** Macrocyclic amine **43** is deprotected under acidic conditions to form macrocycle **73.** Macrocycle **73** and macrocycle **72** are mixed together in an aqueous buffer containing a metal cation such as terbium chloride. Upon metal complexation, the terbium complex **74** is formed. The proximity of the amine and the tosylate increases the rate of reaction to form di-macrocyclic metal complex **75** in improved yield. The metal cation can be removed under acidic conditions. The di-macrocyclic chelator formed from **75** and mono-macrocyclic compounds such as **73** could display utility to sequester metal cations from ores, mining slags, tailings, nuclear waste, or biological systems.

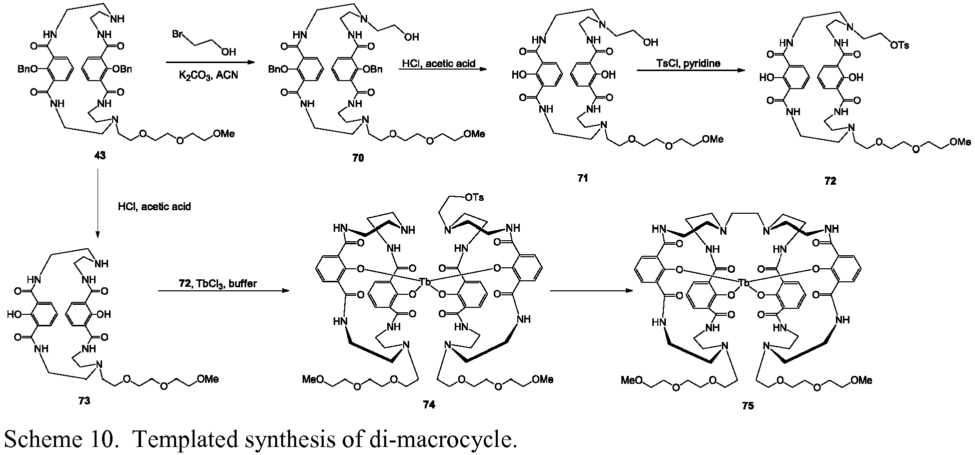

### EXAMPLE 15

### Stepwise synthesis of di-macrocycle (Scheme 11).

Alternative approaches may be used to change the position of the linker arm present on the di-macrocycles. Removal of protective groups from compound **76** under reducing conditions yields the diamine **77.** Removal of protective groups from compound **78** under reducing conditions yields the diamine **79.** Reaction of diamine **79** with di-thiazolide **8** under pseudo-first order conditions forms dithiazolide **80.** This is condensed with diamine **77** under high dilution conditions to form protected macrocycle **81.** Activation of the alcohol moiety present in macrocycle **81** using p-toluenesulfonyl chloride in pyridine forms the tosylate **82.** Macrocylic amine **43** is alkylated with tosylate **82** to provide the protected di-macrocycle **83.** Di-macrocycle **83** is deprotected under acidic conditions to form di-macrocycle **84.**

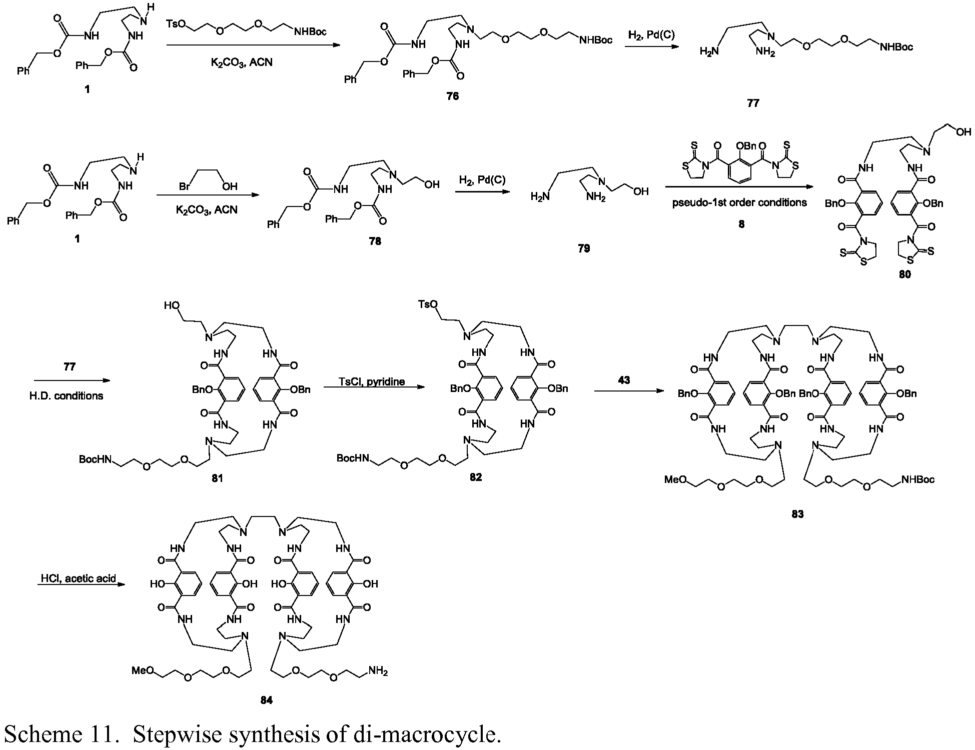

### EXAMPLE 16

### Synthesis of multimeric di-macrocycle species (Scheme 12).

Alternative compositions may be derived by judicious selection of the structural element joining the macrocyclic subunits in di-macrocyclic species. Alkylation of macrocyclic amine **43** with bis-bromomethyl substituted porphyrin **85** yields protected di-macrocycle **86.** Di-macrocycle **86** is deprotected under acidic conditions to form di-macrocycle **87.** Complexation with a metal cation such as terbium(III) in aqueous buffered solution forms a dimer or higher order species such as the coordination polymer **88**. Such supramolecular compositions may display light harvesting properties that are of value for photovoltaic cells, for example.

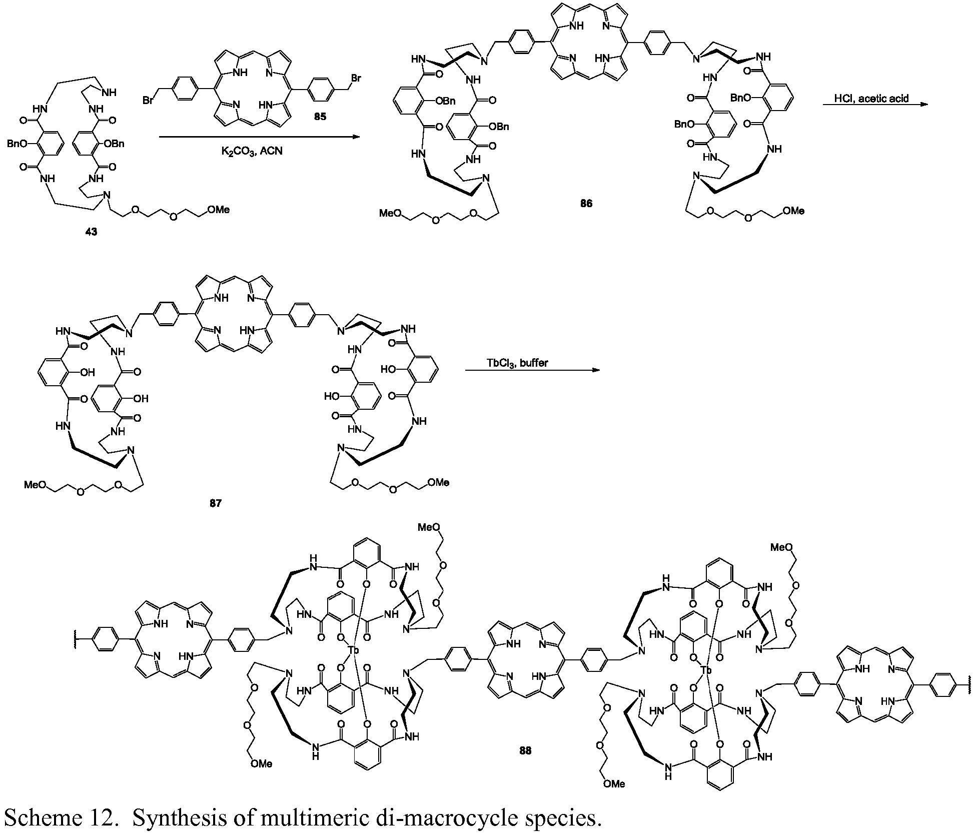

### EXAMPLE 17

### Synthesis of octa-coordinating di-macrocyclic bifunctional chelators (Scheme 13).

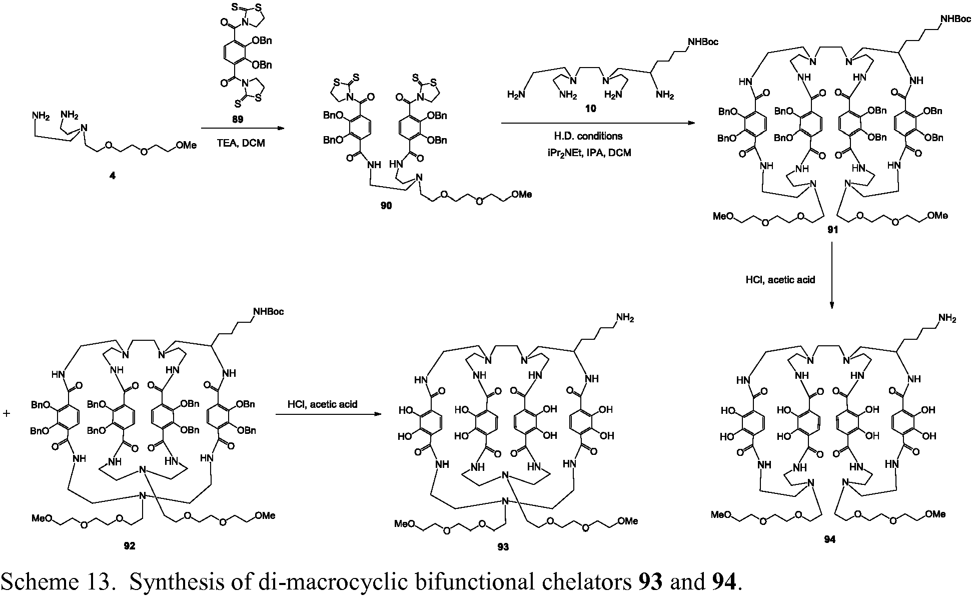

Preparation of terephthalamide macrocyclic ligands began with [2-[2-(2-methoxyethoxy)ethoxy]ethoxy] diethylenetriamine **4,** which was condensed with 2-mercaptothiazole activated amide **89** under pseudo-first order conditions to provide the amide **90,** which was reacted with amine **10** under high dilution conditions to form the di-macrocycles **91** and **92.** Following separation of the two regioisomers by silica gel chromatography, protective groups were removed using a solution of concentrated hydrochloric acid in acetic acid to provide di-macrocycles **93** and **94.** Structural assignment of the regioisomers was performed using tandem mass spectrometry.

2,3-Dibenzyloxy-bis(2-mercaptothiazolide)terephthalamide **89** was synthesized as described (Doble, D.M.J., et al., Inorg. Chem. 2003, 42, 4930-4937.

N,N"-bis[2,3-dibenzyloxy-1-(2-mercaptothiazoleamido)-4-terephthalamido]-N'-[2-[2-(2-methoxyethoxy)ethoxy]ethoxy]-bis(2-aminoethyl)amine **90.** N'-[2-[2-(2-methoxyethoxy)ethoxy]ethoxy]-bis(2-aminoethyl)amine **4** (371 mg, 1.49 mmol) was dissolved in dichloromethane (30 mL) and added using a syringe pump (NE1000) to a solution of 2,3-dibenzyloxy-bis(2-mercaptothiazole)terephthalamide **89** (7.80 g, 13.4 mmol) in dichloromethane (75 mL) over a period of 20 hrs at a rate of 1.50 mL/hr. After a further 22 hr, solvent was removed under reduced pressure, and the crude product was purified by silica gel chromatography using 1 - 2% methanol in dichloromethane as eluents. Fractions containing product were combined, solvent was removed under reduced pressure, and the residue dried in vacuo to provide compound **90** (1.134 g, 65.0%). ¹H NMR (300 MHz, CDCl₃): δ = 7.77 (d, 2H, ArH), 7.35 -7.31 (m, 20H, ArH), 7.18 (d, 2H, ArH), 5.07 (s, 8H, PhCH₂O), 4.36 (t, 4H, NCH₂CH₂S), 3.56 - 3.46 (m, 8H, CH₂CH₂O), 3.38 (t, 2H, CH₂CH₂O), 3.31 - 3.26 (m, 7H, CH₂CH₂N, OMe), 2.92 (t, 4H, NCH₂CH₂S), 2.59 (t, 2H, CH₂CH₂N), 2.47 (t, 4H, CH₂CH₂N). ¹³C NMR (300 MHz, CDCl₃): δ = 201.6, 167.1, 164.6, 150.3, 149.6, 137.3, 136.2, 133.5, 131.0, 129.1, 129.0, 128.9, 128.6, 128.2, 126.8, 124.7, 77.2, 76.4, 72.1, 70.8, 70.7, 70.6, 69.9, 59.3, 55.8, 53.7, 53.5, 38.1, 29.0. FTMS pESI: calculated for C₆₁H₆₆N₅O₁₁S₄ [MH]⁺, 1172.3636, found, 1172.3621.

Benzyl and tert-butyloxycarbonyl-protected di-macrocycles **91** and **92.** A solution of N,N"-bis[2,3-dibenzyloxy-1-(2-mercaptothiazoleamido)-4-terephthalamido]-N'-[2-[2-(2-methoxyethoxy)ethoxy]ethoxy]-bis(2-aminoethyl)amine **90** (1.085 g, 925 µmol) in dichloromethane (50 mL) and a solution of 5-amino-6-[(2-aminoethyl)-[2-[bis(2-aminoethyl)amino]ethyl]amino]hexylcarbamic acid tert-butyl ester **10** (187 mg, 463 µmol) in dichloromethane, isopropyl alcohol (ca. 5%), and diisopropylethylamine (ca. 3%) (50 mL) were added dropwise to dichloromethane (2 L) over a period of four days using two syringe pumps at a rate of 0.5 mL/hr. After an additional two days of reaction, solvent was removed under reduced pressure, and the crude product was purified by silica gel chromatography using 0.1% triethylamine, 5 - 7.5% methanol in dichloromethane as eluents. The silica gel column was prepared so as to have a short section (ca. 1.25") of aluminum oxide (basic, Brockmann I) on its bottom. Di-macrocycle **91** eluted first, with 5% MeOH in dichloromethane. Fractions containing each product were combined, solvent was removed under reduced pressure, and the residues dried in vacuo to provide the protected di-macrocycles **91** and **92** (264 mg and 242 mg, respectively, 24.1%). Di-macrocycle **91:** ¹H NMR (300 MHz, CDCl₃): δ = 7.67 (m, 4H, ArH), 7.29 - 7.25 (m, 40H, ArH), 7.12 -7.00 (m, 4H, ArH), 5.04 - 4.90 (m, 16H, PhCH₂O), 3.54 - 3.29 (m, 26H, CH₂CH₂O, OMe), 2.98 - 2.14 (m, 39H, CH₂CH₂N), 1.67 (m, 4H, CH₂), 1.38 (s, 9H, CH₃), 1.24 (m, 5H, CH, CH₂). ¹³C NMR (600 MHz, CDCl₃): δ = 166.0, 165.8, 155.9, 150.3, 150.2, 136.5, 136.4, 131.8, 128.7, 128.6, 128.4, 128.3, 128.2, 127.8, 125.0, 124.8, 76.7, 76.5, 71.8, 70.5, 70.4, 70.2, 68.8, 68.7, 58.9, 52.4, 51.8, 47.1, 40.3, 37.1, 37.0, 33.6, 29.8, 28.4, 23.4. FTMS pESI: calculated for C₁₂₉H₁₅₇N₁₃O₂₄ [M+2H]²⁺, 1136.0727, found, 1136.0709. Di-macrocycle **92:** ¹H NMR (300 MHz, CDCl₃): δ = 8.17 - 7.57 (m, 4H, ArH), 7.33 - 7.25 (m, 40H, ArH), 7.20 - 6.96 (m, 4H, ArH), 5.29 - 4.93 (m, 16H, PhCH₂O), 3.66 - 3.27 (m, 26H, CH₂CH₂O, OMe), 2.92 - 2.51 (m, 39H, CH₂CH₂N), 1.95 - 1.81 (m, 4H, CH₂), 1.38 (s, 9H, CH₃), 1.24 (m, 5H, CH, CH₂). ¹³C NMR (600 MHz, CDCl₃): δ = 165.7, 165.6, 165.5, 155.9, 150.3, 150.2, 149.9, 136.5, 136.4, 132.1, 128.7, 128.6, 128.5, 128.4, 128.3, 128.0, 124.8, 124.6, 76.7, 76.6, 76.5, 76.3, 71.8, 70.5, 70.3, 70.1, 69.3, 58.9, 54.1, 53.9, 53.5, 53.4, 52.9, 52.3, 52.2, 37.8, 37.7, 37.6, 37.5, 29.6, 28.4, 23.4. FTMS pESI: calculated for C₁₂₉H₁₅₇N₁₃O₂₄ [M+2H]²⁺, 1136.0727, found, 1136.0705.

Di-macrocycle **94.** Benzyl and tert-butyloxycarbonyl-protected di-macrocycle **91** (10 mg, 4.4 µmol) was dissolved in 12N hydrochloric acid (0.5 mL) and glacial acetic acid (0.5 mL). The solution was stirred under inert atmosphere for 44 hr, whereupon HCl was removed with a stream of inert gas. Solvents were removed under reduced pressure and the residue was dried in vacuo. The residue was dissolved in methanol (2 x 200 µL) and transferred to an O-ring microcentrifuge tube. Ether (ca. 1.5 mL) was added, and the tube was placed at 4 °C overnight. The tube was centrifuged at 12,000 rpm for 3 minutes, decanted, the pellet was washed with ether (ca. 1.5 mL) and allowed to air dry. The pellet was dried in vacuo to provide di-macrocycle **94,** pentahydrochloride salt (6.75 mg, 94%). FTMS pESI: calculated for C₆₈H₁₀₁N₁₃O₂₂ [M+2H]²⁺, 725.8587, found, 725.8583. Di-macrocycle **93** was formed from compound **92** following a similar procedure. FTMS pESI: calculated for C₆₈H₁₀₁N₁₃O₂₂ [M+2H]²⁺, 725.8587, found, 725.8590. Tandem mass spectrometry performed on compound **94,** 484.33 MS1 peak [M+3H]³⁺, revealed peaks at mass 352.1688 [M+2H]²⁺, 387.7056 [M+2H]²⁺, 677.3151 [M+H]⁺, and 748.3884 [M+H]⁺, consistent with fragmentation across the ethylene diamine bridge. Similar fragmentation was not observed upon analysis of compound **93.**

### EXAMPLE 18

### Synthesis of an octa-coordinating di-macrocyclic - oligodeoxynucleotide conjugate (Scheme 14).

Di-macrocycle, 4-isothiocyanatophenylthiourea derivative **95.** To di-macrocycle **94** (5.98 mg, 3.7 µmol), dissolved in dimethylformamide (75 µL) and triethylamine (10.2 µL), was added a solution of 1,4-phenyldiisothiocyanate (7.7 mg, 40 µmol) in dimethylformamide (75 µL). The resulting solution was mixed at 800 rpm under inert atmosphere for 1.5 hours. Ether (ca. 1.5 mL) was added, and the resulting suspension placed at 4 °C for 60 minutes. The tube was centrifuged at 12,000 rpm for 3 minutes, decanted, the pellet was washed with ether (ca. 1.5 mL) and allowed to air dry. The pellet was dissolved in methanol (350 µL) and transferred to a new microtube, then precipitated and washed with ether as described above. The pellet was dried in vacuo to provide di-macrocycle, 4-isothiocyanatophenylthiourea derivative **95** (4.71 mg, 78.3%). FTMS pESI: calculated for C₇₆H₁₀₅N₁₅O₂₂S₂ [M+2H]²⁺, 821.8495, found, 821.8492.

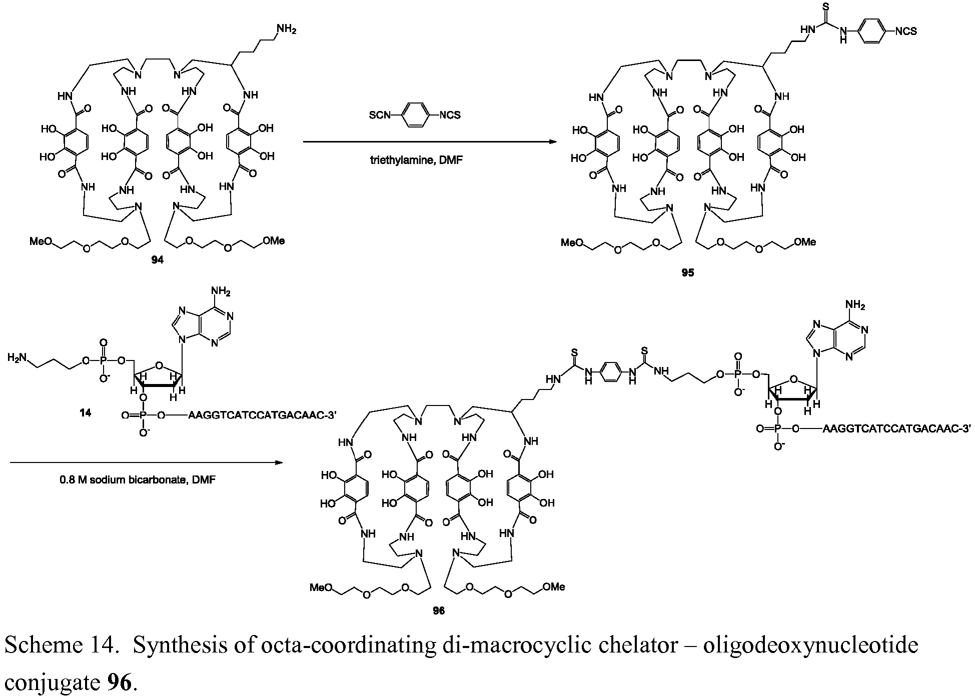

Dimacrocycle - oligodeoxynucleotide conjugate **96.** A DNA 18-base oligonucleotide **(14)** with the sequence 5'- AAGGTCATCCATGACAAC-3' was purchased commercially (Eurogentec, Inc., Seraing, Belgium) and purified using reverse-phase HPLC. The oligonucleotide was modified during synthesis to possess an aminopropyl group attached at the 5'-terminus via a phosphodiester linkage. A solution of DNA oligomer in water (75 µL, 102 nmol) was diluted with sodium bicarbonate buffer (0.8 M, 100 µL) in an eppendorf tube. A solution of di-macrocycle, 4-isothiocyanatophenylthiourea derivative **95** (1.65 mg, 1.00 µmol) in anhydrous DMF (50 µL) was freshly prepared, added to the DNA oligomer and mixed at 1200 rpm using a commercial device (Eppendorf Mixmate®) at ambient temperature for 18 hours. A solution (45 µL) of glycogen (350 µg/mL) in 3M sodium acetate, pH 5.2 was added to the solution. The solution was mixed, absolute ethanol (1.1 mL) was added, the solution was mixed again, and the tube was stored at -20 °C for 1.5 hours. The eppendorf tube was centrifuged at 12,000 rpm for 20 minutes, the supernatant decanted, and the resulting pellet was washed with cold, 70% aqueous ethanol (1.1 mL). The supernatant was decanted, and the pellet was allowed to dry open to the air. The pellet was dissolved in sterile water (100 µL), and an aliquot (2 µL) was removed to quantify by UV-visible absorbance using the extinction coefficient at 260 nm of 181,600 M⁻¹ cm⁻¹. The resulting stock was found to have a concentration of 881 µM (88 nmol, 86% crude yield). There was ca. 70% conversion to conjugate, as estimated from analysis using 20% polyacrylamide gel electrophoresis. The conjugate was used without further purification.

Di-macrocycle **93** was converted to the corresponding isothiocyanate derivative **97** using a protocol similar to that above. Isothiocyanate **97** was coupled with oligonucleotide **14** to provide the corresponding di-macrocycle - oligodeoxynucleotide conjugate **98.**

### EXAMPLE 19

### Synthesis of an octacoordinating di-macrocyclic chelator maleimide derivative.

As an alternative to amine-reactive isothiocyanate functionality, a thiol-reactive maleimide bifunctional chelator may be prepared from di-macrocyclic chelators as illustrated in the example below:

Di-macrocycle, 3-maleimidopropyl derivative **99.** Di-macrocycle **12** (10.12 mg, 6.45 µmol), was dissolved in dimethylformamide (100 µL) and triethylamine (10 µL), and added to 3-maleimidoproprionic acid, N-hydroxysuccinimide ester (3.4 mg, 13 µmol). The resulting solution was mixed at 800 rpm under inert atmosphere for 1 hour. Ether (ca. 1.5 mL) was added, and the resulting suspension placed at 4 °C for 60 minutes. The tube was centrifuged at 12,000 rpm for 3 minutes, decanted, the pellet was washed with ether (ca. 1.5 mL) and allowed to air dry. The pellet was dissolved in methanol (300 µL) then precipitated and washed with ether as described above. The pellet was dried in vacuo to provide di-macrocycle, 3-maleimidopropyl derivative **99** (7.42 mg, 74.8%). FTMS pESI: calculated for C₇₅H₁₀₅N₁₄O₂₁ [M+H]⁺, 1537.7573, found, 1537.7561.

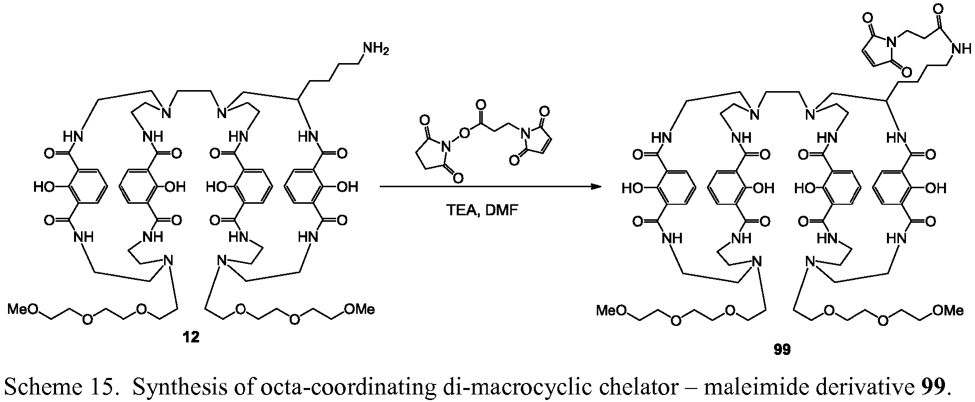

### EXAMPLE 20

### Synthesis of an octa-coordinating di-macrocyclic bifunctional chelator (Scheme 16).

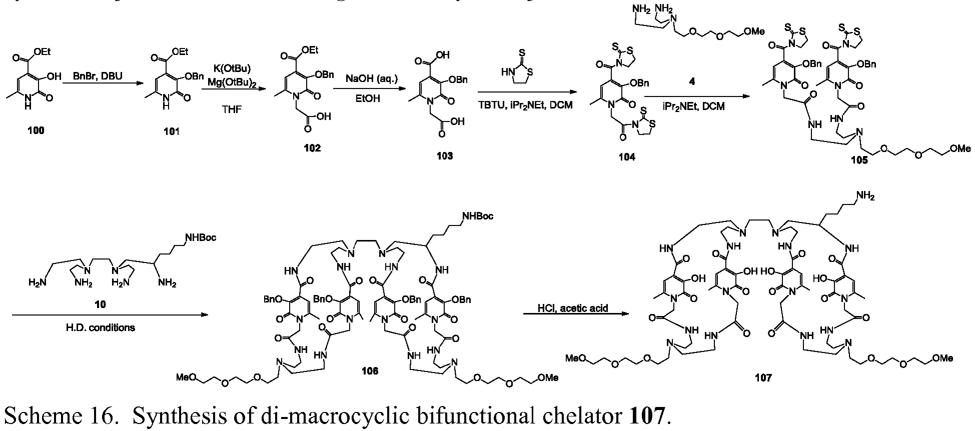

Preparation of a 3,2-HOPO macrocyclic ligand began with 3-hydroxy-6-methyl-2-oxo-1,2-dihydropyridine-4-carboxylic acid ethyl ester **100,** which was alkylated with benzyl bromide to provide benzyl ether **101.** Alkylation of **101** with bromoacetic acid provided monoester **102,** which was saponified to provide diacid **103.** Diacid **103** was activated with O-(benzotriazol-1-yl)-N,N,N',N'-tetramethyluronium tetrafluoroborate (TBTU) in the presence of 2-mercaptothiazole to provide di-thiazolide **104.** Di-thiazolide **104** was condensed with [2-[2-(2-methoxyethoxy)ethoxy]ethoxy] diethylenetriamine **4** under pseudo-first order conditions to provide the activated di-amide **105,** which was reacted with amine **10** under high dilution conditions to form the di-macrocycle **106.** Protective groups were removed using a solution of concentrated hydrochloric acid in acetic acid to provide di-macrocycle **107.**

3-Hydroxy-6-methyl-2-oxo-1,2-dihydropyridine-4-carboxylic acid ethyl ester **100** was synthesized as described (Doble, D.M.J., et al., Inorg. Chem. 2003, 42, 4930-4937.

3-Benzyloxy-6-methyl-2-oxo-1,2-dihydropyridine-4-carboxylic acid ethyl ester **101.** Compound **100** (24.59 g, 124.8 mmol) and 1,8-diazabicycloundec-7-ene (DBU, 28.32 g, 186.0 mmol) were mixed together in isopropyl alcohol (400 mL). The mixture was stirred under N₂(g) and heated to slow reflux. Benzyl bromide (25.60 g, 149.7 mmol) was added dropwise to the isopropyl alcohol mixture under N₂(g). The mixture was stirred under reflux (82-83 °C) for 4 hr. The reaction was allowed to cool to ambient temperature, resulting in a dark brown solution. Solvent was removed under reduced pressure, the residue was dissolved in dichloromethane (40 mL) and the resulting solution was washed with 3M aqueous HCl (2 x 60 mL) to remove DBU. The organic phase was then washed with Millipore water (3 x 60 mL). The solution was dried over magnesium sulfate, filtered, and dried in vacuo to produce a dark brown oil. The product was precipitated by addition of diisopropyl ether (50 mL) and stirred overnight. The precipitate was filtered and dried in vacuo to provide **101** (20.56 g, 57%). ¹H NMR (CDCl₃ + TMS, 300 MHz): δ = 1.28 (t, 3H, CH₃, J = 7.2), 2.33 (s, 3H, CH₃), 4.28 (q, 2H, CH₂, J = 7.2), 5.26 (s, 2H, CH₂), 6.16 (s, 1H, CH), 7.30-7.37 (m, 3H, Ph), 7.49-7.51 (m, 3H, Ph), 12.66 (s, 1H, NH); ¹³C NMR (CDCl₃ + TMS, 400 MHz): δ = 165.3, 162.0, 144.6, 139.4, 137.3, 133.2, 128.57, 128.29, 128.06, 104.3, 74.0, 61.7, 18.61, 14.4, ESI-MS (+), [M + H]⁺ *m*/*z* = 288.1232 (C₁₆H₁₈NO₄, expected 288.1238). [M+H]⁺ calculated for C₁₆H₁₈NO₄ 288.1236, [M+Na]⁺ calculated for C₁₆H₁₇NO₄Na 310.1055, found 310.1055 m/z, mp= 232.4-235.7°C.

3-Benzyloxy-1-carboxymethyl-6-methyl-2-oxo-1,2-dihydropyridine-4-carboxylic acid ethyl ester **102.** Compound **101** (8.15 g, 28 mmol), magnesium tert-butoxide (9.68 g, 56 mmol), and potassium tert-butoxide (3.35 g, 29 mmol) were purged with N₂ then dissolved in anhydrous tetrahydrofuran (28 mL). Bromoacetic acid (9.86 g, 71.0 mmol) was dissolved in anhydrous tetrahydrofuran (5 mL) and added dropwise to the previous mixture under N₂. The mixture was stirred at room temperature for 4 hours, whereupon 3M HCl (30 mL) was added. Organic material was extracted with dichloromethane (20 mL x 3) and the combined organic extracts were dried over magnesium sulfate, filtered, and solvent removed under reduced pressure. Diisopropyl ether was added to the residue to form a precipitate that was filtered and dried in vacuo to provide compound **102** (7.43 g, 75%). IR (cm⁻¹): 2981 (s), 2511 (w), 1733 (s), 1716 (vs), 1645 (s), 1545 (vs), 1471 (s), 1455 (s), 1404 (vs), 1385 (s), 1365 (s), 1337 (s), 1250 (vs), 1200 (vs), 1097 (s), 1050 (s), 1016 (s), 953 (s), 916 (s), 881 (w), 865 (vw), 846 (s), 765 (s). ¹H NMR (CDCl₃ + TMS, 400 MHz): δ = 1.29 (t, 3H, CH₃, *J* = 7.1 Hz), 2.28 (s, 3H, CH₃), 4.30 (q, 2H, CH₂, *J* = 7.1 Hz), 4.78 (s, 2H, CH₂), 5.21 (s, 2H, CH₂), 6.28 (s, 1H, CH), 7.27-7.35 (m, 3H, Ph), 7.45-7.53 (m, 2H, Ph), 8.17 (s, 1H, COOH). ¹³C NMR (CDCl₃ + TMS, 400 MHz): δ = 170.1, 164.9, 161.0, 145.0, 140.1, 136.8, 131.4, 128.6, 128.3, 128.1, 105.5, 74.3, 61.8, 46.5, 20.2, 14.1. ESI-MS (-): *m*/*z* 344.1149 [M-H]⁻ (C₁₈H₁₈NO₆, expected 344.1134). mp: 242.9-245.7 °C.

3-Benzyloxy-1-carboxymethyl-6-methyl-2-oxo-1,2-dihydropyridine-4-carboxylic acid **103.** Sodium hydroxide (0.2 g, 4 mmol) was dissolved in water (5 mL) and added slowly to a solution of compound **102** (865 mg, 2.5 mmol) in ethanol (30 mL). The solution was stirred at ambient temperature overnight, whereupon solvent was removed under reduced pressure. The residue was dissolved in water and the mixture was cooled in an ice bath and acidified. The white precipitate which formed was filtered and dried in vacuo to provide compound **103.** ¹H NMR (DMSO-d⁶, 300 MHz): δ = 7.42-7.26 (m, 5H), 6.19 (s, 1H), 5.01 (s, 2H), 4.70 (s, 2H), 2.19 (s, 3H).

3-Benzyloxy-1-carbonyl(2-mercaptothiazolide)methyl-6-methyl-2-oxo-1,2-dihydropyridine-4-carbonyl(2-mercaptothiazolide) **104.** Diacid **103** (1.00 g, 3.15 mmol), TBTU (2.4 g, 7.57 mmol), 4-dimethylaminopyridine (DMAP, 35 mg, 28 µmol, 0.09 eq.), and 2-mercaptothiazoline (789 mg, 6.62 mmol) were suspended in anhydrous dichloromethane (20 mL). Diisopropylethylamine (1.63 g, 12.6 mmol) was added dropwise to form a solution. After one hour, solvent was removed under reduced pressure and the residue was purified by silica gel chromatography to provide compound **104** (1.228 g, 75%). ¹HNMR (CDCl₃, 300 MHz): δ = 7.41-7.31 (m, 5H), 6.01 (s, 1H), 5.62 (s, 2H), 5.25 (s, 2H), 4.6 (t, 2H, 7.5Hz), 4.29, t, 2H, 7.5), 3.41 (t, 2H, 7.5Hz), 2.87 (t, 2H, 7.5Hz), 2.28 (s, 3H). ¹³C NMR (CDCl₃, 400 MHz): δ = 202.1, 200.8, 168.2, 166.4, 159.3, 141.5, 140.5, 137.6, 133.0, 128.4, 128.4, 128.1, 104.3, 73.9, 55.9, 55.1, 51.3, 29.4, 29.2, 20.5. FTMS pESI: calculated for C₂₂H₂₁N₃O₄S₄ [M+H]⁺, 520.0493; found, 520.0484.

N,N"-bis[3-benzyloxy-1-carbamidomethyl-6-methyl-2-oxo-1,2-dihydropyridine-4-carbonyl(2-mercaptothiazolide)]-N'-[2-[2-(2-methoxyethoxy)ethoxy]ethoxy]-bis(2-aminoethyl)amine **105.** N'-[2-[2-(2-methoxyethoxy)ethoxy]ethoxy]-bis(2-aminoethyl)amine **4** (382 mg, 1.54 mmol) was dissolved in dichloromethane (33 mL) and diisopropylethylamine (0.8 mL) and added using a syringe pump (NE1000) to a solution of 3-benzyloxy-1-carbonyl(2-mercaptothiazolide)methyl-6-methyl-2-oxo-1,2-dihydropyridine-4-carbonyl(2-mercaptothiazolide) **104** (1.99 g, 38.3 mmol) in dichloromethane (50 mL) over a period of 23 hrs at a rate of 1.50 mL/hr. After a further 24 hr, solvent was removed under reduced pressure, and the crude product was purified by silica gel chromatography using 0.1% triethylamine, 2 - 3.5% methanol in dichloromethane as eluents. Fractions containing product were combined, solvent was removed under reduced pressure, and the residue dried in vacuo to provide compound **105** (997 mg, 60.7%). ¹H NMR (600 MHz, CDCl₃): δ = 7.45 - 7.30 (m, 10H, PhH), 6.21 (s, 2H, ArH), 5.19 (s, 4H, PhCH₂O), 4.76 (4H, s, CH₂C=O), 4.29 (t, 4H, NCH₂CH₂S), 3.61 - 3.51 (m, 10H, CH₂O), 3.35 (s, 3H, OCH₃), 3.30 (m, 4H, CH₂NC=O), 2.89 (t, 4H, NCH₂CH₂S), 2.66 (m, 6H, CH₂N), 2.36 (s, 6H, CH₃). ¹³C NMR (600 MHz, CDCl₃): δ = 200.7, 167.0, 165.9, 159.6, 141.8, 141.3, 137.7, 133.2, 128.4, 128.2, 128.1, 104.1, 73.8, 71.8, 70.6, 70.4, 70.2, 58.9, 55.1, 54.4, 52.9, 48.2, 38.0, 29.1, 20.5. FTMS pESI: calculated for C₄₉H₆₀N₇O₁₁S₄ [MH]⁺, 1050.3228, found, 1050.3223.

Benzyl and tert-butyloxycarbonyl-protected di-macrocycle **106.** A solution of N,N"-bis[3-benzyloxy-1-carbamidomethyl-6-methyl-2-oxo-1,2-dihydropyridine-4-carbonyl(2-mercaptothiazolide)] -N'- [2- [2-(2-methoxyethoxy)ethoxy] ethoxy] -bis(2-aminoethyl)amine **105** (924 mg, 880 µmol) in dichloromethane (49.5 mL) and triethylamine (0.5 mL) and a solution of 5 -amino-6- [(2-aminoethyl)-[2- [bis(2-aminoethyl)amino] ethyl] amino]hexylcarbamic acid tert-butyl ester **10** (213 mg, 528 µmol) in dichloromethane, isopropyl alcohol (ca. 5%), and diisopropylethylamine (ca. 3%) (50 mL) were added dropwise to dichloromethane (2 L) over a period of four days using two syringe pumps at a rate of 0.5 mL/hr. After an additional two days of reaction, solvent was removed under reduced pressure, and the crude product was purified by silica gel chromatography using 0.1% triethylamine, 5 - 7.5% methanol in dichloromethane as eluents. The silica gel column was prepared so as to have a short section (ca. 1.25") of aluminum oxide (basic, Brockmann I) on its bottom. Fractions containing product were combined, solvent was removed under reduced pressure, and the residue dried in vacuo to provide the protected di-macrocycle **106** (202 mg, 22.7%). ¹H NMR (300 MHz, CDCl₃): δ = 7.45 - 7.29 (m, 20H, PhH), 6.48 - 6.37 (m, 4H, ArH), 5.56 - 4.78 (m, 8H, PhCH₂O), 4.77 (8H, br s, CH₂C=O), 3.76 - 3.57 (m, 20H, CH₂O), 3.36 (s, 6H, OCH₃), 2.90 (m, 17H, CH₂NC=O, CHNC=O), 2.65 - 2.33 (m, 24H, CH₂N), 2.18 (m, 12H, CH₃), 1.39 (s, 9H, CH₃), 1.03 - 0.98 (m, 6H, CH₂). ¹³C NMR (600 MHz, CDCl₃): δ = 167.5, 163.5, 160.4, 155.9, 128.9, 128.6, 128.5, 128.4, 128.3, 74.5, 74.4, 71.9, 71.0, 70.5, 58.9, 52.9, 46.2, 40.4, 38.9, 28.4, 23.1, 20.3, 20.1, 20.0, 19.9, 11.6, 8.0. FTMS pESI: calculated for C₁₀₅H₁₄₅N₁₇O₂₄ [M+2H]²⁺, 1014.0319, found, 1014.0342.

Di-macrocycle **107.** Benzyl and tert-butyloxycarbonyl-protected di-macrocycle **106** (51 mg, 25 µmol) was dissolved in 12N hydrochloric acid (1.0 mL) and glacial acetic acid (1.0 mL). The solution was stirred under inert atmosphere for 23 hr, whereupon HCl was removed with a stream of inert gas. Solvents were removed under reduced pressure and the residue was dried in vacuo. The residue was dissolved in methanol (600 + 300 µL) and transferred to two O-ring microcentrifuge tubes. Ether (ca. 1.5 mL) was added, and the tubes were placed at 4 °C for 1 hr. The tubes were centrifuged at 12,000 rpm for 3 minutes, decanted, the pellets were washed with ether (ca. 1.5 mL) and allowed to air dry. The pellets were dried in vacuo to provide di-macrocycle **107,** pentahydrochloride salt (39.8 mg, 90%). FTMS pESI: calculated for C₇₂H₁₁₃N₁₇O₂₂ [M+2H]²⁺, 783.9118, found, 783.9140.

### EXAMPLE 21

### Synthesis of di-macrocyclic chelator metal cation complexes (Scheme 17).

Metal cation complexes of di-macrocyclic chelators may be prepared readily, for example, by treatment with the metal cation as a solution in methanol in the presence of a tertiary amine as described below. Stock solutions of the pentahydrochloride salts of chelators **94, 12,** and **107** were prepared at a concentration of 50 mg/mL in methanol (ca. 30 mM). Triethylamine (10 µL, ca. 12 molar equivalents) was added to each stock solution to free the base. Stock solutions of metal cation salts were prepared at a concentration of 5 mM in methanol. Chelator **94, 12,** or **107** (20 µL) was added to the metal cation salt solution (122.4 µL, ca. 1 molar equivalent) at ambient temperature in a 2 mL microcentrifuge tube. A precipitate formed immediately. After standing for 30 minutes, diethyl ether (ca. 1.8 mL) was added, and the samples were stored at 4 °C for 15 minutes. The samples were centrifuged for 3 minutes at 12,000 rpm, whereupon the supernatants were decanted and the pellets allowed to air dry. Samples were analyzed in methanol by mass spectrometry, with results reported below. The europium(III) and terbium(III) complexes of chelator **12** were noted to be luminescent when viewed using a long wavelength (365 nm) UV lamp. Absorption and emission spectra for these species were obtained. Metal cation salts tested include europium(III) chloride hexahydrate (99.99%), terbium chloride hexahydrate (99.9%), thorium nitrate hydrate (99.8%), zirconium acetate (16% solution in dilute acetic acid, diluted in methanol to 5 mM concentration), gadolinium nitrate hydrate (99.9%), lutetium chloride hydrate (99.99+%), yttrium chloride hydrate (99.99%), and dysprosium chloride hydrate (99.99%).

### Results:

**94**·Eu: FTMS pESI: calculated for C₆₈H₉₈N₁₃O₂₂Eu [M]²⁺, 799.8069, found, 799.8087.
**12·**Eu: FTMS pESI: calculated for C₆₈H₉₈N₁₃O₁₈Eu [M]²⁺, 767.8170, found, 767.8195.
**107·**Eu: FTMS pESI: calculated for C₇₂H₁₁₀N₁₇O₂₂Eu [M]²⁺, 857.8599, found, 857.8622.
**94**·Tb: FTMS pESI: calculated for C₆₈H₉₈N₁₃O₂₂Tb [M]²⁺, 803.8096, found, 803.8129.
**12·**Tb: FTMS pESI: calculated for C₆₈H₉₈N₁₃O₁₈Tb [M]²⁺, 771.8198, found, 771.8224.
**107**·Tb: FTMS pESI: calculated for C₇₂H₁₁₀N₁₇O₂₂Tb [M]²⁺, 861.8627, found, 861.8643.
**94**·Th: FTMS -pESI: calculated for C₆₈H₉₃N₁₃O₂₂Th [M]²⁻, 837.8475, found, 837.8511.
**12**·Th: FTMS -pESI: calculated for C₆₈H₉₄N₁₃O₁₈Th [M]⁻, 1612.7226, found, 1612.7273.
**107**·Th: FTMS pESI: calculated for C₇₂H₁₀₉N₁₇O₂₂Th [M]²⁺, 897.9151, found, 897.9172.
**94**·Zr: FTMS -pESI: calculated for C₆₈H₉₂N₁₃O₂₂Zr [M]⁻, 1532.5532, found, 1532.5450.
**94**·Y: FTMS pESI: calculated for C₆₈H₉₈N₁₃O₂₂Y [M]²⁺, 768.7998, found, 768.8015.
**12**·Y: FTMS pESI: calculated for C₆₈H₉₈N₁₃O₁₈Y [M]²⁺, 736.8100, found, 736.8113.
**107**·Y: FTMS pESI: calculated for C₇₂H₁₁₀N₁₇O₂₂Y [M]²⁺, 826.8529, found, 826.8547.
**94**·Gd: FTMS pESI: calculated for C₆₈H₉₈N₁₃O₂₂Gd [M]²⁺, 803.3090, found, 803.3117.
**12**·Gd: FTMS pESI: calculated for C₆₈H₉₈N₁₃O₁₈Gd [M]²⁺, 771.3191, found, 771.3220.
**107**·Gd: FTMS pESI: calculated for C₇₂H₁₁₀N₁₇O₂₂Gd [M]²⁺, 861.3621, found, 861.3652.
**94**·Dy: FTMS pESI: calculated for C₆₈H₉₈N₁₃O₂₂Dy [M]²⁺, 806.3115, found, 806.3148.
**12**·Dy: FTMS pESI: calculated for C₆₈H₉₈N₁₃O₁₈Dy [M]²⁺, 774.3217, found, 774.3245.
**107**·Dy: FTMS pESI: calculated for C₇₂H₁₁₀N₁₇O₂₂Dy [M]²⁺, 864.3646, found, 864.3676.
**94**·Lu: FTMS pESI: calculated for C₆₈H₉₈N₁₃O₂₂Lu [M]²⁺, 811.8173, found, 811.8201.
**12**·Lu: FTMS pESI: calculated for C₆₈H₉₈N₁₃O₁₈Lu [M]²⁺, 779.8275, found, 779.8300.
**107**·Lu: FTMS pESI: calculated for C₇₂H₁₁₀N₁₇O₂₂Lu [M]²⁺, 869.8704, found, 869.8720.

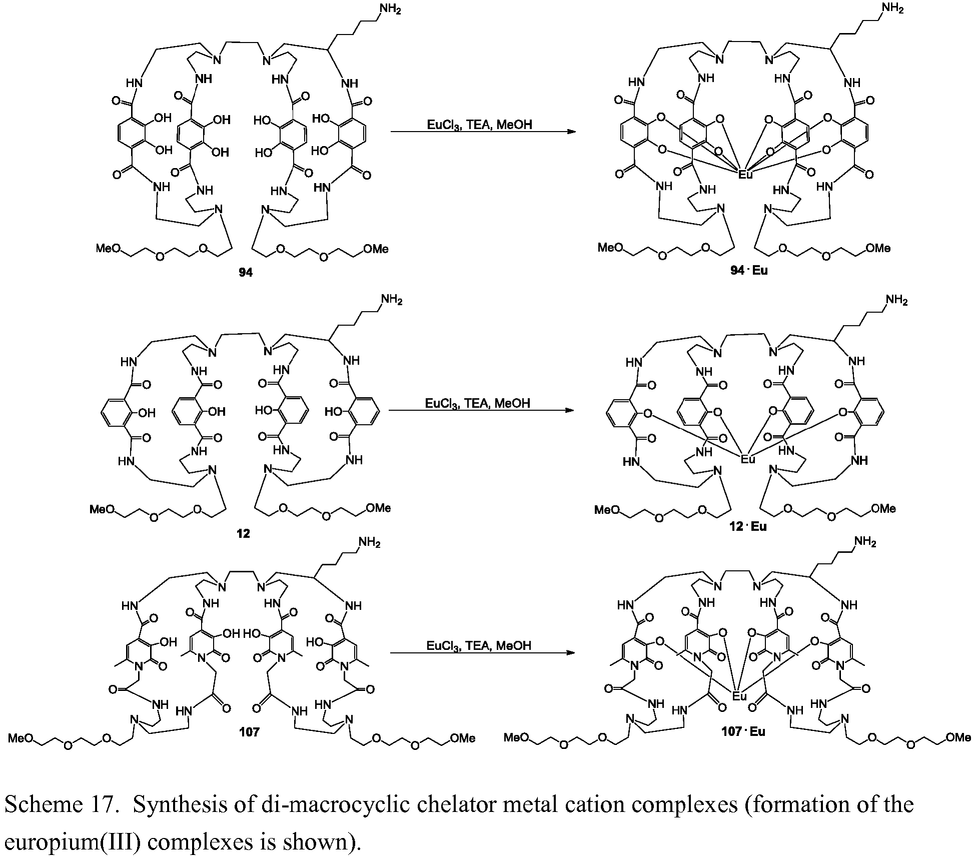

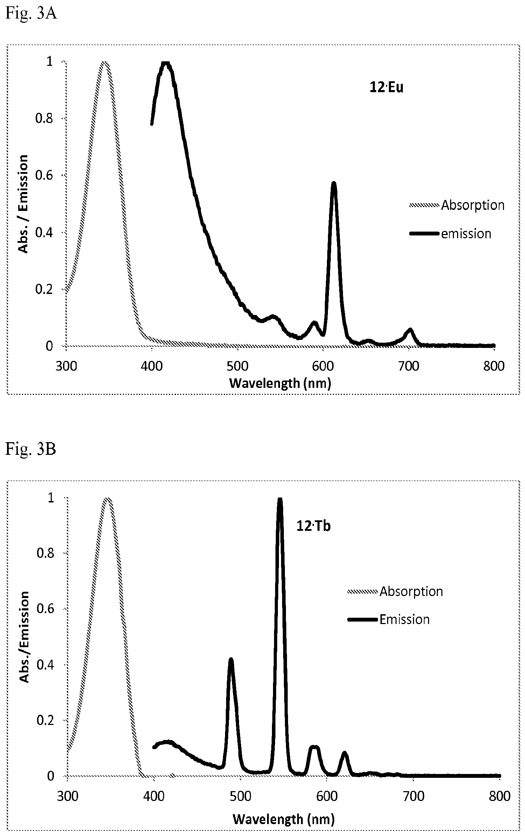

Figure 3A shows absorption and emission spectra of di-macrocyclic chelator **12** with europium(III). Figure 3B shows absorption and emission spectra of di-macrocyclic chelator **12** with terbium(III).

### EXAMPLE 22

### Synthesis of an octa-coordinating di-macrocyclic bifunctional chelator (Scheme 18).

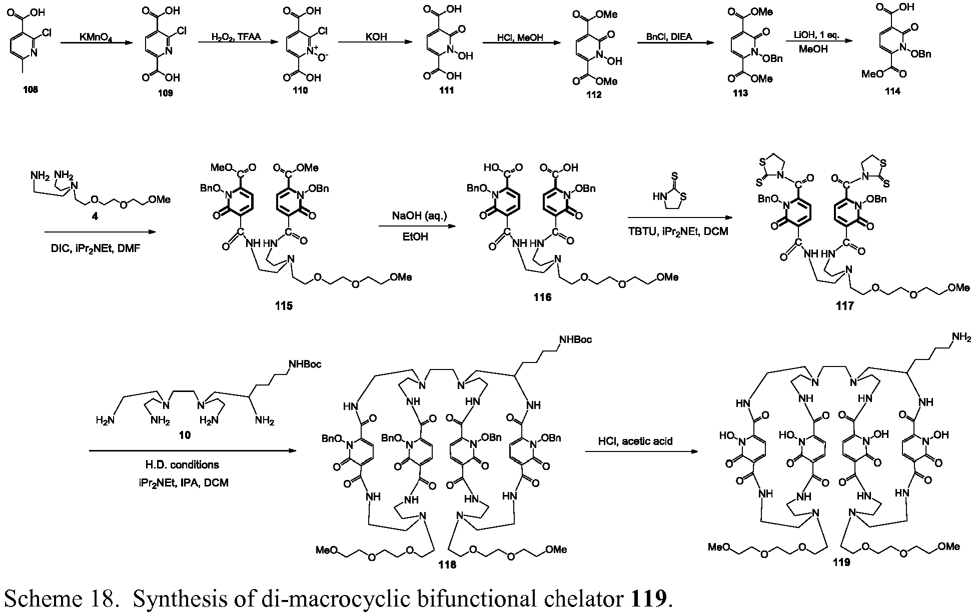

Preparation of a 1,2-HOPO macrocyclic ligand began with 2-chloro-6-methyl-nicotinic acid **108,** which was oxidized with potassium permanganate to provide 2-chloro-pyridine-3,6-dicarboxylic acid **109.** Oxidation of **109** with hydrogen peroxide under acidic conditions provided the pyridine-N-oxide **110,** which was heated with sodium hydroxide to provide 1-hydroxy-3,6-dicarboxy-2(1H)pyridinone **111.** Diacid **111** was treated with methanol under acidic conditions to provide methyl diester **112.** Diester **112** was alkylated with benzyl chloride to provide the benzyl ether **113.** Ether **113** was selectively saponified using lithium hydroxide to provide the lithium salt of mono-acid, monomethyl ester **114,** which was condensed with [2-[2-(2-methoxyethoxy)ethoxy]ethoxy] diethylenetriamine **4** using diisopropylcarbodiimide to provide di-amide **115.** Diamide **115** is saponified with sodium hydroxide to provide the diacid **116,** which may be converted to the the bis(2-mercaptothiazolide) derivative **117** by treatment with 2-mercaptothiazoline in the presence of a uronium salt such as TBTU in the presence of a tertiary amine. Active amide **117** is reacted with amine **10** under high dilution conditions to form the di-macrocycle **118.** Protective groups are removed using a solution of concentrated hydrochloric acid in acetic acid to provide di-macrocycle **119.**

2-Chloro-pyridine-3,6-dicarboxylic acid **109.** To a mixture of 2-chloro-6-methyl-nicotinic acid **108** (25 g, 0.146 mol) and water (1.5 L) in a 3 liter flask equipped with a mechanical stirrer and a heating mantle, potassium hydroxide (15 g, 0.26 mol) was added to form a clear light brown solution. The solution was heated so that the temperature of the reaction mixture was kept in the range of 85-95 °C during the oxidation process. Potassium permanganate was added in 2 - 4 gram portions, with the successive portion added only after the pink color from last portion of potassium permanganate had disappeared. The oxidation was monitored by HPLC and proton NMR (in D₂O-NaOD). Additional potassium permanganate (70 g, 0.44 mol) was used to complete the reaction. The reaction mixture was filtered while it was hot to remove the large amount of MnO₂, and the MnO₂ filter cake washed with boiling water (0.5 L). The combined filtrates were acidified with conc. HCl. The white crystals that formed were collected by filtration and dried under reduced pressure to provide **109** (22 g, 75%). ¹H NMR (500 MHz, DMSO-d₆): δ = 8.12 (d, 1H, J = 7.5 Hz), ArH), 8.34 (d, 1H, J = 7.5 Hz, ArH), 13.85 (br s, 2H, COOH). ¹³C NMR (125 MHz, DMSO-d6, 25 °C): δ = 124.1, 131.4, 141.4, 147.7, 150.0, 164.6, 165.8. (-)-High resolution ESI MS: m/z: 199.9750 [M⁻], calculated 199.9751.

2-Chloro-pyridine-N-oxide-3,6-dicarboxylic acid **110.** 2-Chloro-pyridine-3,6-dicarboxylic acid (22 g , 0.109 mol) was dissolved in 300 mL of trifluoroacetic acid and 30% H₂O₂ (40 mL) was added to this solution while stirring. The solution was heated to 80 °C, and the reaction progress was monitored by HPLC. After the reaction finished, the reaction mixture was concentrated to ca. 50 mL by rotary evaporation and then added to ice water (500 mL). The product immediately precipitated as a finely divided, white crystalline solid. It was isolated by filtration, washed with ice water, and dried in vacuum to provide compound **110** (20 g, 84%). ¹H NMR (300 MHz, DMSO-*d₆*): δ = 8.06 (d, 1H, J = 7.4 Hz, ArH), 8.21(d, 1H, J = 7.4 Hz, ArH). ¹³C NMR (75 MHz, DMSO-d₆, 25 °C): δ = 125.9, 130.6, 133.8, 139.9, 140.5, 160.3, 163.6.

1-Hydroxy-3,6-dicarboxy-2(1H)pyridinone **111.** 2-Chloro-pyridine-N-oxide-3,6-dicarboxylic acid (20 g, 92 mmol) was dissolved in 25% aqueous KOH (250 mL), and the resulting solution was maintained at 80 °C overnight. Reaction progress was monitored by HPLC. Upon completion of the reaction, the solution was cooled in an ice bath and treated with concentrated hydrochloric acid until the acidity of solution reached about pH 2. The brown-yellow suspended solid was isolated by filtration, washed with dilute HCl and cold water (3 x 15 mL), and dried in vacuo to provide compound **111** (15.5 g, 80%). ¹H NMR (300 MHz, DMSO-d₆): δ = 7.19 (d, 1H, J = 7.5 Hz, ArH), 8.06 (d, 1H, J = 7.5 Hz, ArH), 15.35 (br s, 2H, COOH). ¹³C NMR (75 MHz, D₂O-NaOD, 25 °C): δ = 102.6, 123.3, 133.3, 148.1, 160.8, 170.3, 175.2.

Dimethyl 1-hydroxy-6-oxo-1,6-dihydropyridine-2,5-dicarboxylate **112.** Compound **111** (10 g, 50.2 mmol) was suspended in methanol (150 mL) in a 500 mL flask. The suspension was stirred while dry HCl gas was dispersed into the vessel for 15 minutes to form a partial solution. The reaction mixture was heated at reflux overnight, whereupon HPLC revealed that the esterification was finished. The solution was concentrated and the product deposited as crystalline material. This was collected by filtration and dried in a vacuum oven at 40 °C to provide compound **112** (10.3 g, 90%). ¹H NMR (300 MHz, DMSO-*d₆*): δ = 3.92 (s, 3H, CH₃), 3.99 (s, 3H, CH₃), 7.81 (d, 1H, J =8.4 Hz, ArH), 8.56 (d, 1H, J = 8.4 Hz, ArH). ¹³C NMR (75 MHz, DMSO-d₆): δ = 53.8, 54.8, 116.6, 117.9, 135.3, 142.3, 154.9, 157.9, 161.7.

Dimethyl 1-(benzyloxy)-6-oxo-1,6-dihydropyridine-2,5-dicarboxylate **113.** Compound **112** (3 g, 13 mmol) and anhydrous potassium carbonate (4.5 g, 33 mmol) were mixed with benzyl chloride (2.5 g, 20 mmol) in DMF (250 mL). The mixture was heated for 20 h, filtered, and the filtrate evaporated to dryness under reduced pressure. The residue was partitioned in a mixture of 4 M aqueous potassium carbonate (50 mL) and dichloromethane (50 mL). The aqueous phase was extracted with dichloromethane (2 x 25 mL) and solvent was removed from the combined organic extracts under reduced pressure. The crude product was purified by silica gel chromatography using 0 - 3% methanol in dichloromethane as eluents. Fractions containing product were combined, solvent was removed under reduced pressure, and the residue dried in vacuo to provide compound **113** as a pale yellow oil (3.3 g, 80%). ¹H NMR (300 MHz, DMSO-d₆): δ = 3.58 (s, 3H, CH₃), 3.63 (s, 3H, CH₃), 5.12 (s, 2H, PhCH₂), 6.22 (d, 1H, J =7.5 Hz, ArH), 7.09 (s, br, 3H, PhH) 7.30 (m, br, 2H, PhH), 7.80 (d, 1H, J = 7.5 Hz, ArH). ¹³C NMR (75 MHz, DMSO-d₆): δ = 516, 52.7, 77.9, 104.5, 123.8, 127.7, 128.7, 129.2, 132.6, 141.6, 154.7, 158.9, 163.4.

Lithium 1-(benzyloxy)-6-(methoxycarbonyl)-2-oxo-1,2-dihydropyridine-3-carboxylate **114.** To a solution of dimethyl 1-(benzyloxy)-6-oxo-1,6-dihydropyridine-2,5-dicarboxylate **113** (3.2 g, 10 mmol) in methanol (100 mL), cooled with an ice bath, a solution of lithium hydroxide (0.24 g) in a mixture of methanol (20 mL) and water (1 mL) was added under nitrogen. The mixture was allowed to warm to ambient temperature while stirring overnight. The reaction mixture was concentrated to dryness and the residue was recrystallized using methanol to provide compound **114,** lithium salt as white crystals (2.5 g, 81%). ¹H NMR (300 MHz, DMSO-d₆): δ = 3.87 (s, 3H, CH₃), 5.39 (s, 2H, PhCH₂), 6.32 (d, 1H, J =7.5 Hz, ArH), 7.37 (m, br, 3H, PhH) 7.61 (m, br, 2H, PhH), 8.26 (d, 1H, J = 7.8 Hz, ArH). ¹³C NMR (75 MHz, DMSO-d₆): δ = 516, 77.7, 98.6, 115.7,128.3, 128.8 , 129.9, 134.5, 144.5, 155.1, 156.0, 161.1, 165.0.

N,N"-bis[1-benzyloxy-3-carbamido-2-oxo-1,2-dihydropyridine-4-methylcarboxy-N'-[2-[2-(2-methoxyethoxy)ethoxy]ethoxy]-bis(2-aminoethyl)amine **115.** Lithium 1-(benzyloxy)-6-(methoxycarbonyl)-2-oxo-1,2-dihydropyridine-3-carboxylate **114** (496 mg, 1.60 mmol) was suspended in anhydrous methanol (25 mL) and concentrated hydrochloric acid (134 µL, 1.61 mmol) was added to form a solution. Solvents were removed under reduced pressure. N-hydroxysuccinimide (222 mg, 1.93 mmol) was added and the solids were dried in vacuo overnight. Anhydrous dimethylformamide (4 mL) was added to form a solution, diisopropylcarbodiimide (348 µL, 2.25 mmol) was added, and the solution was stirred for 4.5 hr under nitrogen atmosphere. N'-[2-[2-(2-methoxyethoxy)ethoxy]ethoxy]-bis(2-aminoethyl)amine **4** (182 mg, 729 µmol) was dissolved in dimethylformamide (1 mL) and diisopropylethylamine (381 µL, 2.19 mmol) and added to the reaction mixture. After a further 15 hr, water (ca. 1 mL) was added, and solvents were removed under reduced pressure. The residue was dissolved in dichloromethane (15 mL), washed with water (5 mL), and the aqueous fraction was extracted with dichloromethane (2 x 10 mL). The combined dichloromethane extracts were concentrated and the crude product was purified by silica gel chromatography using 0.1% triethylamine, 2 - 5% methanol in dichloromethane as eluents. Fractions containing product were combined, solvent was removed under reduced pressure, and the residue dried in vacuo to provide compound **115** (190 mg, 31.7%). ¹H NMR (600 MHz, CDCl₃): δ = 8.13 (br t, 2H, NH), 8.06 (d, 2H, ArH), 7.54 (m, 4H, PhH), 7.34 (m, 6H, PhH), 6.22 (d, 2H, ArH), 5.32 (s, 4H, PhCH₂O), 3.90 (6H, s, CO₂CH₃), 3.32 - 3.16 (m, 12H, CH₂O, CH₂NC=O), 2.97 (s, 3H, OCH₃), 2.96 (m, 2H, CH₂O), 2.41 (m, 4H, CH₂N), 2.33 (m, 2H, CH₂N). ¹³C NMR (600 MHz, CDCl₃): δ = 164.7, 160.3, 155.6, 148.1, 143.6, 133.5, 130.6, 129.4, 128.4, 122.3, 102.4, 79.5, 71.4, 70.0, 69.6, 69.5, 69.2, 58.4, 54.2, 53.5, 52.5, 37.6. FTMS pESI: calculated for C₄₁H₅₀N₅O₁₃ [M+H]⁺, 820.3400, found, 820.3387.

N,N"-bis[1-benzyloxy-3-carbamido-2-oxo-1,2-dihydropyridine-4-carboxy-N'-[2-[2-(2-methoxyethoxy)ethoxy]ethoxy]-bis(2-aminoethyl)amine **116** disodium salt. Compound **115** is dissolved in methanol and treated with 1 M sodium hydroxide solution (2 molar equivalents) for one hour at ambient temperature. Solvents are removed under reduced pressure to provide compound **116** as the disodium salt.

N,N"-bis[1-benzyloxy-3-carbamido-2-oxo-1,2-dihydropyridine-4-carbonyl(2-mercaptothiazolide) -N'-[2-[2-(2-methoxyethoxy)ethoxy]ethoxy]-bis(2-aminoethyl)amine **117.** Disodium salt **116** (1 molar equivalent), TBTU (2.2 molar equivalents), and 2-mercaptothiazoline (2.2 molar equivalents) are suspended in anhydrous dichloromethane (20 mL). Diisopropylethylamine (3 molar equivalents) is added dropwise to form a solution. After one hour, solvent is removed under reduced pressure and the residue is purified by silica gel chromatography to provide compound **117.**

Benzyl and tert-butyloxycarbonyl-protected di-macrocycle **118.** A solution of compound **117** (ca. 1 g) in dichloromethane (49.5 mL) and triethylamine (0.5 mL) and a solution of 5-amino-6-[(2-aminoethyl)-[2-[bis(2-aminoethyl)amino]ethyl]amino]hexylcarbamic acid tert-butyl ester **10** (one molar equivalent) in dichloromethane, isopropyl alcohol (ca. 5%), and diisopropylethylamine (ca. 3%) (50 mL) are added dropwise to dichloromethane (2 L) over a period of four days using two syringe pumps at a rate of 0.5 mL/hr. After an additional two days of reaction, solvent is removed under reduced pressure, and the crude product is purified by silica gel chromatography. The silica gel column is prepared so as to have a short section (ca. 1.25") of aluminum oxide (basic, Brockmann I) on its bottom. Fractions containing product are combined, solvent is removed under reduced pressure, and the residue dried in vacuo to provide the protected di-macrocycle **118.**

Di-macrocycle **119.** Benzyl and tert-butyloxycarbonyl-protected di-macrocycle **118** (ca. 50 mg) is dissolved in 12N hydrochloric acid (1.0 mL) and glacial acetic acid (1.0 mL). The solution is stirred under inert atmosphere for ca. one day, whereupon HCl is removed with a stream of inert gas. Solvents are removed under reduced pressure and the residue is dried in vacuo. The residue is dissolved in methanol (600 + 300 µL) and transferred to two O-ring microcentrifuge tubes. Ether (ca. 1.5 mL) is added, and the tubes are placed at 4 °C for 1 hr. The tubes are centrifuged at 12,000 rpm for 3 minutes, decanted, the pellets are washed with ether (ca. 1.5 mL) and allowed to air dry. The pellets are dried in vacuo to provide di-macrocycle **119,** pentahydrochloride salt.

### EXAMPLE 23

### Synthesis of an octa-coordinating di-macrocyclic bifunctional chelator (Scheme 19).

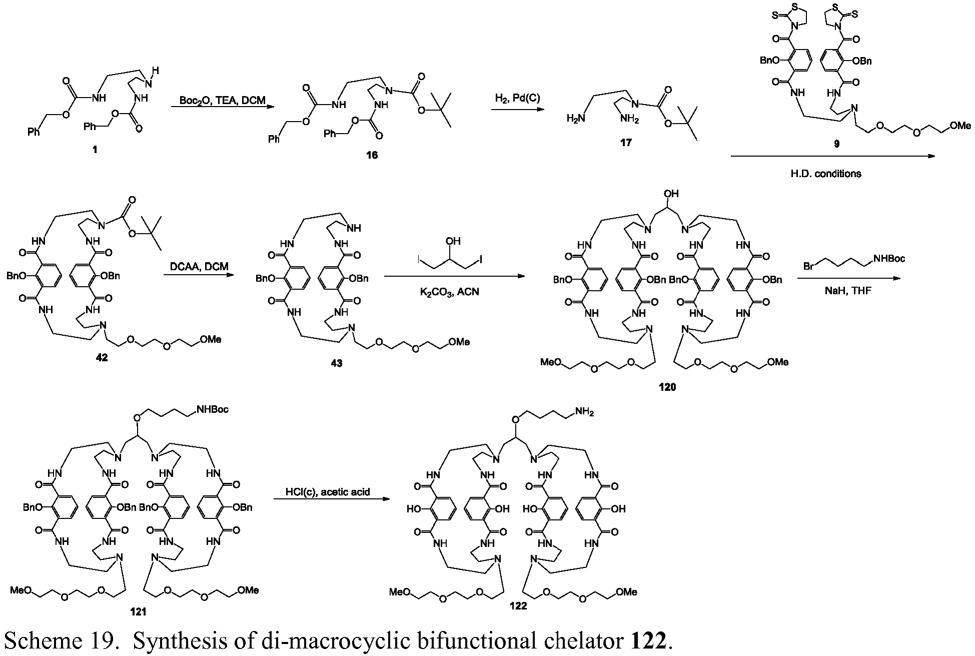

For ease of manufacture, it might be desirable to prepare di-macrocylic chelators using a convergent synthesis. One such approach, exemplified for a chelator containing four isophthalamide coordinating units, is shown is Scheme 19. Here, the mono-macrocyclic intermediate **42** is prepared by cyclization of dithiazolide **9** with diamine **17.** Deprotection of **42** under acidic conditions provides secondary amine **43,** which may be alkylated in one pot to provide the symmetrical di-macrocycle **120.** Di-macrocycle **120** is further alkylated to provide ether **121.** Deprotection of di-macrocycle **121** under acidic conditions forms the symmetric di-macrocyclic chelator **122.** It is to be noted that only one regioisomer is formed using this approach.

N,N"-Bis(carbobenzyloxy)-N'-tert-butyloxycarbonyl-bis(2-aminoethyl)amine **16**. *N,N"-*Di-Z-diethylenetriamine **1** (1.00 g, 2.69 mmol) was dried overnight in vacuo. Anhydrous dichloromethane (25 mL) was added, and the resulting solution was treated with triethylamine (0.751 mL, 5.39 mmol) and Boc anhydride (0.928 mL, 4.04 mmol) for 27 hr. The solution was transferred to a separatory funnel using dichloromethane (25 mL) and washed with 1 M sodium hydroxide (50 mL). The aqueous phase was extracted with dichloromethane (2 x 25 mL) and solvent was removed from the combined organic extracts under reduced pressure. The crude product was purified by silica gel chromatography using 1 - 2% methanol in dichloromethane as eluents. Fractions containing product were combined, solvent was removed under reduced pressure, and the residue dried in vacuo to provide compound **16** (1.283 g, 100%). ¹H NMR (300 MHz, CDCl₃): δ = 7.32 (s, 10H, PhH), 5.07 (s, 4H, PhCH₂O), 3.32 (m, 8H, CH₂N), 1.41 (s, 9H, Me). ¹³C NMR (600 MHz, CDCl₃): δ = 156.3, 136.9, 128.4, 128.0, 80.4, 66.6, 48.3, 47.4, 40.4, 40.0, 28.3.

N'-tert-butyloxycarbonyl-bis(2-aminoethyl)amine **17.** N,N"-Bis(carbobenzyloxy)-N'-tert-butyloxycarbonyl-bis(2-aminoethyl)amine **16** (1.150 g, 2.44 mmol) was dissolved in ethyl alcohol (50 mL). Palladium on carbon (10% wet, 115 mg) was added, and the atmosphere was exchanged for hydrogen. After 15 hr, methanol (50 mL) was added to the resulting suspension to form a solution. This was filtered through Celite® to remove catalyst, the Celite was washed with methanol (50 mL), solvent was removed under reduced pressure, and the residue dried in vacuo to provide compound **17** (483 mg, 97.4%). ¹H NMR (300 MHz, CDCl₃): δ = 3.23 (m, 4H, CH₂NC=O), 2.79 (t, 4H, CH₂N), 1.31 (s, 6H, CH₃), 1.20 (s, 3H, CH₃). ¹³C NMR (300 MHz, CDCl₃): δ = 156.4, 79.9, 51.0, 41.1, 28.7. FTMS pESI: calculated for C₉H₂₂N₃O₂ [MH]⁺, 204.1707, found, 204.1703.

Benzyl and tert-butyloxycarbonyl-protected mono-macrocycle **42.** A solution of N,N"-bis[1-benzyloxy-2-(2-mercaptothiazoleamido)-6-benzoyl]-N'-[2-[2-(2-methoxyethoxy)ethoxy]ethoxy]-bis(2-aminoethyl)amine **9** (1.214 g, 1.26 mmol) in dichloromethane (45 mL) and triethylamine (529 µL) and a solution of N'-tert-butyloxycarbonyl-bis(2-aminoethyl)amine **17** (257 mg, 1.26 mmol) in methanol (45 mL) and triethylamine (529 µL) were added dropwise to dichloromethane (1 L) over a period of four days using two syringe pumps at a rate of 0.5 mL/hr. After an additional two days of reaction, solvent was removed under reduced pressure, and the crude product was purified by silica gel chromatography using 0.1% triethylamine, 2 - 5% methanol in dichloromethane as eluents. Fractions containing product were combined, solvent was removed under reduced pressure, and the residue dried in vacuo to provide the protected mono-macrocycle **42** (776 mg, 66.3%). ¹H NMR (600 MHz, MeOD): δ = 7.66 - 7.17 (m, 16H, PhH, ArH), 4.62 (s, 4H, PhCH₂O), 3.54 - 3.31 (m, 14H, CH₂O, CH₂NC=O), 3.32 (s, 3H, OCH₃), 2.63 (m, 6H, CH₂N), 1.41 (s, 9H, CH₃). ¹³C NMR (600 MHz, MeOD): δ = 168.0, 153.9, 131.7, 128.6, 128.3, 128.2, 124.3, 80.1, 77.6, 71.5, 70.0, 69.8, 68.8, 57.7, 53.6, 52.6, 39.4, 37.9. FTMS pESI: calculated for C₅₀H₆₅N₆O₁₁ [M+H]⁺, 925.4706, found, 925.4684.

Mono-macrocycle **43.** Protected mono-macrocycle **42** (50 mg, 54 µmol) was dissolved in dichloromethane (500 µL) and dichloroacetic acid (500 µL) was added. After 3 hr, the reaction mixture was transferred to three microcentrifuge tubes and diethyl ether (ca. 1.5 mL/tube) was added. The tubes were stored at 4 °C for 30 minutes, centrifuged at 12,000 rpm for 3 minutes, and decanted. The pellets were washed with ether (ca. 1.5 mL) and allowed to air dry. The pellets were dried in vacuo to provide mono-macrocycle **43,** dichloroacetate salt (15.4 mg, 30%). FTMS pESI: calculated for C₄₅H₅₇N₆O₉ [M+H]⁺, 825.4182, found, 825.4186.

Di-macrocycle **120.** Mono-macrocycle **43,** dichloroacetate salt (75 mg, 79 µmol) and potassium carbonate (3 molar equivalents) are dried overnight in vacuo. Anhydrous acetonitrile (2 mL) and 1,3-diiodo-2-propanol (0.5 molar equivalents) are added and the resulting suspension is heated at reflux for 24 hr. Upon cooling, solvent is removed under reduced pressure, and the residue is dissolved in dichloromethane (ca. 10 mL) and washed with water (ca. 5 mL). Solvent is removed under reduced pressure, and the crude product is purified by silica gel chromatography using 0.1% triethylamine, methanol in dichloromethane as eluents. Fractions containing product are combined, solvent is removed under reduced pressure, and the residue dried in vacuo to provide di-macrocycle **120.**

Di-macrocycle **121.** Di-macrocycle **120** (50 mg, 29 µmol) is dissolved in anhydrous tetrahydrofuran (2 mL) and cooled in an ice bath. Sodium hydride (1 molar equivalent) is added, followed by 4-(Boc-amino) butyl bromide (1 molar equivalent). The reaction mixture is allowed to warm to ambient temperature, and after reaction is complete, solvent is removed under reduced pressure. The residue is dissolved in dichloromethane (ca. 10 mL) and washed with water (ca. 5 mL). Solvent is removed under reduced pressure, and the crude product is purified by silica gel chromatography using 0.1% triethylamine, methanol in dichloromethane as eluents. Fractions containing product are combined, solvent is removed under reduced pressure, and the residue dried in vacuo to provide di-macrocycle **121.**

Di-macrocycle **122.** Benzyl and tert-butyloxycarbonyl-protected di-macrocycle **121** (ca. 50 mg) is dissolved in 12N hydrochloric acid (1.0 mL) and glacial acetic acid (1.0 mL). The solution is stirred under inert atmosphere for ca. one day, whereupon HCl is removed with a stream of inert gas. Solvents are removed under reduced pressure and the residue is dried in vacuo. The residue is dissolved in methanol (600 + 300 µL) and transferred to two O-ring microcentrifuge tubes. Ether (ca. 1.5 mL) is added, and the tube is placed at 4 °C for 1 hr. The tubes are centrifuged at 12,000 rpm for 3 minutes, decanted, the pellets are washed with ether (ca. 1.5 mL) and allowed to air dry. The pellets are dried in vacuo to provide di-macrocycle **122,** pentahydrochloride salt.

### EXAMPLE 24

### Synthesis of an octa-coordinating di-macrocyclic bifunctional chelator (Scheme 20).

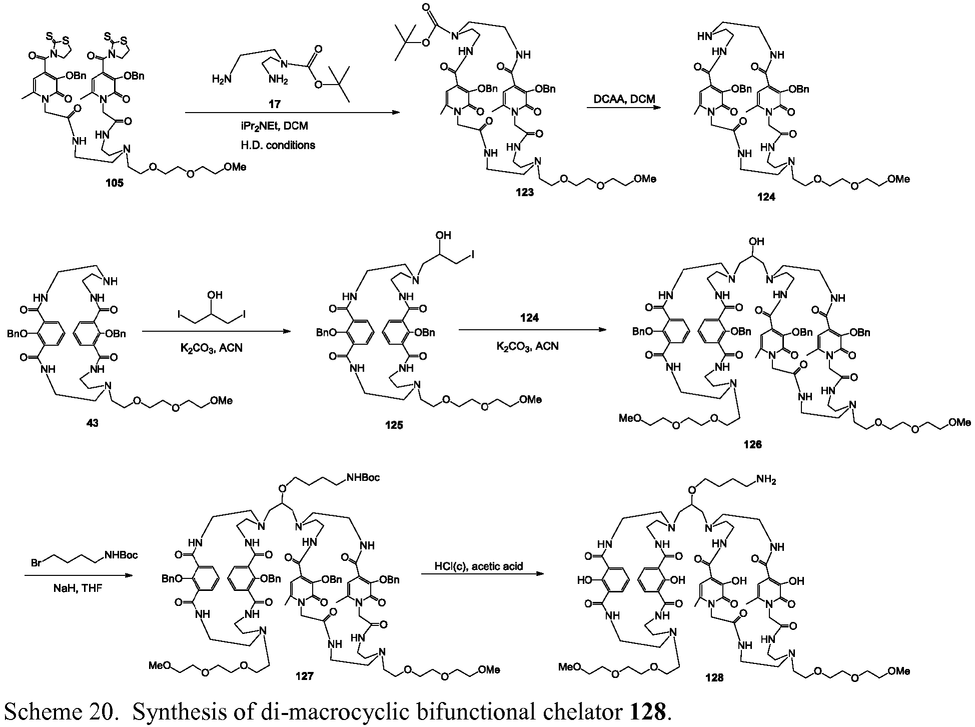

To one skilled in the art, it should be possible to use the methods disclosed above to prepare octa-coordinating di-macrocyclic bifunctional chelators that combine dissimilar coordinating groups. One example of such as synthesis is shown in Scheme 20, wherein the di-macrocyclic chelator **128,** which contains two isophthalamide and two 3-hydroxypyridin-2-one coordinating groups, is described. Dithiazolide **105** is condensed with N'-tert-butyloxycarbonyl-bis(2-aminoethyl)amine **17** to provide protected mono-macrocycle **123.** This is converted to the secondary amine **124** using acidic conditions. Mono-macrocycle **43** is reacted with an excess of 1,3-diiodo-2-propanol to provide the iodide **125.** Iodide **125** is used to alkylate the secondary amine **124** under basic conditions to form di-macrocycle **126.** Di-macrocycle **126** may be alkylated to form ether **127,** and protective groups are removed under acidic conditions to provide di-macrocycle **128** as the penta-hydrochloride salt. Similar syntheses to prepare di-macrocyclic chelators bearing alternative coordinating groups are envisioned. Such approaches may yield bifunctional chelators that combine advantageous properties of the individual coordinating groups, such as luminescence in the presence of certain lanthanide ions (to help assess, for example, biodistribution) with strong metal cation binding properties or more facile kinetics of metalation.

## Claims

1. A di-macrocycle having the structure: 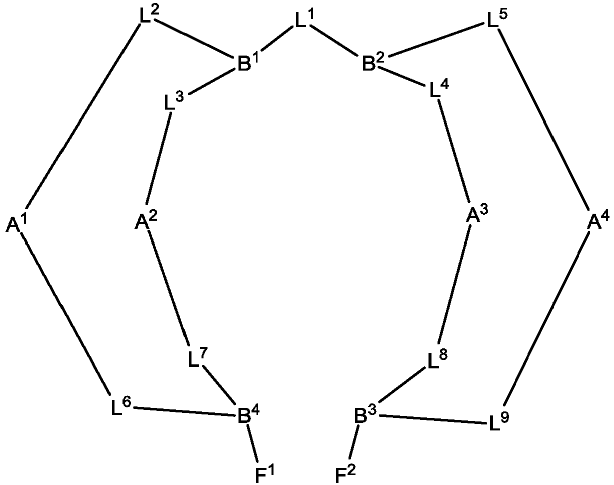 wherein
B¹, B², B³, and B⁴ are independently selected from N, C, B, Si, and P;
F¹ and F² are independently selected from H, C₁-C₈ alkyl, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and S and C₃-C₈ heterocycloalkyl, and wherein, when F1 and F2 are not H, F1 and F2 each comprise a modifying moiety selected from a solubilizing group, an estradiol-derived moiety, , an oligonucleotide, ssDNA, dsDNA, RNA, and a peptide, and optionally wherein F1 and F2 are independently selected from a polyether and a peptide;
L¹, L², L³, L⁴, L⁵, L⁶, L⁷, L⁸, and L⁹ are independently selected from C₁-C₈ alkyl optionally substituted with a linker, heteroalkyl of 2, 3, 4, 5 or 6 atoms selected from C, N, O, Si and S such that the heteroalkyl contains at least on C atom and at least one heteroatom, and C₃-C₈ heterocycloalkyl;
A¹, A², A³ and A⁴ are members independently selected from the general structures: 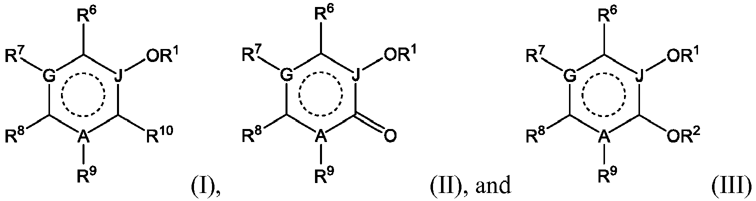 wherein
A and G are independently selected from carbon, nitrogen and oxygen;
J is selected from carbon and nitrogen;
each R¹ and R² are independently selected from H, and a single negative charge;
each R⁶, R⁷, R⁸, R⁹, and R¹⁰ are independently selected from
a bond to L², L³, L⁴, L⁵, L⁶, L⁷, L⁸, or L⁹, alkanediyl attached to L², L³, L⁴, L⁵, L⁶, L⁷, L⁸, or L⁹,
H, alkyl, heteroalkyl, halogen, CN,
CF₃, -C(O)R¹⁷, -SO₂NR¹⁷R¹⁸, -NR^{I7}R¹⁸, -OR¹⁷, -S(O)₂R¹⁷, -COOR¹⁷,-S(O)₂OR¹⁷, -OC(O)R¹⁷, -C(O)NR¹⁷R¹⁸, -NR¹⁷C(O)R¹⁸, -NR¹⁷SO₂R¹⁸, and -NO₂,
wherein
at least two of R⁶, R⁷, R⁸, R⁹, and R¹⁰ are optionally joined to form a ring system selected from cycloalkyl, heterocycloalkyl, aryl and heteroaryl;
R¹⁷ and R¹⁸ are independently selected from H, alkyl, heteroalkyl, aryl, heteroaryl and heterocycloalkyl; and
R¹⁷ and R¹⁸, together with the atoms to which they are attached, are optionally joined to form a 5-, 6- or 7-membered ring.
when A is oxygen, R⁹ is not present; and
when G is oxygen, R⁷ is not present;
A¹ is attached to L² and L⁶ through two members selected from R⁶, R⁷, R⁸, R⁹ and R¹⁰;
A² is attached to L³ and L⁷ through two members selected from R⁶, R⁷, R⁸, R⁹ and R¹⁰;
A³ is attached to L⁴ and L⁸ through two members selected from R⁶, R⁷, R⁸, R⁹ and R¹⁰; and
A⁴ is attached to L⁵ and L⁹ through two members selected from R⁶, R⁷, R⁸, R⁹ and R¹⁰.

2. The di-macrocycle according to claim 1, wherein
when A¹ has a structure according to formula (I), A¹ is attached to L² and L⁶ through R⁶ and R¹⁰;
when A¹ has a structure according to formula (II) or (III), A¹ is attached to L² and L⁶ through R⁶ and R⁹;
when A² has a structure according to formula (I), A² is attached to L³ and L⁷ through R⁶ and R¹⁰;
when A² has a structure according to formula (II) or (III), A² is attached to L³ and L⁷ through R⁶ and R⁹;
when A³ has a structure according to formula (I), A³ is attached to L⁴ and L⁸ through R⁶ and R¹⁰;
when A³ has a structure according to formula (II) or (III), A³ is attached to L⁴ and L⁸ through R⁶ and R⁹;
when A⁴ has a structure according to formula (I), A⁴ is attached to L⁵ and L⁹ through R⁶ and R¹⁰; and
when A⁴ has a structure according to formula (II) or (III), A⁴ is attached to L⁵ and L⁹ through R⁶ and R⁹.

3. The di-macrocycle according to claim 2, wherein
when A¹ has a structure according to formula (I), at least one of R⁶ and R¹⁰ of A¹ is a bond attached to L² or L⁶;
when A² has a structure according to formula (I), at least one of R⁶ and R¹⁰ of A² is a bond attached to L³ or L⁷;
when A³ has a structure according to formula (I), at least one of R⁶ and R¹⁰ of A³ is a bond attached to L⁴ or L⁸; and
when A⁴ has a structure according to formula (I), at least one of R⁶ and R¹⁰ of A⁴ is a bond attached to L⁵ or L⁹.

4. The di-macrocycle according to claim 1, wherein each of A¹, A², A³ and A⁴ is independently selected from:

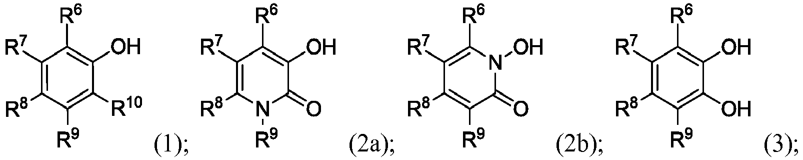

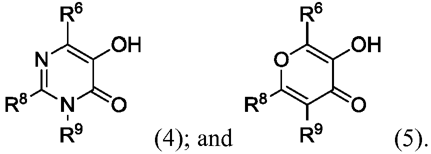

5. The di-macrocycle according to claim 4, wherein
when A¹ has a structure according to formula (1), A¹ is attached to L² and L⁶ through R⁶ and R¹⁰;
when A¹ has a structure according to formula (2a), (2b), (3), (4) or (5), A¹ is attached to L² and L⁶ through R⁶ and R⁹;
when A² has a structure according to formula (1), A² is attached to L³ and L⁷ through R⁶ and R¹⁰;
when A² has a structure according to formula (2a), (2b), (3), (4) or (5), A² is attached to L³ and L⁷ through R⁶ and R⁹;
when A³ has a structure according to formula (1), A³ is attached to L⁴ and L⁸ through R⁶ and R¹⁰;
when A³ has a structure according to formula (2a), (2b), (3), (4) or (5), A³ is attached to L⁴ and L⁸ through R⁶ and R⁹;
when A⁴ has a structure according to formula (1), A⁴ is attached to L⁵ and L⁹ through R⁶ and R¹⁰; and
when A⁴ has a structure according to formula (2a), (2b), (3), (4) or (5), A⁴ is attached to L⁵ and L⁹ through R⁶ and R⁹.

6. The di-macrocycle according to claim 5, wherein
when A¹ has a structure according to formula (1), at least one of R⁶ and R¹⁰ of A¹ is a bond attached to L² or L⁶;
when A² has a structure according to formula (1), at least one of R⁶ and R¹⁰ of A² is a bond attached to L³ or L⁷;
when A³ has a structure according to formula (1), at least one of R⁶ and R¹⁰ of A³ is a bond attached to L⁴ or L⁸; and
when A⁴ has a structure according to formula (1), at least one of R⁶ and R¹⁰ of A⁴ is a bond attached to L⁵ or L⁹.

7. The di-macrocycle according to any one of claims 1-6, having the structure: 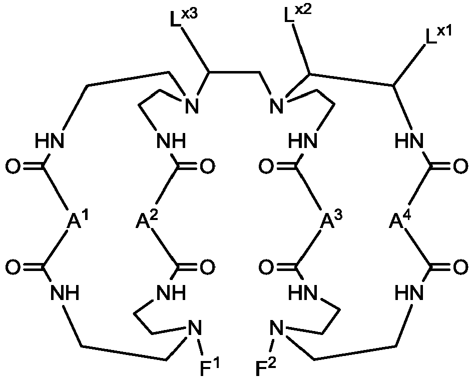 wherein L^{x1}, L^{x2} and L^{x3} are independently selected from H and a linker.

8. The di-macrocycle according to claim **7,** having the structure:

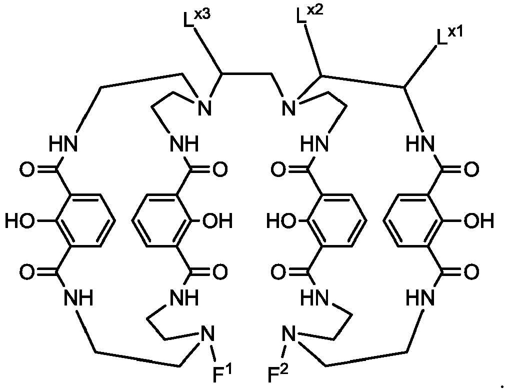

9. The di-macrocycle according to any one of claims **1-6,** having the structure: 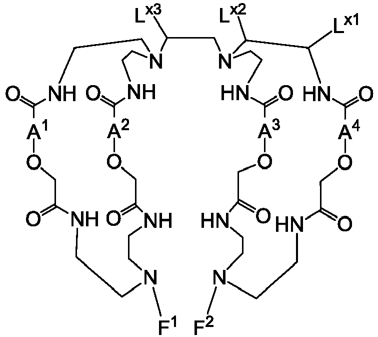 wherein L^{x1}, L^{x2} and L^{x3} are independently selected from H and a linker.

10. The di-macrocycle according to claim **9,** having the structure:

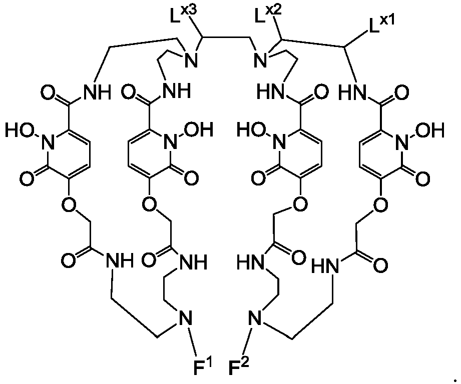

11. A di-macrocycle having the structure: 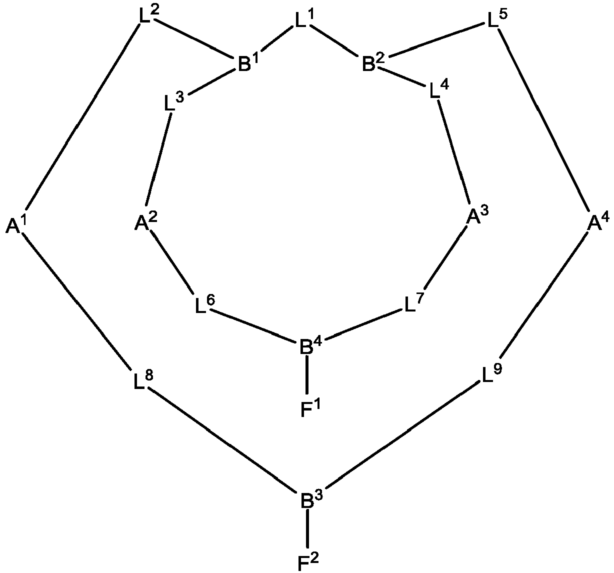 wherein
B¹, B², B³, and B⁴ are independently selected from N, C, B, Si, and P;
F¹ and F² are independently selected from H, C₁-C₈ alkyl, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and S and C₃-C₈ heterocycloalkyl, and wherein, when F1 and F2 are not H, F1 and F2 each comprise a modifying moiety selected from a solubilizing group, an estradiol-derived moiety, an oligonucleotide, ssDNA, dsDNA, RNA, and a peptide, and optionally wherein F1 and F2 are independently selected from a polyether and a peptide;
L¹, L², L³, L⁴, L⁵, L⁶, L⁷, L⁸, and L⁹ are independently selected from C₁-C₈ alkyl optionally substituted with a linker, heteroalkyl of 2, 3, 4, 5 or 6 atoms selected from C, N, O, Si and S such that the heteroalkyl contains at least one C atom and at least one heteroatom, C₅-C₈ aryl, C₃-C₈ heteroaryl wherein the aryl groups contain 1, 2, 3 or 4 heteroatoms selected N, O and ⁵ and C₃-C₈ heterocycloalkyl;
A¹, A², A³ and A⁴ are members independently selected from the general structures: 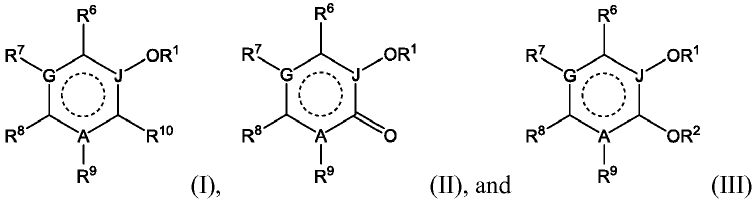 wherein
A and G are independently selected from carbon, nitrogen and oxygen;
J is selected from carbon and nitrogen;
each R¹ and R² are independently selected from H, and a single negative charge;
each R⁶, R⁷, R⁸, R⁹, and R¹⁰ are independently selected from
a bond to L², L³, L⁴, L⁵, L⁶, L⁷, L⁸, or L⁹, alkanediyl attached to L², L³, L⁴, L⁵, L⁶, L⁷, L⁸, or L⁹,
H, alkyl, heteroalkyl, halogen, CN,
CF₃, -C(O)R¹⁷, -SO₂NR¹⁷R¹⁸, -NR¹⁷R¹⁸, -OR¹⁷, -S(O)₂R¹⁷, -COOR¹⁷,-S(O)₂OR¹⁷, -OC(O)R¹⁷, -C(O)NR¹⁷R¹⁸, -NR¹⁷C(O)R¹⁸, -NR¹⁷SO₂R¹⁸, and -NO₂,
wherein
at least two of R⁶, R⁷, R⁸, R⁹, and R¹⁰ are optionally joined to form a ring system selected from cycloalkyl, heterocycloalkyl, aryl and heteroaryl;
R¹⁷ and R¹⁸ are independently selected from H, alkyl, heteroalkyl, aryl, heteroaryl and heterocycloalkyl; and
R¹⁷ and R¹⁸, together with the atoms to which they are attached, are optionally joined to form a 5-, 6- or 7-membered ring.
when A is oxygen, R⁹ is not present; and
when G is oxygen, R⁷ is not present;
A¹ is attached to L² and L⁸ through two members selected from R⁶, R⁷, R⁸, R⁹ and R¹⁰;
A² is attached to L³ and L⁶ through two members selected from R⁶, R⁷, R⁸, R⁹ and R¹⁰;
A³ is attached to L⁴ and L⁷ through two members selected from R⁶, R⁷, R⁸, R⁹ and R¹⁰; and
A⁴ is attached to L⁵ and L⁹ through two members selected from R⁶, R⁷, R⁸, R⁹ and R¹⁰.

12. The di-macrocycle according to claim **1** or claim **11,** wherein said di-macrocycle is covalently modified with at least one linker.

13. The di-macrocycle according to claim **12,** wherein one of L¹, L², L³, L⁴, L⁵, L⁶, L¹, L⁸, and L⁹ is substituted with a linker.

14. The di-macrocycle according to any one of claims **11-13,** wherein
when A¹ has a structure according to formula (I), A¹ is attached to L² and L⁸ through R⁶ and R¹⁰;
when A¹ has a structure according to formula (II) or (III), A¹ is attached to L² and L⁸ through R⁶ and R⁹;
when A² has a structure according to formula (I), A² is attached to L³ and L⁶ through R⁶ and R¹⁰;
when A² has a structure according to formula (II) or (III), A² is attached to L³ and L⁶ through R⁶ and R⁹;
when A³ has a structure according to formula (I), A³ is attached to L⁴ and L⁷ through R⁶ and R¹⁰;
when A³ has a structure according to formula (II) or (III), A³ is attached to L⁴ and L⁷ through R⁶ and R⁹;
when A⁴ has a structure according to formula (I), A⁴ is attached to L⁵ and L⁹ through R⁶ and R¹⁰; and
when A⁴ has a structure according to formula (II) or (III), A⁴ is attached to L⁵ and L⁹ through R⁶ and R⁹.

15. The di-macrocycle according to any one of claims **11-14,** wherein each of A¹, A², A³ and A⁴ is independently selected from:

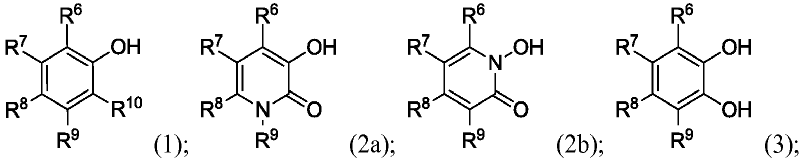

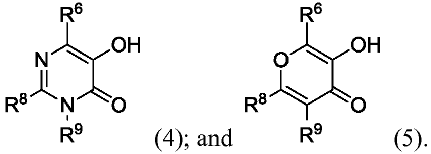

16. The di-macrocycle according to claim **15,** wherein
when A¹ has a structure according to formula (1), A¹ is attached to L² and L⁸ through R⁶ and R¹⁰;
when A¹ has a structure according to formula (2a), (2b), (3), (4) or (5), A¹ is attached to L² and L⁸ through R⁶ and R⁹;
when A² has a structure according to formula (1), A² is attached to L³ and L⁶ through R⁶ and R¹⁰;
when A² has a structure according to formula (2a), (2b), (3), (4) or (5), A² is attached to L³ and L⁶ through R⁶ and R⁹;
when A³ has a structure according to formula (1), A³ is attached to L⁴ and L⁷ through R⁶ and R¹⁰;
when A³ has a structure according to formula (2a), (2b), (3), (4) or (5), A³ is attached to L⁴ and L⁷ through R⁶ and R⁹;
when A⁴ has a structure according to formula (1), A⁴ is attached to L⁵ and L⁹ through R⁶ and R¹⁰; and
when A⁴ has a structure according to formula (2a), (2b), (3), (4) or (5), A⁴ is attached to L⁵ and L⁹ through R⁶ and R⁹.

17. The di-macrocycle according to any one of claims **11-16,** having the structure: 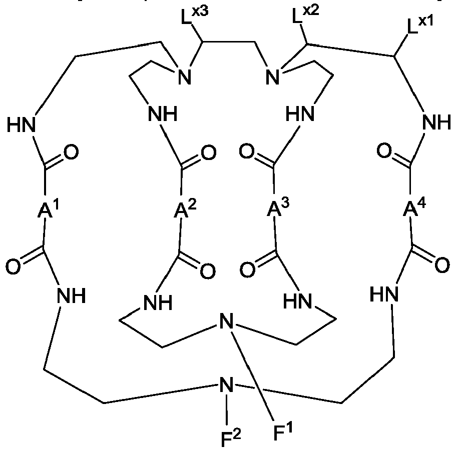 wherein L^{x1}, L^{x2} and L^{x3} are independently selected from H and a linker.

18. The di-macrocycle according to claim **17,** having the structure:

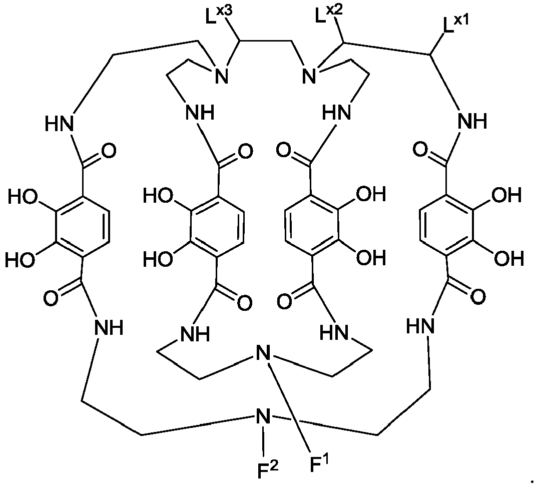

19. The di-macrocycle according to claim **17,** having the structure:

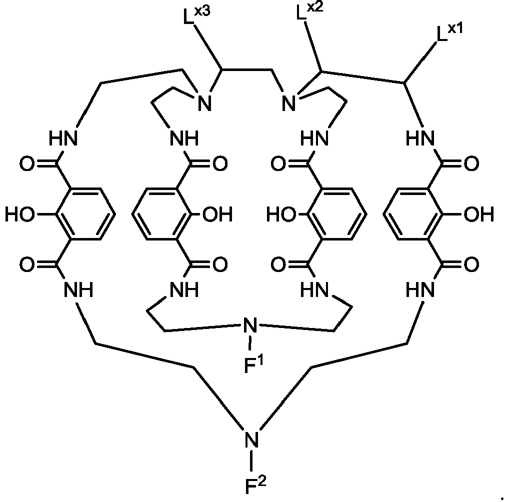

20. A complex comprising a di-macrocycle according to any one of the preceding claims and a metal ion,
optionally wherein the metal ion is a lanthanide, optionally the lanthanide is selected from Eu and Tb,
optionally wherein the metal ion is an actinide,
optionally wherein the metal ion is selected from yttrium(III), europium(III), terbium(III), zirconium(IV) and thorium(IV),
optionally wherein the metal ion is a radionuclide selected from isotopes of U, Pu, Fe, Cu, Sm, Gd, Tb, Dy, Ho, Er, Yb, Lu, Y, Th, Zr, In, Ga, Bi, Ra, At and Ac,
optionally wherein said metal ion is ²²⁷Th(IV) or ⁸⁹Zr(IV),
optionally wherein said metal is a member selected from ¹⁷⁷Lu, ¹⁶⁶Ho, ¹⁵³Sm, ⁹⁰Y, ⁸⁶Y, ¹⁶⁶Dy, ¹⁶⁵Dy, ¹⁶⁹Er, ¹⁷⁵Yb, ²²⁵Ac, ¹⁴⁹Tb, ¹⁵³Gd, and ²³⁰U or
optionally wherein said metal is a member selected from ¹¹¹In, ⁶⁷Ga, ⁶⁷Cu, ⁶⁴Cu, ¹⁸⁶Re, ¹⁸⁸Re, ¹¹¹Ag, ¹⁰⁹Pd, ²¹²Pb, ²⁰³Pb, ²¹²Bi, ²¹³Bi, ^{195m}Pt, ²⁰¹T1, ⁵⁵Co, and ^{99m}Tc.

21. The di-macrocycle according to any one of claims 1 to 19 or the complex comprising a di-macrocycle according to claim 20 wherein the estradiol-derived group is selected from estradiol and the estradiol-derived group having the structure.

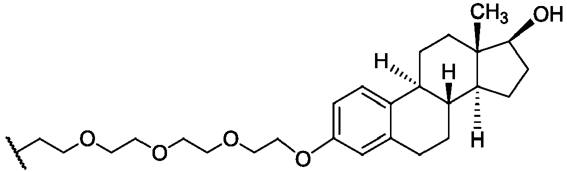

## Patentansprüche

1. Di-Macrocyclus, aufweisend die folgende Struktur: 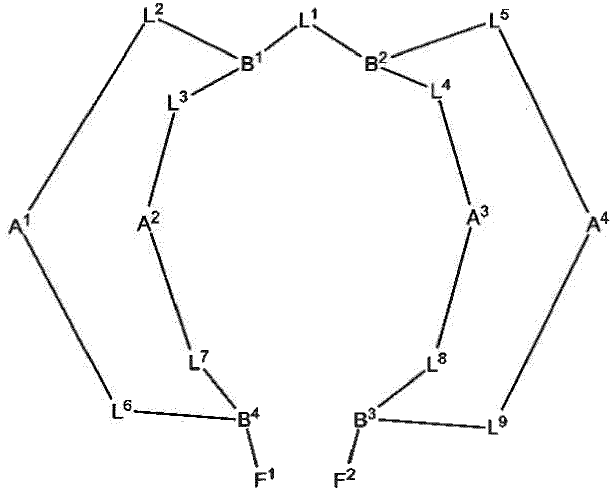
wobei B¹, B², B³ und B⁴ unabhängig voneinander ausgewählt sind aus N, C, B, Si und P;
F¹ und F² unabhängig voneinander ausgewählt sind aus H, C₁-C₈-Alkyl, Heteroalkyl von 2, 3, 4, 5 oder 6 Atomen ausgewählt aus C, N, O, Si und S, sodass das Heteroalkyl zumindest ein C-Atom und zumindest ein Heteroatom, C₅-C₈-Aryl, C₃-C₈-Heteroaryl enthält, wobei die Arylgruppen 1, 2, 3 oder 4 Heteroatome ausgewählt aus N, O und S und C₃-C₈-Heterocycloalkyl enthalten, und wobei, wenn F1 und F2 nicht H sind, F1 und F2 jeweils eine modifizierende Komponente umfassen, die aus einer solubilisierenden Gruppe, einer von Estradiol abgeleiteten Komponente, einem Oligonukleotid, ssDNA, dsDNA, RNA und einem Peptid ausgewählt ist, und wobei gegebenenfalls F1 und F2 unabhängig voneinander aus einem Polyether und einem Peptid ausgewählt sind;
L¹, L², L³, L⁴, L⁵, L⁶, L⁷, L⁸ und L⁹ unabhängig voneinander ausgewählt aus C₁-C₈-Alkyl gegebenenfalls substituiert mit einem Linker, Heteroalkyl von 2, 3, 4, 5 oder 6 Atomen, ausgewählt aus C, N, O, Si und S, sodass das Heteroalkyl zumindest ein C-Atom und zumindest ein Heteroatom enthält, und C₃-C₈-Heterocycloalkyl;
wobei A¹, A², A³ und A⁴ Glieder sind, die unabhängig voneinander aus den allgemeinen Strukturen ausgewählt sind: 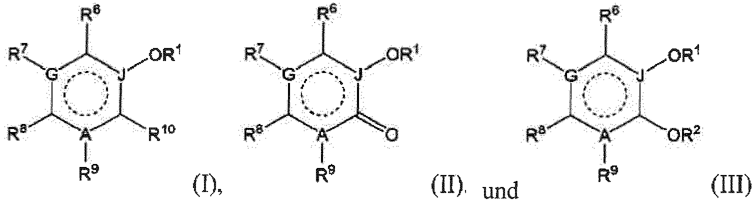
Wobei A und G unabhängig voneinander aus Kohlenstoff, Stickstoff und Sauerstoff ausgewählt sind;
J aus Kohlenstoff und Stickstoff ausgewählt ist;
jedes R¹ und R² unabhängig aus H ausgewählt sind, und einer einfach negativen Ladung;
jedes R⁶, R⁷, R⁸, R⁹ und R¹⁰ unabhängig voneinander ausgewählt sind aus einer Bindung an L², L³, L⁴, L⁵, L⁶, L⁷, L⁸ oder L⁹, Alkanediyl gebunden an L², L³, L⁴, L⁵, L⁶, L⁷, L⁸ oder L⁹, H, Alkyl, Heteroalkyl, Halogen, CN, CF₃, -C(O)R¹⁷, -SO₂NR¹⁷R¹⁸, -NR¹⁷R¹⁸, -OR¹⁷, -S(O)₂R¹⁷, -COOR¹⁷, - S(O)₂OR¹⁷, -OC(O)R¹⁷, -C(O)NR¹⁷R¹⁸, -NR¹⁷C(O)R¹⁸, -NR¹⁷SO₂R¹⁸ und -NO₂, wobei zumindest zwei von R⁶, R⁷, R⁸, R⁹ und R¹⁰ gegebenenfalls verbunden sind, um ein Ringsystem ausgewählt aus Cycloalkyl, Heterocycloalkyl, Aryl und Heteroaryl zu bilden;
R¹⁷ und R¹⁸ unabhängig voneinander ausgewählt sind aus H, Alkyl, Heteroalkyl, Aryl, Heteroaryl und Heterocycloalkyl; und R¹⁷ und R¹⁸, zusammen mit den Atomen, an die sie gebunden sind, gegebenenfalls verbunden sind, um einen 5-, 6- oder 7-gliedrigen Ring zu bilden, wenn A Sauerstoff ist, R⁹ nicht vorhanden ist; und wenn G Sauerstoff ist, R⁷ nicht vorhanden ist;
A¹ ist an L² und L⁶ durch zwei Glieder ausgewählt aus R⁶, R⁷, R⁸, R⁹ und R¹⁰ gebunden;
A² ist an L³ und L⁷ durch zwei Glieder ausgewählt aus R⁶, R⁷, R⁸, R⁹ und R¹⁰ gebunden;
A³ ist an L⁴ und L⁸ durch zwei Glieder ausgewählt aus R⁶, R⁷, R⁸, R⁹ und R¹⁰ gebunden;
A⁴ ist an L⁵ und L⁹ durch zwei Glieder ausgewählt aus R⁶, R⁷, R⁸, R⁹ und R¹⁰ gebunden.

2. Di-Macrocyclus nach Anspruch 1, wobei wenn A¹ eine Struktur nach Formel (I) aufweist, A¹ an L² und L⁶ durch R⁶ und R¹⁰ gebunden ist;
wenn A¹ eine Struktur nach Formel (II) oder (III) aufweist, ist A¹ an L² und L⁶ durch R⁶ und R⁹ gebunden;
wenn A² eine Struktur nach Formel (I) aufweist, ist A² an L³ und L⁷ durch R⁶ und R¹⁰ gebunden;
wenn A² eine Struktur nach Formel (II) oder (III) aufweist, ist A² an L³ und L⁷ durch R⁶ und R⁹ gebunden;
wenn A³ eine Struktur nach Formel (I) aufweist, ist A³ an L⁴ und L⁸ durch R⁶ und R¹⁰ gebunden;
wenn A³ eine Struktur nach Formel (II) oder (III) aufweist, ist A³ an L⁴ und L⁸ durch R⁶ und R⁹ gebunden;
wenn A⁴ eine Struktur nach Formel (I) aufweist, ist A⁴ an L⁵ und L⁹ durch R⁶ und R¹⁰ gebunden; und
wenn A⁴ eine Struktur nach Formel (II) oder (III) aufweist, ist A⁴ an L⁵ und L⁹ durch R⁶ und R⁹ gebunden.

3. Di-Macrocyclus nach Anspruch 2, wobei wenn A¹ eine Struktur nach Formel (I) aufweist, zumindest eines von R⁶ und R¹⁰ von A¹ eine Bindung ist, die an L² oder L⁶ gebunden ist.
wenn A² eine Struktur nach Formel (I) aufweist, ist zumindest eines von R⁶ und R¹⁰ von A² eine Bindung, die an L³ oder L⁷ gebunden ist;
wenn A³ eine Struktur nach Formel (I) aufweist, ist zumindest eines von R⁶ und R¹⁰ von A³ eine Bindung, die an L⁴ oder L⁸ gebunden ist; und
wenn A⁴ eine Struktur nach Formel (I) aufweist, ist zumindest eines von R⁶ und R¹⁰ von A⁴ eine Bindung, die an L⁵ oder L⁹ gebunden ist.

4. Di-Macrocyclus nach Anspruch **1,** wobei jedes von A¹, A², A³ und A⁴ unabhängig voneinander ausgewählt ist aus:

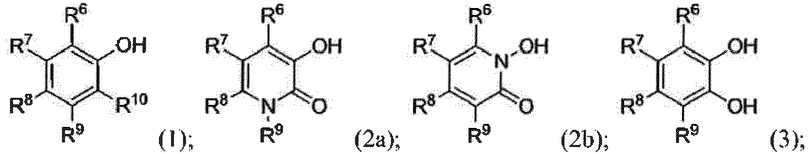

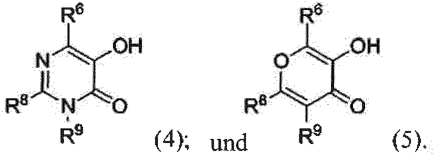

5. Di-Macrocyclus nach Anspruch 4, wobei wenn A¹ eine Struktur nach Formel (1) aufweist, A¹ an L² und L⁶ durch R⁶ und R¹⁰ gebunden ist;
wenn A¹ eine Struktur nach Formel (2a), (2b), (3), (4) oder (5) aufweist, ist A¹ an L² und L⁶ durch R⁶ und R⁹ gebunden;
wenn A² eine Struktur nach Formel (1) aufweist, ist A² an L³ und L⁷ durch R⁶ und R¹⁰ gebunden;
wenn A² eine Struktur nach Formel (2a), (2b), (3), (4) oder (5) aufweist, ist A² an L³ und L⁷ durch R⁶ und R⁹ gebunden;
wenn A³ eine Struktur nach Formel (1) aufweist, ist A³ an L⁴ und L⁸ durch R⁶ und R¹⁰ gebunden;
wenn A³ eine Struktur nach Formel (2a), (2b), (3), (4) oder (5) aufweist, ist A³ an L⁴ und L⁸ durch R⁶ und R⁹ gebunden;
wenn A⁴ eine Struktur nach Formel (1) aufweist, ist A⁴ an L⁵ und L⁹ durch R⁶ und R¹⁰ gebunden;
und wenn A⁴ eine Struktur nach Formel (2a), (2b), (3), (4) oder (5) aufweist, ist A⁴ an L⁵ und L⁹ durch R⁶ und R⁹ gebunden.

6. Di-Macrocyclus nach Anspruch 5, wobei wenn A¹ eine Struktur der Formel (1) aufweist, zumindest eins von R⁶ und R¹⁰ von A¹ eine Bindung ist, die an L² oder L⁶ gebunden ist;
wenn A² eine Struktur nach Formel (1) aufweist, ist zumindest eines von R⁶ und R¹⁰ von A² eine Bindung, die an L³ oder L⁷ gebunden ist;
wenn A³ eine Struktur nach Formel (1) aufweist, ist zumindest eines von R⁶ und R¹⁰ von A³ eine Bindung, die an L⁴ oder L⁸ gebunden ist; und
wenn A⁴ eine Struktur nach Formel (1) aufweist, ist zumindest eines von R⁶ und R¹⁰ von A⁴ eine Bindung, die an L⁵ oder L⁹ gebunden ist.

7. Di-Macrocyclus nach einem der Ansprüche **1-6,** aufweisend die folgende Struktur: 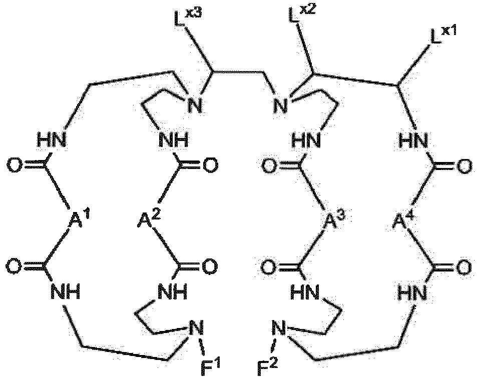 wobei L^{x1}, L^{x2} und L^{x3} unabhängig voneinander ausgewählt sind aus H und einem Linker.

8. Di-Macrocyclus nach Anspruch 7, aufweisend die folgende Struktur:

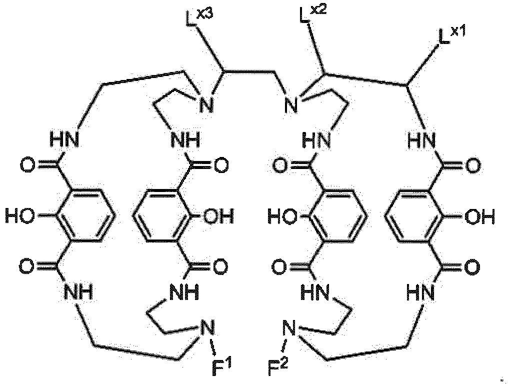

9. Di-Macrocyclus nach einem der Ansprüche **1-6,** aufweisend die folgende Struktur: 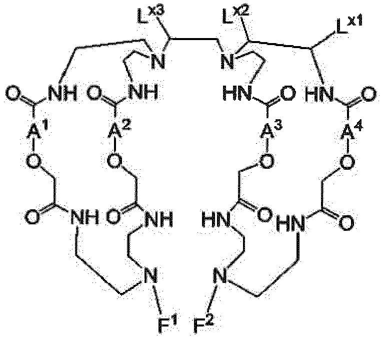 wobei L^{x1}, L^{x2} und L^{x3} unabhängig voneinander ausgewählt aus H und einem Linker.

10. Di-Macrocyclus nach Anspruch 9, aufweisend die folgende Struktur:

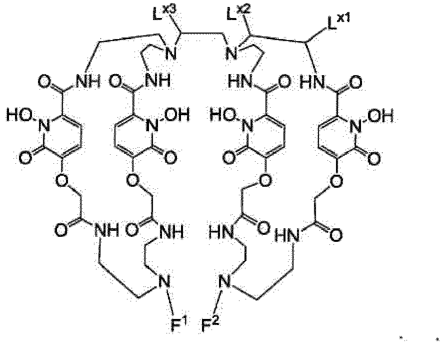

11. Di-Macrocyclus, aufweisend die folgende Struktur: 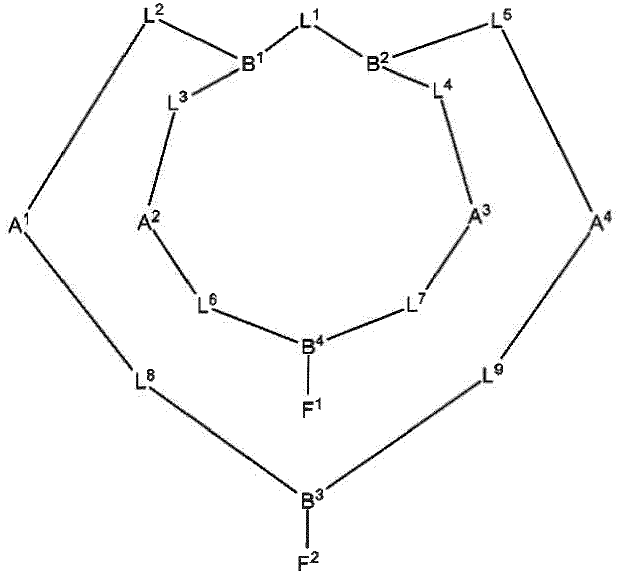
Wobei B¹, B², B³ und B⁴ unabhängig voneinander ausgewählt sind aus N, C, B, Si und P;
F¹ und F² unabhängig voneinander ausgewählt sind aus H, C₁-C₈-Alkyl, Heteroalkyl von 2, 3, 4, 5 oder 6 Atomen ausgewählt aus C, N, O, Si und S, sodass das Heteroalkyl zumindest ein C-Atom und zumindest ein Heteroatom, C₅-C₈-Aryl, C₃-C₈-Heteroaryl enthält, wobei die Arylgruppen 1, 2, 3 oder 4 Heteroatome ausgewählt aus N, O und S und C₃-C₈-Heterocycloalkyl enthalten, und wobei, wenn F1 und F2 nicht H sind, F1 und F2 jeweils eine modifizierende Komponente umfassen, die aus einer solubilisierenden Gruppe, einer von Estradiol abgeleiteten Komponente, einem Oligonukleotid, ssDNA, dsDNA, RNA und einem Peptid ausgewählt ist, und wobei gegebenenfalls F1 und F2 unabhängig voneinander aus einem Polyether und einem Peptid ausgewählt sind;
L¹, L², L³, L⁴, L⁵, L⁶, L⁷, L⁸ und L⁹ unabhängig voneinander ausgewählt aus C₁-C₈-Alkyl gegebenenfalls substituiert mit einem Linker, Heteroalkyl von 2, 3, 4, 5 oder 6 Atomen, ausgewählt aus C, N, O, Si und S, sodass das Heteroalkyl zumindest ein C-Atom und zumindest ein Heteroatom C₅-C₈-Aryl, C₃-C₈-Heteroaryl enthält, wobei die Arylgruppen 1, 2, 3 oder 4 Heteroatome ausgewählt aus N, O und S und C₃-C₈-Heterocycloalkyl enthalten;
Wobei A¹, A², A³ und A⁴ Glieder sind, die unabhängig voneinander aus den allgemeinen Strukturen ausgewählt sind: 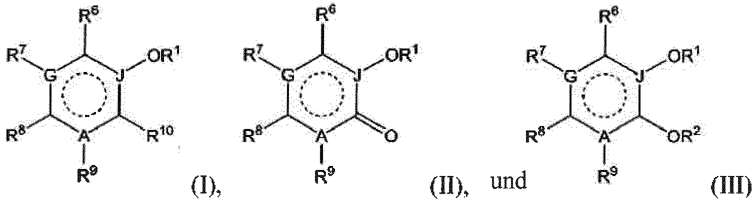
Wobei A und G unabhängig voneinander aus Kohlenstoff, Stickstoff und Sauerstoff ausgewählt sind;
J aus Kohlenstoff und Stickstoff ausgewählt ist;
jedes R¹ und R² unabhängig aus H ausgewählt sind, und einer einfach negativen Ladung;
jedes R⁶, R⁷, R⁸, R⁹ und R¹⁰ unabhängig voneinander ausgewählt ist aus einer Bindung an L², L³, L⁴, L⁵, L⁶, L⁷, L⁸ oder L⁹, Alkanediyl gebunden an L², L³, L⁴, L⁵, L⁶, L⁷, L⁸ oder L⁹, H, Alkyl, Heteroalkyl, Halogen, CN, CF₃, -C(O)R¹⁷, -SO₂NR¹⁷R¹⁸, -NR¹⁷R¹⁸, -OR¹⁷, -S(O)₂R¹⁷,-COOR¹⁷, - S(O)₂OR¹⁷, -OC(O)R¹⁷, -C(O)NR¹⁷R¹⁸, -NR¹⁷C(O)R¹⁸, -NR¹⁷SO₂R¹⁸ und -NO₂, wobei zumindest zwei von R⁶, R⁷, R⁸, R⁹ und R¹⁰ gegebenenfalls verbunden sind, um ein Ringsystem ausgewählt aus Cycloalkyl, Heterocycloalkyl, Aryl und Heteroaryl zu bilden;
R¹⁷ und R¹⁸ unabhängig voneinander ausgewählt sind aus H, Alkyl, Heteroalkyl, Aryl, Heteroaryl und Heterocycloalkyl; und R¹⁷ und R¹⁸, zusammen mit den Atomen, an die sie gebunden sind, gegebenenfalls verbunden sind, um einen 5-, 6- oder 7-gliedrigen Ring zu bilden, wenn A Sauerstoff ist, R⁹ nicht vorhanden ist; und wenn G Sauerstoff ist, R⁷ nicht vorhanden ist;
A¹ ist an L² und L⁸ durch zwei Glieder ausgewählt aus R⁶, R⁷, R⁸, R⁹ und R¹⁰ gebunden;
A² ist an L³ und L⁶ durch zwei Glieder ausgewählt aus R⁶, R⁷, R⁸, R⁹ und R¹⁰ gebunden;
A³ ist an L⁴ und L⁷ durch zwei Glieder ausgewählt aus R⁶, R⁷, R⁸, R⁹ und R¹⁰ gebunden;
A⁴ ist an L⁵ und L⁹ durch zwei Glieder ausgewählt aus R⁶, R⁷, R⁸, R⁹ und R¹⁰ gebunden.

12. Di-Macrocyclus nach Anspruch **1** oder Anspruch **11,** wobei der Di-Macrocyclus mit zumindest einem Linker kovalent modifiziert ist.

13. Di-Macrocyclus nach Anspruch **12,** wobei jedes von L¹, L², L³, L⁴, L⁵, L⁶, L⁷, L⁸ und L⁹ mit einem Linker substituiert ist.

14. Di-Macrocyclus nach einem der Ansprüche **11-13,** wobei wenn A¹ eine Struktur nach Formel (I) aufweist, A¹ an L² und L⁸ durch R⁶ und R¹⁰ gebunden ist;
wenn A¹ eine Struktur nach Formel (II) oder (III) aufweist, ist A¹ an L² und L⁸ durch R⁶ und R⁹ gebunden;
wenn A² eine Struktur nach Formel (I) aufweist, ist A² an L³ und L⁶ durch R⁶ und R¹⁰ gebunden;
wenn A² eine Struktur nach Formel (II) oder (III) aufweist, ist A² an L³ und L⁶ durch R⁶ und R⁹ gebunden;
wenn A³ eine Struktur nach Formel (I) aufweist, ist A³ an L⁴ und L⁷ durch R⁶ und R¹⁰ gebunden;
wenn A³ eine Struktur nach Formel (II) oder (III) aufweist, ist A³ an L⁴ und L⁷ durch R⁶ und R⁹ gebunden;
wenn A⁴ eine Struktur nach Formel (I) aufweist, ist A⁴ an L⁵ und L⁹ durch R⁶ und R¹⁰ gebunden; und
wenn A⁴ eine Struktur nach Formel (II) oder (III) aufweist, ist A⁴ an L⁵ und L⁹ durch R⁶ und R⁹ gebunden.

15. Di-Macrocyclus nach einem der Ansprüche **11-14,** wobei jedes von A¹, A², A³ und A⁴ unabhängig voneinander ausgewählt ist aus:

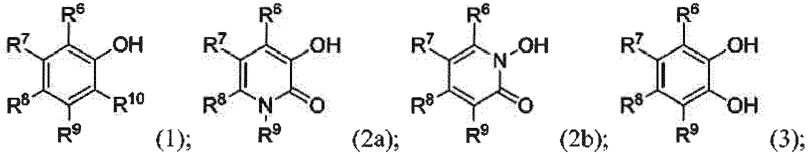

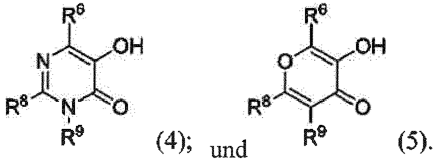

16. Di-Macrocyclus nach Anspruch **15,** wobei wenn A¹ eine Struktur nach Formel (1) aufweist, A¹ an L² und L⁸ durch R⁶ und R¹⁰ gebunden ist;
wenn A¹ eine Struktur nach Formel (2a), (2b), (3), (4) oder (5) aufweist, ist A¹ an L² und L⁸ durch R⁶ und R⁹ gebunden;
wenn A² eine Struktur nach Formel (1) aufweist, ist A² an L³ und L⁶ durch R⁶ und R¹⁰ gebunden;
wenn A² eine Struktur nach Formel (2a), (2b), (3), (4) oder (5) aufweist, ist A² an L³ und L⁶ durch R⁶ und R⁹ gebunden;
wenn A³ eine Struktur nach Formel (1) aufweist, ist A³ an L⁴ und L⁷ durch R⁶ und R¹⁰ gebunden;
wenn A³ eine Struktur nach Formel (2a), (2b), (3), (4) oder (5) aufweist, ist A³ an L⁴ und L⁷ durch R⁶ und R⁹ gebunden;
wenn A⁴ eine Struktur nach Formel (1) aufweist, ist A⁴ an L⁵ und L⁹ durch R⁶ und R¹⁰ gebunden; und
wenn A⁴ eine Struktur nach Formel (2a), (2b), (3), (4) oder (5) aufweist, ist A⁴ an L⁵ und L⁹ durch R⁶ und R⁹ gebunden.

17. Di-Macrocyclus nach einem der Ansprüche **11-16,** aufweisend die folgende Struktur: 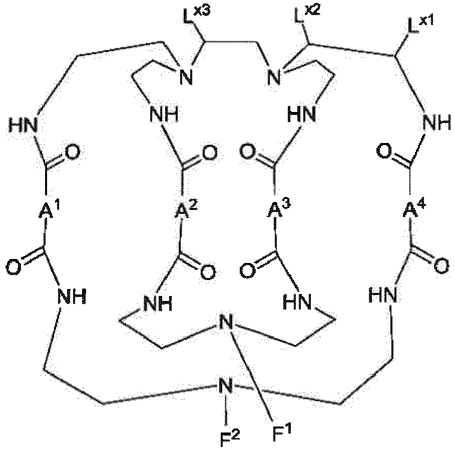 wobei L^{x1}, L^{x2} und L^{x3} unabhängig voneinander ausgewählt sind aus H und einem Linker.

18. Di-Macrocyclus nach Anspruch **17,** aufweisend die folgende Struktur:

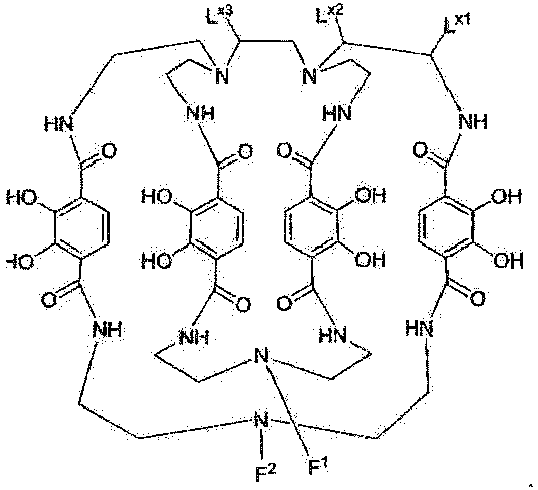

19. Di-Macrocyclus nach Anspruch **17,** aufweisend die folgende Struktur:

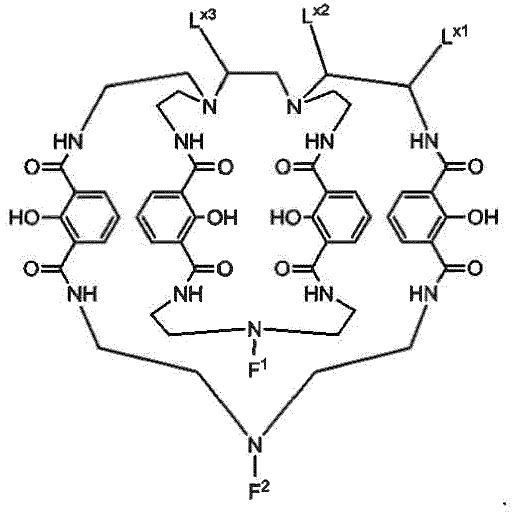

20. Komplex, umfassend einen Di-Macrocyclus nach einem der vorhergehenden Ansprüche und ein Metallion, wobei das Metallion gegebenenfalls ein Lanthanid ist, gegebenenfalls das Lanthanid ausgewählt ist aus Eu und Tb, wobei das Metallion gegebenenfalls ein Actinid ist, wobei das Metallion gegebenenfalls ausgewählt ist aus Yttrium(III), Europium(III), Terbium(III), Zirconium(IV) und Thorium(IV), wobei das Metallion gegebenenfalls ein Radionuklid ist, ausgewählt aus Isotopen von U, Pu, Fe, Cu, Sm, Gd, Tb, Dy, Ho, Er, Yb, Lu, Y, Th, Zr, In, Ga, Bi, Ra, At und Ac, wobei das Metallion gegebenenfalls ²²⁷Th(IV) oder ⁸⁹Zr(IV) ist, wobei das Metallion gegebenenfalls ein Glied ist, ausgewählt aus ¹⁷⁷Lu, ¹⁶⁶Ho, ¹⁵³Sm, ⁹⁰Y, ⁸⁶Y, ¹⁶⁶Dy, ¹⁶⁵Dy, ¹⁶⁹Er, ¹⁷⁵Yb, ²²⁵Ac, ¹⁴⁹Tb, ¹⁵³Gd und ²³⁰U oder wobei das Metallion gegebenenfalls ein Glied ist, ausgewählt aus ¹¹¹In, ⁶⁷Ga, ⁶⁷Cu, ⁶⁴Cu, ¹⁸⁶Re, ¹⁸⁸Re, ¹¹¹Ag, ¹⁰⁹Pd, ²¹²Pb, ²⁰³Pb, ²¹²Bi, ²¹³Bi, ^{195m}Pt, ²⁰¹T1, ⁵⁵Co und ^{99m}Tc.

21. Di-Macrocyclus nach einem der Ansprüche 1 bis 19 oder Komplex, umfassend einen Di-Macrocyclus nach Anspruch 20, wobei die von Estradiol abgeleitete Gruppe ausgewählt ist aus Estradiol und die von Estradiol abgeleitete Gruppe die folgende Struktur aufweist

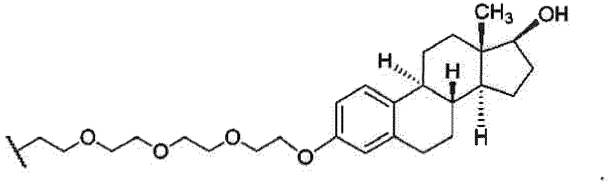

## Revendications

1. Di-macrocycle présentant la structure : 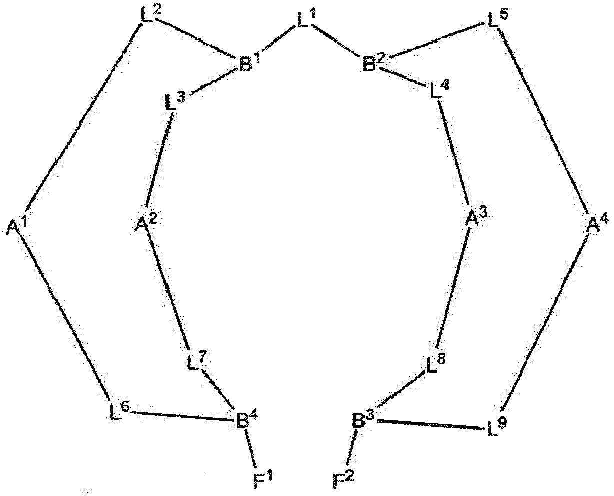 dans laquelle
B¹, B², B³ et B⁴ sont indépendamment choisis parmi un atome N, C, B, Si et P ;
F¹ et F² sont indépendamment choisis parmi un atome H, un groupe (C₁ à C₈)alkyle, un groupe hétéroalkyle de 2, 3, 4, 5 ou 6 atomes choisis parmi C, N, O, Si et S de sorte que le groupe hétéroalkyle contienne au moins un atome C et au moins un hétéroatome, un groupe (C₅ à C₈)aryle, un groupe (C₃ à C₈)hétéroaryle, lesdits groupes aryle contenant 1, 2, 3 ou 4 hétéroatomes choisis parmi N, O et S et un groupe (C₃ à C₈)hétérocycloalkyle, et, lorsque F1 et F2 ne représentent pas un atome H, F1 et F2 comprenant chacun une fraction de modification choisie parmi un groupe de solubilisation, une fraction dérivée d'estradiol, un oligonucléotide, un ADN simple brin, un ADN double brin, un ARN et un peptide, et éventuellement F1 et F2 étant indépendamment choisis parmi un polyéther et un peptide ;
L¹, L², L³, L⁴, L⁵, L⁶, L⁷, L⁸ et L⁹ sont indépendamment choisis parmi un groupe (C₁ à C₈)alkyle éventuellement substitué par un lieur, un groupe hétéroalkyle de 2, 3, 4, 5 ou 6 atomes choisis parmi C, N, O, Si et S de sorte que le groupe hétéroalkyle contienne au moins un atome C et au moins un hétéroatome, et un groupe (C₃ à C₈)hétérocycloalkyle;
A¹, A², A³ et A⁴ sont des membres indépendamment choisis parmi les structures générales : 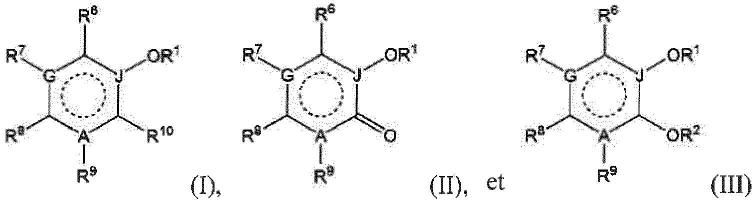 dans lesquelles
A et G sont indépendamment choisis parmi un atome de carbone, d'azote et d'oxygène ;
J est choisi parmi un atome de carbone et d'azote ;
chaque groupe R¹ et R² sont indépendamment choisis parmi un atome H, et une simple charge négative ;
chaque groupe R⁶, R⁷, R⁸, R⁹ et R¹⁰ sont indépendamment choisis parmi une liaison à L², L³, L⁴, L⁵, L⁶, L⁷, L⁸ ou L⁹, un groupe alcanediyle fixé à L², L³, L⁴, L⁵, L⁶, L⁷, L⁸ ou L⁹,
un atome H, un groupe alkyle, hétéroalkyle, halogéno, CN,
CF₃, -C(O)R¹⁷, -SO₂NR¹⁷R¹⁸, -NR¹⁷R¹⁸, -OR¹⁷, -S(O)₂R¹⁷, -COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸ et -NO₂,
dans lesquelles
au moins deux des groupes R⁶, R⁷, R⁸, R⁹ et R¹⁰ sont éventuellement joints pour former un système cyclique choisis parmi un groupe cycloalkyle, hétérocycloalkyle, aryle et hétéroaryle ;
R¹⁷ et R¹⁸ sont indépendamment choisis parmi un atome H, un groupe alkyle, hétéroalkyle, aryle, hétéroaryle et hétérocycloalkyle ; et
R¹⁷ et R¹⁸, conjointement avec les atomes auxquels ils sont fixés, sont éventuellement joints pour former un noyau à 5, 6 ou 7 chaînons,
lorsque A représente un atome d'oxygène, R⁹ n'est pas présent ; et
lorsque G représente un atome d'oxygène, R⁷ n'est pas présent ;
A¹ est fixé à L² et L⁶ par l'intermédiaire de deux membres choisis parmi R⁶, R⁷, R⁸, R⁹ et R¹⁰;
A² est fixé à L³ et L⁷ par l'intermédiaire de deux membres choisis parmi R⁶, R⁷, R⁸, R⁹ et R¹⁰_{;}
A³ est fixé à L⁴ et L⁸ par l'intermédiaire de deux membres choisis parmi R⁶, R⁷, R⁸, R⁹ et R¹⁰ ; et
A⁴ est fixé à L⁵ et L⁹ par l'intermédiaire de deux membres choisis parmi R⁶, R⁷, R⁸, R⁹ et R¹⁰.

2. Di-macrocycle selon la revendication **1,** dans lequel
lorsque A¹ présente une structure selon la formule (I), A¹ est fixé à L² et L⁶ par l'intermédiaire de R⁶ et R¹⁰ ;
lorsque A¹ présente une structure selon la formule (II) ou (III), A¹ est fixé à L² et L⁶ par l'intermédiaire de R⁶ et R⁹ ;
lorsque A² présente une structure selon la formule (I), A² est fixé à L³ et L⁷ par l'intermédiaire de R⁶ et R¹⁰ ;
lorsque A² présente une structure selon la formule (II) ou (III), A² est fixé à L³ et L⁷ par l'intermédiaire de R⁶ et R⁹ ;
lorsque A³ présente une structure selon la formule (I), A³ est fixé à L⁴ et L⁸ par l'intermédiaire de R⁶ et R¹⁰;
lorsque A³ présente une structure selon la formule (II) ou (III), A³ est fixé à L⁴ et L⁸ par l'intermédiaire de R⁶ et R⁹ ;
lorsque A⁴ présente une structure selon la formule (I), A⁴ est fixé à L⁵ et L⁹ par l'intermédiaire de R⁶ et R¹⁰; et
lorsque A⁴ présente une structure selon la formule (II) ou (III), A⁴ est fixé à L⁵ et L⁹ par l'intermédiaire de R⁶ et R⁹.

3. Di-macrocycle selon la revendication **2,** dans lequel
lorsque A¹ présente une structure selon la formule (I), au moins un groupe parmi R⁶ et R¹⁰ de A¹ représente une liaison fixée à L² ou L⁶ ;
lorsque A² présente une structure selon la formule (I), au moins un groupe parmi R⁶ et R¹⁰ de A² représente une liaison fixée à L³ ou L⁷ ;
lorsque A³ présente une structure selon la formule (I), au moins un groupe parmi R⁶ et R¹⁰ de A³ représente une liaison fixée à L⁴ ou L⁸; et
lorsque A⁴ présente une structure selon la formule (I), au moins un groupe parmi R⁶ et R¹⁰ de A⁴ représente une liaison fixée à L⁵ ou L⁹.

4. Di-macrocycle selon la revendication **1,** dans lequel chacun des groupes A¹, A², A³ et A⁴ est indépendamment choisi parmi :

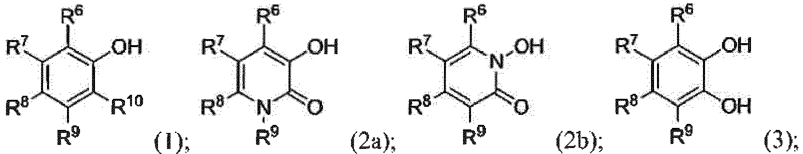

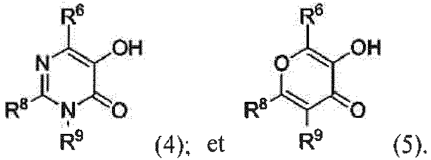

5. Di-macrocycle selon la revendication **4,** dans lequel
lorsque A¹ présente une structure selon la formule (1), A¹ est fixé à L² et L⁶ par l'intermédiaire de R⁶ et R¹⁰;
lorsque A¹ présente une structure selon la formule (2a), (2b), (3), (4) ou (5), A¹ est fixé à L² et L⁶ par l'intermédiaire de R⁶ et R⁹ ;
lorsque A² présente une structure selon la formule (1), A² est fixé à L³ et L⁷ par l'intermédiaire de R⁶ et R¹⁰;
lorsque A² présente une structure selon la formule (2a), (2b), (3), (4) ou (5), A² est fixé à L³ et L⁷ par l'intermédiaire de R⁶ et R⁹ ;
lorsque A³ présente une structure selon la formule (1), A³ est fixé à L⁴ et L⁸ par l'intermédiaire de R⁶ et R¹⁰ ;
lorsque A³ présente une structure selon la formule (2a), (2b), (3), (4) ou (5), A³ est fixé à L⁴ et L⁸ par l'intermédiaire de R⁶ et R⁹ ;
lorsque A⁴ présente une structure selon la formule (1), A⁴ est fixé à L⁵ et L⁹ par l'intermédiaire de R⁶ et R¹⁰; et
lorsque A⁴ présente une structure selon la formule (2a), (2b), (3), (4) or (5), A⁴ est fixé à L⁵ et L⁹ par l'intermédiaire de R⁶ et R⁹.

6. Di-macrocycle selon la revendication **5,** dans lequel
lorsque A¹ présente une structure selon la formule (1), au moins un groupe parmi R⁶ et R¹⁰ de A¹ représente une liaison fixée à L² ou L⁶ ;
lorsque A² présente une structure selon la formule (1), au moins un groupe parmi R⁶ et R¹⁰ de A² représente une liaison fixée à L³ ou L⁷ ;
lorsque A³ présente une structure selon la formule (1), au moins un groupe parmi R⁶ et R¹⁰ de A³ représente une liaison fixée à L⁴ ou L⁸ ; et
lorsque A⁴ présente une structure selon la formule (1), au moins un groupe parmi R⁶ et R¹⁰ de A⁴ représente une liaison fixée à L⁵ ou L⁹.

7. Di-macrocycle selon l'une quelconque des revendications **1 à 6,** présentant la structure : 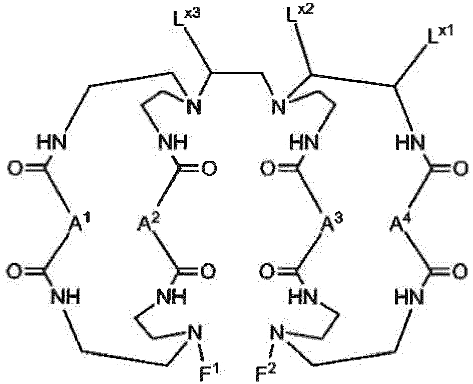 dans laquelle L^{x1}, L^{x2} et L^{x3} sont indépendamment choisis parmi un atome H et un lieur.

8. Di-macrocycle selon la revendication 7, présentant la structure :

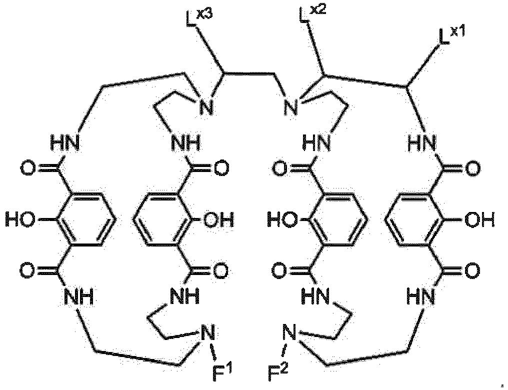

9. Di-macrocycle selon l'une quelconque des revendications 1 à 6, présentant la structure : 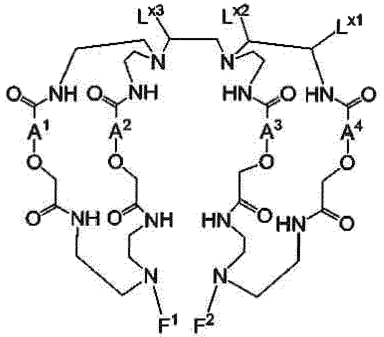 dans laquelle L^{x1}, L^{x2} et L^{x3} sont indépendamment choisis parmi un atome H et un lieur.

10. Di-macrocycle selon la revendication 9, présentant la structure :

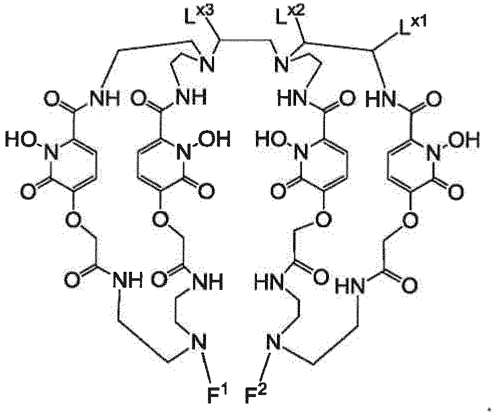

11. Di-macrocycle présentant la structure : 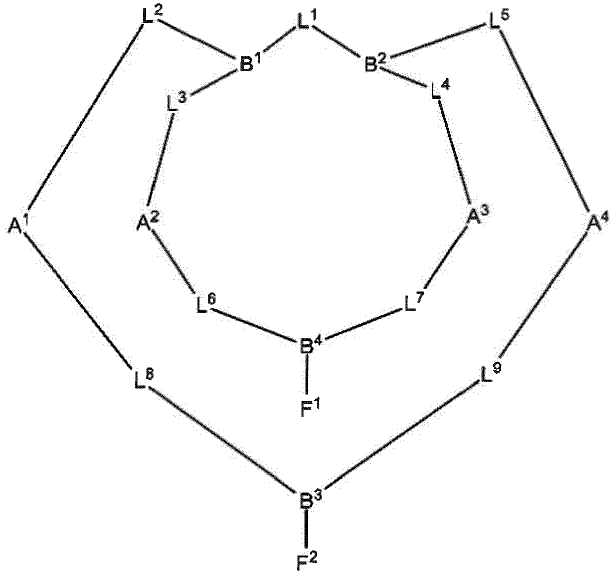 dans laquelle
B¹, B², B³ et B⁴ sont indépendamment choisis parmi un atome N, C, B, Si et P ;
F¹ et F² sont indépendamment choisis parmi un atome H, un groupe (C₁ à C₈)alkyle, un groupe hétéroalkyle de 2, 3, 4, 5 ou 6 atomes choisis parmi C, N, O, Si et S de sorte que le groupe hétéroalkyle contienne au moins un atome C et au moins un hétéroatome, un groupe (C₅ à C₈)aryle, un groupe (C₃ à C₈)hétéroaryle, lesdits groupes aryle contenant 1, 2, 3 ou 4 hétéroatomes choisis parmi N, O et S et un groupe (C₃ à C₈)hétérocycloalkyle, et, lorsque F1 et F2 ne représentent pas un atome H, F1 et F2 comprenant chacun une fraction de modification choisie parmi un groupe de solubilisation, une fraction dérivée d'estradiol, un oligonucléotide, un ADN simple brin, un ADN double brin, un ARN et un peptide, et éventuellement F1 et F2 étant indépendamment choisis parmi un polyéther et un peptide ;
L¹, L², L³, L⁴, L⁵, L⁶, L⁷, L⁸ et L⁹ sont indépendamment choisis parmi un groupe (C₁ à C8)alkyle éventuellement substitué par un lieur, un groupe hétéroalkyle de 2, 3, 4, 5 ou 6 atomes choisis parmi C, N, O, Si et S de sorte que le groupe hétéroalkyle contienne au moins un atome C et au moins un hétéroatome, un groupe (C₅ à C₈)aryle, (C₃ à C₈)hétéroaryle, lesdits groupes aryle contenant 1, 2, 3 ou 4 hétéroatomes choisis parmi N, O et S et un groupe (C₃ à C₈)hétérocycloalkyle;
A¹, A², A³ et A⁴ sont des membres indépendamment choisis parmi les structures générales : 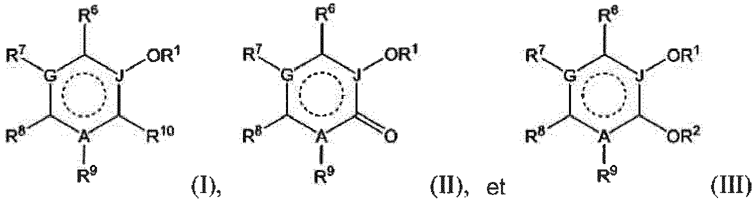 dans lesquelles
A et G sont indépendamment choisis parmi un atome de carbone, d'azote et d'oxygène ;
J est choisi parmi un atome de carbone et d'azote ;
chaque groupe R¹ et R² sont indépendamment choisis parmi un atome H, et une simple charge négative ;
chaque groupe R⁶, R⁷, R⁸, R⁹ et R¹⁰ sont indépendamment choisis parmi
une liaison à L², L³, L⁴, L⁵, L⁶, L⁷, L⁸ ou L⁹, un groupe alcanediyle fixé à L², L³, L⁴, L⁵, L⁶, L⁷, L⁸ ou L⁹,
un atome H, un groupe alkyle, hétéroalkyle, halogéno, CN,
CF₃, -C(O)R¹⁷, -SO₂NR¹⁷R¹⁸, -NR¹⁷R¹⁸, -OR¹⁷, -S(O)₂R¹⁷,-COOR¹⁷, -S(O)₂OR¹⁷, -OC(O)R¹⁷, -C(O)NR¹⁷R¹⁸, -NR¹⁷C(O)R¹⁸, -NR¹⁷SO₂R¹⁸ et -NO₂,
dans lesquelles
au moins deux des groupes R⁶, R⁷, R⁸, R⁹ et R¹⁰ sont éventuellement joints pour former un système cyclique choisis parmi un groupe cycloalkyle, hétérocycloalkyle, aryle et hétéroaryle ;
R¹⁷ et R¹⁸ sont indépendamment choisis parmi un atome H, un groupe alkyle, hétéroalkyle, aryle, hétéroaryle et hétérocycloalkyle ; et
R¹⁷ et R¹⁸, conjointement avec les atomes auxquels ils sont fixés, sont éventuellement joints pour former un noyau à 5, 6 ou 7 chaînons,
lorsque A représente un atome d'oxygène, R⁹ n'est pas présent ; et
lorsque G représente un atome d'oxygène, R⁷ n'est pas présent ;
A¹ est fixé à L² et L⁸ par l'intermédiaire de deux membres choisis parmi R⁶, R⁷, R⁸, R⁹ et R¹⁰;
A² est fixé à L³ et L⁶ par l'intermédiaire de deux membres choisis parmi R⁶, R⁷, R⁸, R⁹ et R¹⁰;
A³ est fixé à L⁴ et L⁷ par l'intermédiaire de deux membres choisis parmi R⁶, R⁷, R⁸, R⁹ et R¹⁰; et
A⁴ est fixé à L⁵ et L⁹ par l'intermédiaire de deux membres choisis parmi R⁶, R⁷, R⁸, R⁹ et R¹⁰.

12. Di-macrocycle selon la revendication **1** ou **11,** ledit di-macrocycle étant modifié de manière covalente avec au moins un lieur.

13. Di-macrocycle selon la revendication **12,** l'un des groupes L¹, L², L³, L⁴, L⁵, L⁶, L⁷, L⁸ et L⁹ étant substitué par un lieur.

14. Di-macrocycle selon l'une quelconque des revendications **11 à 13,** dans lequel
lorsque A¹ présente une structure selon la formule (I), A¹ est fixé à L² et L⁸ par l'intermédiaire de R⁶ et R¹⁰;
lorsque A¹ présente une structure selon la formule (II) ou (III), A¹ est fixé à L² et L⁸ par l'intermédiaire de R⁶ et R⁹ ;
lorsque A² présente une structure selon la formule (I), A² est fixé à L³ et L⁶ par l'intermédiaire de R⁶ et R¹⁰;
lorsque A² présente une structure selon la formule (II) ou (III), A² est fixé à L³ et L⁶ par l'intermédiaire de R⁶ et R⁹;
lorsque A³ présente une structure selon la formule (I), A³ est fixé à L⁴ et L⁷ par l'intermédiaire de R⁶ et R¹⁰;
lorsque A³ présente une structure selon la formule (II) ou (III), A³ est fixé à L⁴ et L⁷ par l'intermédiaire de R⁶ et R⁹;
lorsque A⁴ présente une structure selon la formule (I), A⁴ est fixé à L⁵ et L⁹ par l'intermédiaire de R⁶ et R¹⁰; et
lorsque A⁴ présente une structure selon la formule (II) ou (III), A⁴ est fixé à L⁵ et L⁹ par l'intermédiaire de R⁶ et R⁹.

15. Di-macrocycle selon l'une quelconque des revendications **11 à 14,** dans lequel chacun des groupes A¹, A², A³ et A⁴ est indépendamment choisi parmi :

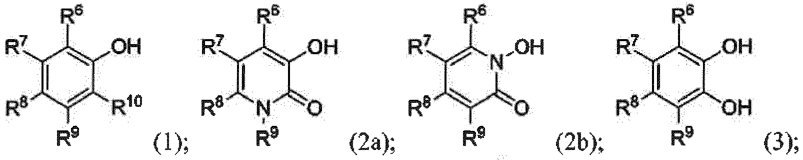

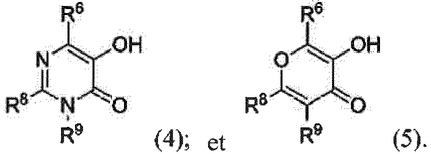

16. Di-macrocycle selon la revendication **15,** dans lequel
lorsque A¹ présente une structure selon la formule (1), A¹ est fixé à L² et L⁸ par l'intermédiaire de R⁶ et R¹⁰ ;
lorsque A¹ présente une structure selon la formule (2a), (2b), (3), (4) ou (5), A¹ est fixé à L² et L⁸ par l'intermédiaire de R⁶ et R⁹;
lorsque A² présente une structure selon la formule (1), A² est fixé à L³ et L⁶ par l'intermédiaire de R⁶ et R¹⁰ ;
lorsque A² présente une structure selon la formule (2a), (2b), (3), (4) or (5), A² est fixé à L³ et L⁶ par l'intermédiaire de R⁶ et R⁹ ;
lorsque A³ présente une structure selon la formule (1), A³ est fixé à L⁴ et L⁷ par l'intermédiaire de R⁶ et R¹⁰ ;
lorsque A³ présente une structure selon la formule (2a), (2b), (3), (4) or (5), A³ est fixé à L⁴ et L⁷ par l'intermédiaire de R⁶ et R⁹;
lorsque A⁴ présente une structure selon la formule (1), A⁴ est fixé à L⁵ et L⁹ par l'intermédiaire de R⁶ et R¹⁰ ; et
lorsque A⁴ présente une structure selon la formule (2a), (2b), (3), (4) or (5), A⁴ est fixé à L⁵ et L⁹ par l'intermédiaire de R⁶ et R⁹.

17. Di-macrocycle selon l'une quelconque des revendications **11 à 16,** présentant la structure : 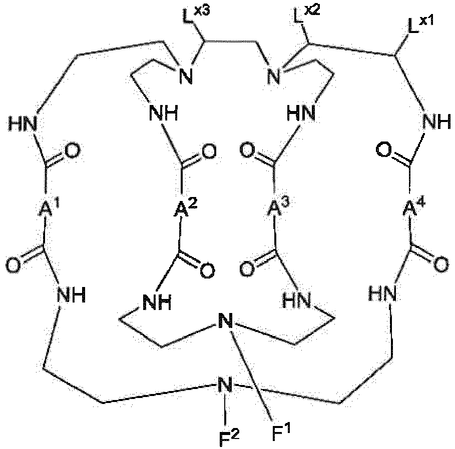 dans laquelle L^{x1}, L^{x2} et L^{x3} sont indépendamment choisis parmi un atome H et un lieur.

18. Di-macrocycle selon la revendication **17,** présentant la structure :

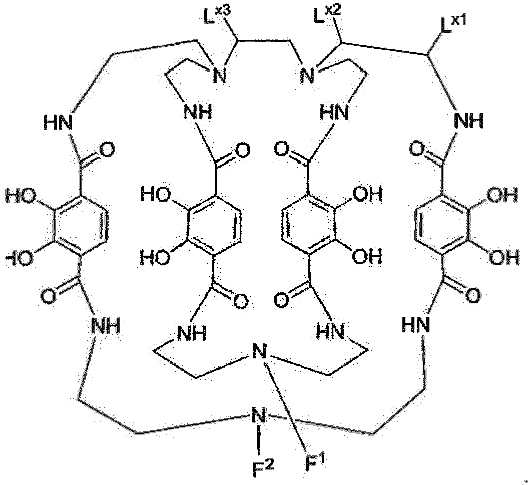

19. Di-macrocycle selon la revendication **17,** présentant la structure :

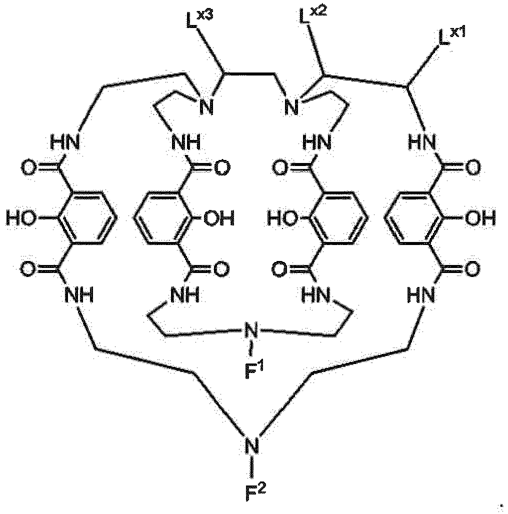

20. Complexe comprenant un di-macrocycle selon l'une quelconque des revendications précédentes et un ion métallique,
éventuellement ledit ion métallique étant un lanthanide, éventuellement ledit lanthanide étant choisi parmi Eu et Tb,
éventuellement ledit ion métallique étant un actinide,
éventuellement ledit ion métallique étant choisi parmi l'yttrium(III), l'europium(III), le terbium(III), le zirconium(IV) et le thorium(IV),
éventuellement ledit ion métallique étant un radionucléide choisi parmi les isotopes de U, Pu, Fe, Cu, Sm, Gd, Tb, Dy, Ho, Er, Yb, Lu, Y, Th, Zr, In, Ga, Bi, Ra, At et Ac,
éventuellement ledit ion métallique étant ²²⁷Th(IV) or ⁸⁹Zr(IV),
éventuellement ledit métal étant un élément choisi parmi ¹⁷⁷Lu, ¹⁶⁶Ho, ¹⁵³Sm, ⁹⁰Y, ⁸⁶Y, ¹⁶⁶Dy, ¹⁶⁵Dy, ¹⁶⁹Er, ¹⁷⁵Yb, ²²⁵Ac, ¹⁴⁹Tb, ¹⁵³Gd et ²³⁰U ou
éventuellement ledit métal étant un élément choisi parmi ¹¹¹In, ⁶⁷Ga, ⁶⁷Cu, ⁶⁴Cu, ¹⁸⁶Re, ¹⁸⁸Re, ¹¹¹Ag, ¹⁰⁹Pd, ²¹²Pb, ²⁰³Pb, ²¹²Bi, ²¹³Bi, ^{195m}Pt, ²⁰¹Tl, ⁵⁵Co et ^{99m}Tc.

21. Di-macrocycle selon l'une quelconque des revendications 1 à 19 ou complexe comprenant un di-macrocycle selon la revendication 20, ledit groupe dérivé d'estradiol étant choisi parmi un estradiol et le groupe dérivé d'estradiol présentant la structure :
